(19) Europäisches Patentamt
European Patent Office
Office européen des brevets

(11) **EP 2 114 562 B1**

(12) **EUROPÄISCHE PATENTSCHRIFT**

(45) Veröffentlichungstag und Bekanntmachung des Hinweises auf die Patenterteilung:
**01.11.2017 Patentblatt 2017/44**

(21) Anmeldenummer: **08707887.9**

(22) Anmeldetag: **14.01.2008**

(51) Int Cl.:
*B01J 23/00* (2006.01)        *B01J 23/888* (2006.01)
*B01J 27/186* (2006.01)       *B01J 32/00* (2006.01)
*B01J 37/00* (2006.01)        *C07C 45/35* (2006.01)
*C07C 51/25* (2006.01)        *C07C 47/22* (2006.01)
*C07C 51/215* (2006.01)

(86) Internationale Anmeldenummer:
**PCT/EP2008/050342**

(87) Internationale Veröffentlichungsnummer:
**WO 2008/087116 (24.07.2008 Gazette 2008/30)**

(54) **VERFAHREN ZUR HERSTELLUNG VON KATALYSATORFORMKÖRPERN, DEREN AKTIVMASSE EIN MULTIELEMENTOXID IST**

METHOD FOR PRODUCING CATALYST MOULDED BODIES WHOSE ACTIVE MASS IS A MULTI-ELEMENT OXIDE

PROCÉDÉ DE PRODUCTION DE CORPS MOULÉS, DONT LA MASSE ACTIVE EST UN OXYDE MULTI-ÉLÉMENTS

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **19.01.2007 DE 102007003778**
**19.01.2007 US 885701 P**
**26.01.2007 DE 102007004961**
**26.01.2007 US 886757 P**

(43) Veröffentlichungstag der Anmeldung:
**11.11.2009 Patentblatt 2009/46**

(73) Patentinhaber: **BASF SE**
**67056 Ludwigshafen am Rhein (DE)**

(72) Erfinder:
• **RAICHLE, Andreas**
**67063 Ludwigshafen (DE)**
• **ROSOWSKI, Frank**
**68239 Mannheim (DE)**
• **HUBER, Sabine**
**67117 Limburgerhof (DE)**
• **CREMER, Ulrich**
**68161 Mannheim (DE)**
• **ALTWASSER, Stefan**
**67157 Wachenheim (DE)**
• **MÜLLER-ENGEL, Klaus Joachim**
**76297 Stutensee (DE)**

(56) Entgegenhaltungen:
**WO-A-95/07144          WO-A-2005/030393**
**US-A- 4 764 498        US-A- 5 773 382**
**US-A1- 2005 131 253**

**Beschreibung**

Vorliegende Erfindung betrifft ein Verfahren zur Herstellung von Katalysatorformkörpern, deren Aktivmasse ein Multielementoxid ist, bei dem man ein feinteiliges Vorläufergemisch, das als ein feinteiliges Formungshilfsmittel (bzw. Formgebungshilfsmittel) Graphit zugesetzt enthält, zur gewünschten Geometrie formt (verdichtet) und die dabei resultierenden Katalysatorvorläuferformkörper bei erhöhter Temperatur unter Erhalt der Katalysatorformkörper, deren Aktivmasse ein Multielementoxid ist, thermisch behandelt.

Ferner betrifft die vorliegende Erfindung Verfahren von heterogen katalysierten Gasphasenreaktionen, z. B. Gasphasenpartialoxidationen organischer Verbindungen, bei denen vorgenannte Katalysatorformkörper als Katalysatoren verwendet werden. Beispiele für solche heterogen katalysierten Partialoxidationsverfahren sind die Herstellung von Acrolein aus Propylen (WO 2005/030393), die Herstellung von Methacrylsäure aus Methacrolein (EP-A 467 144) sowie die Herstellung von Maleinsäureanhydrid aus n-Butan (WO 01/68245).

Acrolein bildet beispielsweise ein wichtiges Zwischenprodukt bei der Herstellung von Acrylsäure, die durch heterogen katalysierte partielle Oxidation von Acrolein erhältlich ist. Acrylsäure bildet ein bedeutendes Monomeres, das als solches oder in Form seiner Alkylester radikalisch polymerisiert werden kann. Die resultierenden Polymerisate eignen sich u. a. als superabsorbierende Materialien oder als Klebstoffe. In entsprechender Weise eignet sich auch Methacrylsäure als solche oder in Form ihrer Alkylester zur Herstellung radikalischer Polymerisate. Eine herausragende Position nimmt dabei z. B. der Methylester der Methacrylsäure ein, der insbesondere zur Herstellung von Polymethylmethacrylat Verwendung findet, das als Kunststoffglas eingesetzt wird. Maleinsäureanhydrid ist ein wichtiges Zwischenprodukt bei der Synthese von $\gamma$-Butyrolacton, Tetrahydrofuran und 1,4-Butandiol, welche ihrerseits als Lösungsmittel eingesetzt werden oder beispielsweise zu Polymeren wie Polytetrahydrofuran oder Polyvinylpyrrolidon weiterverarbeitet werden.

Unter einer vollständigen Oxidation einer organischen Verbindung mit molekularem Sauerstoff wird in dieser Schrift verstanden, dass die organische Verbindung unter reaktiver Einwirkung von molekularem Sauerstoff so umgesetzt wird, dass der in der organischen Verbindung insgesamt enthaltene Kohlenstoff in Oxide des Kohlenstoffs und der in der organischen Verbindung insgesamt enthaltene Wasserstoff in Oxide des Wasserstoffs umgewandelt wird. Alle davon verschiedenen Umsetzungen einer organischen Verbindung unter reaktiver Einwirkung von molekularem Sauerstoff werden hier als Partialoxidationen einer organischen Verbindung zusammengefasst.

Im besonderen sollen in dieser Schrift unter Partialoxidationen solche Umsetzungen organischer Verbindungen unter reaktiver Einwirkung von molekularem Sauerstoff verstanden werden, bei denen die partiell zu oxidierende organische Verbindung nach beendeter Umsetzung wenigstens ein Sauerstoffatom mehr chemisch gebunden enthält als vor Durchführung der Partialoxidation. Der Begriff der partiellen Oxidation soll in dieser Schrift aber auch die oxidative Dehydrierung und partielle Ammoxidation, d. h., eine partielle Oxidation im Beisein von Ammoniak, umfassen.

Es ist allgemein bekannt, dass Multielementoxide (insbesondere Multimetalloxide) insbesondere geeignete Aktivmassen für Katalysatoren zur Durchführung heterogen katalysierter Partialoxidationen organischer Verbindungen in der Gasphase sind (vgl. z. B. alle in der DE-A 103 53 014 sowie in der DE-A 103 60 396 aufgeführten heterogen katalysierten Partialoxidationen).

Eingangs beschriebene Verfahren zur Herstellung von Katalysatorformkörpern sind z. B. aus den Schriften WO 01/68245, WO 2005/030393, DE-A 10 2005 035 978, EP-A 1 060 792, WO 03/078310, WO 03/78059, DE-A 10 2005 037 678, EP-A 467 144 und aus Research Disclosure RD 2005-497012 vorbekannt. Die Mehrzahl der vorgenannten Schriften misst dabei der Beschaffenheit des als Formungshilfsmittel verwendeten Graphits im Hinblick auf die Leistungsfähigkeit des resultierenden Katalysatorformköpers (insbesondere als Katalysator für heterogen katalysierte Partialxidationen organischer Verbindungen in der Gasphase) überhaupt keine Bedeutung zu.

Lediglich in Ausführungsbeispielen der WO 2005/030393 sowie in Vergleichsbeispielen von Research Disclosure RD 2005-497012 wird wertfrei darauf hingewiesen, dass die spezifische Oberfläche des verwendeten Graphit 6 bis 13 $m^2$/g betrug und sein Partikeldurchmesser $d_{50}$ einen Wert von 19,3 $\mu m$ aufwies.

D. h., der Zusatz des Formungshilfsmittels Graphit erfolgt bei der Katalysatorformkörperherstellung in vorgenannten Schriften im wesentlichen ausschließlich als Gleitmittel, d. h., um die mechanische Abnutzung der Formungswerkzeuge zu mindern.

Die US-A 2005/0131253 betrifft ein Verfahren zur Herstellung von Katalysatorformkörpern auf Basis von Multielementoxiden unter Mitverwendung von feinteiligem Graphit als Formungshilfsmittel mit einem Partikeldurchmesser $d_{50}$ von 10 bis 50 $\mu m$. In den beispielhaften Ausführungsformen beträgt $d_{50}$ 31 und 29 $\mu m$. Als wesentliche Eigenschaft des feinteiligen Graphit wird jedoch nicht dessen Teilchengröße, sondern dessen "combustion initiating temperature" erachtet, die wenigstens 50 °C oberhalb der angewandten Kalzinationstemperatur liegen sollte.

Nachteilig an den beschriebenen Verfahren des Standes der Technik ist jedoch, dass sie den Einfluss der Beschaffenheit der Körnung des als Formungshilfsmittel eingesetzten feinteiligen Graphit auf das katalytische Erscheinungsbild (insbesondere auf die Selektivität der Zielproduktbildung bei mit ihnen heterogen katalysierten partiellen Gasphasenoxidationen organischer Ausgangsverbindungen) der resultierenden Katalysatorformkörper nicht erkannten. Vor diesem Hintergrund bestand die Aufgabe der vorliegenden Erfindung darin, ein verbessertes Verfahren zur Herstellung von Katalysatorformkörpern zur Verfügung zu stellen, bei dem insbesondere Katalysatorformkörper resultieren, die insbesondere verbesserte Zielproduktselektivitäten bei ihrer Verwendung als Katalysatoren für heterogen katalysierte partielle Gasphasenoxidationen organischer Ausgangsverbindungen ermöglichen.

Demgemäss wurde ein Verfahren zur Herstellung von Katalysatorformkörpern, deren Aktivmasse ein Multielementoxid ist, und bei dem man ein feinteiliges Vorläufergemisch, das als ein feinteiliges Formungshilfsmittel Graphit zugesetzt enthält, zur gewünschten Geometrie formt (verdichtet) und die dabei resultierenden Katalysatorvorläuferformkörper bei erhöhter Temperatur unter Erhalt der Katalysatorformkörper, deren Aktivmasse ein Multielementoxid ist, thermisch behandelt, gefunden, das dadurch gekennzeichnet ist, dass

a) für die spezifische Oberfläche $O_G$ des feinteiligen Graphit gilt:

$$0{,}5 \ m^2/g \leq O_G \leq 5 \ m^2/g,$$

und

b) für den Partikeldurchmesser des feinteiligen Graphit gilt:

$$90 \ \mu m \leq d_{50} \leq 200 \ \mu m,$$

$$20 \ \mu m \leq d_{10} \leq 90 \ \mu m,$$

$$150 \ \mu m \leq d_{90} \leq 300 \ \mu m.$$

D. h., die Verwendung von im Vergleich zum Stand der Technik begrenzt grobkörnigem Graphit mit geringer spezifischer Oberfläche vermag die erfindungsgemäße Aufgabe zu lösen.

Der Katalysatorformkörper kann z. B. eine Kugel mit einem Durchmesser von 2 bis 10 mm, oder ein Vollzylinder mit einem Außendurchmesser und einer Höhe von 2 bis 10 mm, oder ein Ring, dessen Außendurchmesser und Höhe 2 bis 10 mm betragen und dessen Wandstärke 1 bis 3 mm beträgt, sein.

Erfindungsgemäß ist die Verwendung solcher feinteiligen Graphite als Formungshilfsmittel, für die zutrifft, dass einerseits 90 $\mu m$ (oder 100 $\mu m$) $\leq d_{50} \leq$ 170 $\mu m$, und andererseits 0,5 $m^2/g \leq O_G \leq$ 5 $m^2/g$, vorzugsweise 0,5 $m^2/g \leq O_G \leq$ 4 $m^2/g$, besser 0,75 $m^2/g \leq O_G \leq$ 3 $m^2/g$, besonders bevorzugt 1 $m^2/g \leq O_G \leq$ 2,5 $m^2/g$ und ganz besonders bevorzugt 1,25 $M^2/g \leq O_G \leq$ 2,5 $m^2/g$ bzw. 1,5 $m^2/g \leq O_G \leq$ 2 $m^2/g$.

Erfindungsgemäß erfüllen im vorgenannten Raster bei einem $d_{50}$ von $\geq$ 90 $\mu m$ und $\leq$ 200 $\mu m$ die Partikeldurchmesser $d_{10}$ und $d_{90}$ des feinteiligen Graphits gleichzeitig die folgenden Bedingungen:

$$20 \ \mu m \leq d_{10} \leq 90 \ \mu m,$$

und

$$150 \ \mu m \leq d_{90} \leq 300 \ \mu m.$$

In der Regel wird dabei $d_{10} < d_{50} < d_{90}$ sein.

Grundsätzlich kann für das erfindungsgemäße Verfahren sowohl synthetischer (synthetisch erzeugter) als auch natürlicher (natürlich vorkommender) Graphit eingesetzt werden. Erfindungsgemäß bevorzugt ist der Einsatz von natürlichem Graphit, da dieser aufgrund seiner ausgeprägten Schichtstruktur besonders günstige Gleitmitteleigenschaften (Schmiermitteleigenschaften) aufweist. Erfindungsgemäß ganz besonders vorteilhafte natürliche Graphite sind solche, die im wesentlichen frei von mineralischen Verunreinigungen sind.

Ferner ist es für das erfindungsgemäße Herstellverfahren vorteilhaft, wenn bei der thermischen Behandlung der Katalysatorvorläuferformkörper bis zum Erhalt der Katalysatorformkörper (bei dieser Behandlung bildet sich aus der Vorläufermasse die katalytisch aktive Multielementoxidmasse) wenigstens 1 Gew.-%, bevorzugt wenigstens 2 Gew.-%, besonders bevorzugt wenigstens 3 Gew.-%, und ganz besonders bevorzugt wenigsten 5 Gew.-% der in den Katalysatorvorläuferformkörpern enthaltenen Gewichtsmenge an Graphit (gerechnet als reine Menge Kohlenstoff) in gasförmig entweichende Verbindungen gewandelt (z. B. zu CO und/oder $CO_2$ verbrannt) wird. Erfindungsgemäß zweckmäßig beträgt der vorgenannte, bei der thermischen Behandlung der Katalysatorvorläuferformkörper bis zum Erhalt der Katalysatorformkörper gasförmig entweichende Gewichtsanteil der in den Katalysatorvorläuferformkörpern enthaltenen Gewichtsmenge an Graphit (gerechnet als reine Menge Kohlenstoff) jedoch nicht mehr als 70 Gew.-%, bevorzugt nicht mehr als 60 Gew.-%, besonders bevorzugt nicht mehr als 50 Gew.-% und ganz besonders bevorzugt nicht mehr als 40 Gew.-% bzw. nicht mehr als 30 Gew.-%. Anwendungstechnisch zweckmäßig liegt der vorgenannte Gewichtsanteil bei 5 bis 15 Gew.-%.

Typische Graphiteinsatzmengen belaufen sich beim erfindungsgemäßen Verfahren auf 0,1 bis 35 bzw. bis 20, oder 0,5 bis 10, oder 1 bzw. 2 bis 5 Gew.-% (gerechnet als reiner Kohlenstoff und bezogen auf die Masse des Katalysatorvorläuferformkörpers). Häufig beträgt die so bezogene Graphiteinsatzmenge 2 bis 5 Gew.-%. Wird eine besonders porenbildende Wirkung gewünscht, kann aber auch eine Graphiteinsatzmenge von 15 bis 35 Gew.-% vorteilhaft sein.

Normalerweise ist das feinteilige Vorläufergemisch, das als ein feinteiliges Formungshilfsmittel (Verdichtungshilfsmittel) erfindungsgemäß feinteiligen Graphit zugesetzt enthält, bei der Formgebung trocken (anfasstrocken). In der Regel enthält das feinteilige Vorläufergemisch den erfindungsgemäß zuzusetzenden Graphit als einziges Formungshilfsmittel zugesetzt. Grundsätzlich kann der erfindungsgemäße Zusatz an Graphit aber auch gemeinsam mit anderen Formungshilfsmitteln wie z. B. Bornitrid, Ruß, Polyethylenglycol, Stearinsäure, Stärke, Polyacrylsäure, Mineraloder Pflanzenöl, Wasser, feinteiligem Teflonpulver (z. B. Pulver der Fa. Aldrich 43093-5) und/oder Bortrifluorid zum feinteiligen Vorläufergemisch erfolgen.

Die thermische Behandlung der Katalysatorvorläuferformkörper kann erfindungsgemäß vorteilhaft und an die individuelle Art der jeweils angestrebten Multielementoxid(aktiv)masse angepasst bei Temperaturen (die von außen auf die Katalysatorformkörper einwirken) von 150 bis 650°C erfolgen. Häufig wird die thermische Behandlung der Katalysatorvorläuferformkörper bei Temperaturen im Bereich von 200 °C bis 600°C, bzw. 250°C bis 550°C, bzw. 300°C bis 500°C erfolgen. Die Dauer der thermischen Behandlung kann sich dabei über einen Zeitraum von wenigen Stunden bis zu mehreren Tagen erstrecken. Die thermische Behandlung kann dabei unter Vakuum, unter inerter Atmosphäre (z. B. $N_2$, Edelgase, Wasserdampf, $CO_2$ etc.), unter reduzierender Atmosphäre (z. B. $H_2$ oder $NH_3$) oder unter oxidierender Atmosphäre erfolgen. In der Regel werden oxidierende Atmosphären molekularen Sauerstoff enthalten. Typische oxidierende Atmosphären sind Gemische aus Inertgas ($N_2$, Edelgase, Wasserdampf, $CO_2$ etc.) und molekularem Sauerstoff. Üblicherweise wird der Gehalt an molekularem Sauerstoff dabei wenigstens 0,1 Vol.-%, häufig wenigstens 0,2 Vol-%, vielfach wenigstens 0,5 Vol.-%, oft wenigstens 1 Vol.-%, oder wenigstens 10 Vol.-%, oder wenigstens 20 Vol.-% betragen. Selbstverständlich kann der Gehalt an molekularem Sauerstoff in solchen Gemischen aber auch 30 Vol.-%, oder 40 Vol.-%, oder 50 Vol.-%, oder 70 Vol.-%, oder mehr betragen.

Selbstverständlich kommt als Atmosphäre für die thermische Behandlung aber auch reiner molekularer Sauerstoff in Betracht. Häufig wird die thermische Behandlung unter Luft erfolgen. Generell kann die thermische Behandlung der Katalysatorvorläuferformkörper unter stehender oder unter fließender Gasatmosphäre (z. B. im Luftstrom) erfolgen.

Der Begriff der Atmosphäre (bzw. der Gasatmosphäre) in welcher die thermische Behandlung erfolgt ist dabei in dieser Schrift so zu verstehen, dass er sich aus den Katalysatorvorläuferformkörpern im Rahmen der thermischen Behandlung aufgrund von Zersetzungsvorgängen entwickelnde Gase nicht umfasst. Selbstverständlich kann die Gasatmosphäre in welcher die thermische Behandlung erfolgt, aber auch ausschließlich oder teilweise aus diesen Gasen bestehen. Im Rahmen der bei dem erfindungsgemäßen Verfahren erfolgenden thermischen Behandlung können sowohl die Behandlungstemperatur, als auch die Behandlungsatmosphäre über die Behandlungsdauer zeitlich konstant oder auch zeitlich variabel gestaltet sein.

Da Graphit als Kohlenstoff enthaltende Substanz brennbar ist, erfolgt die thermische Behandlung der das feinteilige Graphit erfindungsgemäß zugesetzt enthaltenden Katalysatorvorläuferformkörper in an sich bekannter Weise anwendungstechnisch zweckmäßig normalerweise so, dass sich das in den Katalysatorvorläuferformkörpern enthaltene Graphit während der thermischen Behandlung nicht entzündet, um so über die von außen bei der thermischen Behandlung auf die Katalysatorvorläuferformkörper einwirkende Temperatur gegebenenfalls im Formkörperinneren weit hinausgehende Temperaturen (sogenannte Heißpunkttemperaturen im Kalzinationsgut) weitestgehend zu vermeiden, da derartige Temperaturspitzen die katalytische Qualität der resultierenden Katalysatorformkörper mindern können. Diese Zielsetzung erfordert insbesondere dann eine gewisse Sorgfalt, wenn die Atmosphäre in welcher die thermische Behandlung erfolgt, molekularen Sauerstoff als solchen und/oder molekularen Sauerstoff liefernde Bestandteile enthält und/oder aus der Zusammensetzung der Vorläufermasse selbst heraus eine Graphit oxidierende Wirkung resultiert. Dabei ist zu berücksichtigen, dass natürlich vorkommender Graphit in der Regel ein Gemisch aus Graphit und mineralischen Bestandteilen ist, die eine Entzündung des Graphits zu katalysieren vermögen. Auch beeinflussen die Körnung sowie die Oberflächenbeschaffenheit des erfindungsgemäß verwendeten Graphits dessen Entzündungsverhalten.

In diesem Zusammenhang ist es für das erfindungsgemäße Verfahren von generellem Vorteil, wenn diejenige Temperatur $T_i$ (die Probentemperatur bzw. genauer die Ofentemperatur), bei der bei einer simultanen dynamischen Differenzkalorimetrie-Thermogravimetrie des erfindungsgemäß zu verwendenden Graphits erstmals ein Gewichtsverlust

zu beobachten ist ($T_i$ soll in dieser Schrift als "Temperatur des Verbrennungsanfanges" bezeichnet werden) wenigstens 50°C, besser wenigsten 75°C, noch besser wenigstens 100°C, bevorzugt wenigstens 125°C und besonders bevorzugt wenigstens 150°C größer ist, als die im Rahmen der erfindungsgemäßen thermischen Behandlung der Katalysatorvorläuferformkörper auf diese für einen Zeitraum von wenigstens 30 Minuten einwirkende höchste Temperatur.

Da das Ergebnis einer Ermittlung von $T_i$ mittels einer simultanen dynamischen Differenzkalorimetrie - Thermogravimetrie nicht nur eine Funktion des mit dieser Methode zu überprüfenden Graphits, sondern auch eine ausgeprägte Funktion des gewählten Überprüfungsrahmens (z. B. auch der untersuchten Probenmenge) ist, bezieht sich die in dieser Schrift verwendete Temperatur $T_i$ auf das nachfolgend beschriebene Überprüfungsprinzip, einschließlich des angegebenen Überprüfungsrahmens:

Die zu überprüfende (untersuchende) Graphitprobe (Probenmenge = 10 mg) wird in einem offenen Probentiegel ($Al_2O_3$) in einem einen vertikalen Rohrofen aufweisenden Prüfgerät (Netzsch STA 449 C Jupiter®; Gerätetyp: simultane Thermoanalyse, Kopplung von dynamischem Wärmestromdifferenz-Kalorimeter und kompensierender Thermowaage mit vertikalem Rohrofen) der NETZSCH-Gerätebau GmbH, Wittelsbacherstraße 42, D-95100 Selb/Bayern einem vorgegebenen Temperaturprogramm unterworfen (Heizrate = 0,3 K/min; Temperaturbereich = 30 °C (Starttemperatur) bis 1000 °C (Endtemperatur)). Als Ofenatmosphäre wird Luft mit einer Durchflussrate von 20,0 $cm^3$/min verwendet.

In Abhängigkeit von der Ofentemperatur wird aus der Temperaturdifferenz zwischen der zu prüfenden Graphitprobe und einem in definiertem thermischem Kontakt zur Probe stehenden leeren inerten Referenzprobentiegel ($Al_2O_3$) der Wärmefluss von bzw. zu der Probe bestimmt. Probentiegel mit Probe, Referenzprobentiegel ohne Probe und die Thermofühler befinden sich auf einer Waage. Dadurch ist simultan zur dynamischen Differenzkalorimetrie (DSC = Dynamic Scanning Calorimetry; Methode zur Quantifizierung kalorischer Effekte) eine Thermogravimetrie (TG) möglich (= Methode zur Messung der Massenänderung (bzw. Gewichtsänderung) einer Probe im Verlauf des Temperaturprogramms; bestimmt wird die Probenmasse in Abhängigkeit von der Ofentemperatur).

Die TG-Auflösung beträgt 0,1 $\mu$g und die DSC-Auflösung liegt bei < 1 $\mu$W. Im übrigen erfolgt die Prüfung gemäß DIN 51006 und DIN 51007.

$T_i$-Werte von erfindungsgemäß zu verwendenden Graphiten $\geq$ 500°C, bevorzugt $\geq$ 550°C erweisen sich bei vielen erfindungsgemäßen Herstellungsverfahren als vorteilhaft. In der Regel liegt $T_i$ jedoch bei Werten $\leq$ 700°C, meist $\leq$ 650°C.

Für das erfindungsgemäße Verfahren ist es weiterhin günstig , wenn bei der vorstehend beschriebenen Thermogravimetrie des erfindungsgemäß zu verwendenden Graphits diejenige Temperatur $T_m$ (Probentemperatur bzw. besser Ofentemperatur), bei der die Abnahme der Masse der Graphitprobe mit der Zunahme der Temperatur ihren maximalen Wert erreicht (in dieser Schrift als "Temperatur des Verbrennungsmaximums" bezeichnet) möglichst hoch liegt. Mit Vorteil beträgt $T_m$ für erfindungsgemäß zu verwendende Graphite $\geq$ 680 °C, bevorzugt $\geq$ 700 °C, und besonders bevorzugte $\geq$ 750°C. In der Regel liegt $T_m$ bei Werten $\leq$ 900°C, vielfach bei Werten $\leq$ 850°C.

Zusätzlich ist es für das erfindungsgemäße Verfahren günstig, wenn bei der vorstehend beschriebenen Thermogravimetrie des erfindungsgemäßen Graphits diejenige Temperatur $T_f$ (Probentemperatur bzw. besser Ofentemperatur), ab der kein weiterer Gewichtsverlust mehr zu beobachten ist (an der der Gewichtsverlust beendet ist) 150 bis 350°C (zweckmäßig 250°C) oberhalb von $T_i$ liegt.

An dieser Stelle sei festgehalten, dass sich alle Angaben in dieser Schrift zu spezifischen Oberflächen von Graphiten auf Bestimmungen nach DIN 66131 (Bestimmungen der spezifischen Oberfläche von Feststoffen durch Gasadsorption ($N_2$) nach Brunauer-Emmet-Teller (BET)) beziehen.

Alle Angaben in dieser Schrift zu Porengesamtvolumina sowie zu Porendurchmesserverteilungen auf diese Porengesamtvolumina sowie zu spezifischen Oberflächen (jeweils von den Katalysatorformkörpern) beziehen sich auf Bestimmungen mit der Methode der Quecksilberporosimetrie in Anwendung des Gerätes Auto Pore 9220 der Fa. Micromeritics GmbH, 4040 Neuss, DE (Bandbreite 30 Å bis 0,3 mm). Im übrigen wird die Bestimmungsmethode angegeben.

Zur Bestimmung von Partikeldurchmesserverteilungen sowie den aus diesen entnommenen Partikeldurchmessern $d_{10}$, $d_{50}$ und $d_{90}$ wurde das jeweilige feinteilige Pulver über eine Dispergierrinne in den Trockendispergierer Sympatec RODOS (Sympatec GmbH, System-Partikel-Technik, Am Pulverhaus 1, D-38678 Clausthal-Zellerfeld) geführt und dort mit Druckluft trocken dispergiert und im Freistrahl in die Messzelle geblasen. In dieser wird dann nach ISO 13320 mit dem Laserbeugungsspektrometer Malvern Mastersizer S (Malvern Instruments, Worcestshire WR14 1AT, United Kingdom) die volumenbezogene Partikeldurchmesserverteilung bestimmt. Die als Messergebnis angegebenen Partikeldurchmesser $d_x$ sind dabei so definiert, dass X % des Gesamtpartikelvolumens aus Partikeln mit diesem oder einem kleineren Durchmesser bestehen.

Zur Bestimmung des Graphitgehalts (gerechnet als reine Kohlenstoffmenge) im Katalysatorvorläuferformkörper und im fertiggestellten Katalysatorformkörper kann man z. B. eine repräsentative Anzahl der jeweiligen Formkörper zu einem Pulver zerkleinern. Die Bestimmung erfolgt dann an einer jeweils identischen Anteilsmenge die absolut in zweckmäßiger Weise eine Masse von 50 bis 20 mg aufweist. Diese pulverförmige Probe wird dann in Gegenwart eines Sauerstoffstroms in ein auf etwa 1000°C erhitztes waagerechtes Quarzrohr gegeben und geglüht. Das dabei erhaltene Verbrennungsgas wird durch eine IR-Zelle geleitet und die in diesem enthaltene Menge an Kohlendioxid durch Infrarotabsorption quantitativ bestimmt. Aus der Menge an detektiertem Kohlendioxid kann der jeweilige Graphitgehalt (gerechnet als reine Kohlenstoffmenge) zurückgerechnet werden (vgl. "Quantitative Organische Elementaranalyse", VCN Verlagsgesellschaft mbH, Weinheim; Ausgabe 1991; ISBN: 3-527-28056-1, Seite 225 ff).

Die Partikeldurchmesser des feinteiligen Vorläufergemischs (das zugesetzte Formungshilfsmittel ausgenommen) werden (für wenigstens 90 % des Gewichts des Vorläufergemischs) bei seiner Formung zur gewünschten Geometrie des Katalysatorvorläuferformkörpers in der Regel im Bereich von 10 bis 2000 $\mu$m liegen. Vielfach werden vorgenannte Partikeldurchmesser im Bereich 20 bis 1800 $\mu$m, oder 30 bis 1700 $\mu$m, oder 40 bis 1600 $\mu$m, oder 50 bis 1500 $\mu$m liegen. Besonders häufig werden diese Partikeldurchmesser 100 bis 1500 $\mu$m, oder 150 bis 1500 $\mu$m betragen.

Im Regelfall erfolgt die Formung (Verdichtung) des feinteiligen Vorläufergemischs zur Geometrie des Katalysatorvorläuferformkörpers durch Einwirkung äußerer Kräfte (Druck) auf das feinteilige Vorläufergemisch. Der dabei anzuwendende Formgebungsapparat bzw. die dabei anzuwendende Formgebungsmethode unterliegt dabei keiner Beschränkung. Ebenso unterliegt die angestrebte Geometrie des Katalysatorvorläuferformkörpers keiner Beschränkung. D. h., die Katalysatorvorläuferformkörper können regelmäßig oder unregelmäßig geformt sein, wobei regelmäßig geformte Formkörper in der Regel bevorzugt sind.

Der Katalysatorvorläuferformkörper kann Kugelgeometrie aufweisen. Dabei kann der Kugeldurchmesser z. B. 2 bis 10 mm, oder 4 bis 8 mm betragen. Die Geometrie des Katalysatorvorläuferformkörpers kann aber auch vollzylindrisch oder hohlzylindrisch sein.

In beiden Fällen können Aussendruchmesser und Höhe z. B. 2 bis 10 mm oder 2 bzw. 4 bis 8 mm betragen. Im Fall von Hohlzylindern ist in der Regel eine Wandstärke von 1 bis 3 mm zweckmäßig. Selbstverständlich kommen als Katalysatorvorläufergeometrie aber auch alle diejenigen Geometrien in Betracht, die in der WO 02/062737 offenbart und empfohlen werden. In der Regel weicht beim erfindungsgemäßen Verfahren die Geometrie des resultierenden Katalysatorformkörpers von der Geometrie des Katalysatorvorläuferformkörpers nur unwesentlich ab.

An dieser Stelle soll festgehalten werden, dass erfindungsgemäß besonders vorteilhafte Verfahren zur Herstellung von Katalysatorformkörpern und damit besonders günstige Katalysatorformkörper dann resultieren, wenn man in den in den Schriften Research Disclosure RD 2005-497012, EP-A 1 060 792, DE-A 10 2005 035 978, DE-A 10 2005 037 678, WO 03/78059, WO 03/78310, DE-A 198 55 913, WO 02/24620, DE-A 199 22 113, WO 01/68245, EP-A 467 114, US-A 2005/0131253, WO 02/062737 und WO 05/030393 offenbarten Herstellverfahren an den Stellen, wo feinteiliger Graphit mitverwendet wird, erfindungsgemäß zu verwendenden Graphit einsetzt und alle übrigen Herstellmaßnahmen unverändert beibehält. Die dabei resultierenden Katalysatorformkörper sind dann in völlig entsprechender Weise wie in den Schriften DE-A 199 22 113, WO 01/68245, EP-A 467 114, DE-A 198 55 913, US-A 2005/0131253, WO 02/24620, WO 03/78059, WO 03/078310, WO 02/062737, Research Disclosure RD 2005-497012, EP-A 1 060 792, DE-A 10 2005 035 978, DE-A 10 2005 037 678, und WO 05/030393 beschrieben für die entsprechenden heterogen katalysierten Gasphasenreaktionen einsetzbar. Sie sind dabei insbesondere dann vorteilhaft, wenn als Graphit der natürliche Graphit Asbury 3160 und/oder der synthetische Graphit Asbury 4012 der Firma Asbury Graphite Mills, Inc. New Jersey 08802, USA eingesetzt wird. Insbesondere bei Verwendung synthetischer Graphite ist es für die Schmiereigenschaften in der Regel förderlich, erhöhte Einsatzmengen des zu verwendenden Graphits anzuwenden. Generell, aber vor allem bei Graphiteinsatzmengen $\geq$ 3 Gew.-%, kann es zur Vermeidung einer zu schnellen Zersetzung (die z. B. zur Rissbildung oder zur Minderung der Katalysatorqualität führen kann) vorteilhaft sein, im Temperaturbereich zwischen $T_i$ und $T_m$ eine oder mehrere Haltedauern zu verlängern, zusätzliche Heizrampen bzw. Heizzonen zu ergänzen und/oder Temperaturen anzupassen.

Die Formgebung kann beim erfindungsgemäßen Verfahren z. B. durch Strangpressen, Tablettieren oder Extrudieren erfolgen. Dabei wird das feinteilige Vorläufergemisch, wie bereits erwähnt, üblicherweise anfasstrocken eingesetzt. Es kann jedoch bis zu 10 % seines Gesamtgewichtes Substanzen zugesetzt enthalten, die bei Normalbedingungen (25 °C, 1 atm) flüssig sind. Das erfindungsgemäße Verfahren ist aber auch dann anwendbar, wenn das feinteilige Vorläufergemisch überhaupt keine solchen flüssigen Substanzen mehr enthält. Selbstverständlich kann das feinteilige Vorläufergemisch aber auch aus festen Solvaten (z. B. Hydraten) bestehen, die solche flüssigen Substanzen in chemisch und/oder physikalisch gebundener Form aufweisen.

Die im Rahmen des erfindungsgemäßen Verfahrens angewandten Formgebungsdrucke werden im allgemeinen 50 kg/cm$^2$ bis 5000 kg/cm$^2$ betragen. Vorzugsweise betragen die Formgebungsdrucke 200 bis 3500 kg/cm$^2$, besonders bevorzugt 600 bis 2500 kg/cm$^2$. Das Vorgenannte gilt insbesondere dann, wenn als Formgebungsverfahren die Tablettierung angewendet wird. Die Grundzüge des Tablettierens sind z. B. in "Die Tablette", Handbuch der Entwicklung, Herstellung und Qualitätssicherung, W.A. Ritschel und A. Bauer-Brandl, 2. Auflage, Editio Cantor Verlag Aulendorf, 2002 beschrieben und in völlig entsprechender Weise auf ein erfindungsgemäßes Tablettierverfahren übertragbar.

Als Multielementoxidmassen kommen im Rahmen des erfindungsgemäßen Verfahrens sowohl Aktivmassen in Betracht, die neben Sauerstoff sowohl Metalle als auch Nichtmetalle als elementare Konstituenten enthalten. Häufig handelt es sich bei den Multielementoxid(aktiv)massen aber um reine Multimetalloxid(aktiv)massen.

Für eine Anwendung des erfindungsgemäßen Verfahrens besonders günstige Multielementoxid(aktiv)massen samt zugehöriger Vorläufermassen sind z. B. jene, die in den Schriften WO 2005/030393, EP-A 467 144, EP-A 1 060 792, DE-A 198 55 913, WO 01/68245, EP-A 1060792, Research Disclosure RD 2005- 497012, WO 03/078310, DE-A 102005035978, DE-A 102005037678, WO 03/78059, WO 03/078310, DE-A 199 22 113, WO 02/24620, WO 02/062737 und US-A 2005/0131253 offenbart sind.

Erfindungsgemäß verwendbare feinteilige Vorläufergemische sind in einfachster Weise z. B. dadurch erhältlich, indem man von Quellen der elementaren Konstituenten der angestrebten Aktivmasse (Multielementoxidmasse) ein feinteiliges, möglichst inniges, der Stöchiometrie der gewünschten Aktivmasse entsprechend zusammengesetztes, formbares Gemisch erzeugt, dem noch Formungs- und gegebenenfalls Verstärkungshilfsmittel zugesetzt werden können (und/oder von Anfang an eingearbeitet werden können).

Als Quellen für die elementaren Konstituenten der gewünschten Aktivmasse kommen grundsätzlich solche Verbindungen in Betracht, bei denen es sich bereits um Oxide handelt und/oder um solche Verbindungen, die durch Erhitzen, wenigstens in Anwesenheit von gasförmigem molekularem Sauerstoff und/oder von gasförmigen Sauerstoff freisetzenden Komponenten, in Oxide überführbar sind. Prinzipiell kann die Sauerstoffquelle aber auch z. B. in Form eines Peroxids Bestandteil des Vorläufergemisches selbst sein. Auch kann das Vorläufergemisch Verbindungen wie $NH_4OH$, $(NH_4)_2CO_3$, $NH_4NO_3$, $NH_4CHO_2$, $CH_3COOH$, $NH_4CH_3CO_2$, Ammoniumoxalat und/oder organische Komponenten wie z. B. Stearinsäure zugesetzt enthalten, die bei der thermischen Behandlung als Porenbildner zu vollständig gasförmig entweichenden Verbindungen zerfallen und/oder zersetzt werden können.

Das, vorzugsweise innige, Vermischen der Ausgangsverbindungen (Quellen) zur Herstellung des feinteiligen formbaren Vorläufergemischs, kann beim erfindungsgemäßen Verfahren in trockener oder in nasser Form erfolgen. Erfolgt es in trockener Form, so werden die Ausgangsverbindungen zweckmäßigerweise als feinteilige Pulver (mit einem Partikeldurchmesser $d_{50}$ zweckmäßig im Bereich 1 bis 200 $\mu$m, vorzugsweise 2 bis 180 $\mu$m, besonders bevorzugt 3 bis 170 $\mu$m und ganz besonders bevorzugt 4 bis 160 $\mu$m, oder 5 bis 150 $\mu$m bzw. 10 bis 120 $\mu$m, oder 15 bis 80 $\mu$m liegend) eingesetzt. Nach Zusatz der erfindungsgemäßen Formungshilfsmittel sowie gegebenenfalls erfolgendem Zusatz weiterer Formungs- und/oder Verstärkerhilfsmittel kann anschließend die Formgebung erfolgen. Solche Verstärkungshilfsmittel können beispielsweise Mikrofasern aus Glas, Asbest, Siliciumcarbid und/oder Kaliumtitanat sein. Ganz generell kann beim erfindungsgemäßen Verfahren eine Ausgangsverbindung Quelle für mehr als einen elementaren Konstituenten sein.

Anstatt das feinteilige Vorläufergemisch als solches unmittelbar zur gewünschten Geometrie zu formen, ist es häufig zweckmäßig, als einen ersten Formgebungsschritt zunächst eine Zwischenkompaktierung desselben durchzuführen, um das Pulver zu vergröbern (in der Regel auf Partikeldurchmesser von 100 bis 2000 $\mu$m, bevorzugt 150 bis 1500 $\mu$m, besonders bevorzugt 400 bis 1250 $\mu$m, oder 400 bis 1000 $\mu$m, oder 400 bis 800 $\mu$m).

Dabei kann bereits vor der Zwischenkompaktierung erfindungsgemäß zu verwendender Graphit als Kompaktierhilfsmittel zugesetzt werden. Anschließend erfolgt auf der Grundlage des vergröberten Pulvers die endgültige (eigentliche) Formgebung, wobei bei Bedarf zuvor nochmals feinteiliger erfindungsgemäßer Graphit (sowie gegebenenfalls weitere Formungs- und/oder Verstärkungshilfsmittel) zugegeben werden kann.

Erfindungsgemäß bevorzugt erfolgt das innige Vermischen jedoch in nasser Form. Üblicherweise werden dabei die Ausgangsverbindungen z. B. in Form einer wässrigen (aber auch Flüssigkeiten wie iso-Butanol kommen als Lösungs- und/oder Dispergiermedium in Betracht) Lösung und/oder Suspension miteinander vermischt. Besonders innige formbare Gemische werden dabei dann erhalten, wenn ausschließlich von in gelöster Form vorliegenden Quellen der elementaren Konstituenten ausgegangen wird. Als Lösungsmittel wird bevorzugt Wasser eingesetzt (aber auch Flüssigkeiten wie iso-Butanol kommen als Lösungsmittel in Betracht). Anschließend wird die erhaltene Lösung oder Suspension getrocknet, wobei der Trocknungsprozess vorzugsweise durch Sprühtrocknung mit Austrittstemperaturen von 100 bis 150 °C erfolgt (in manchen Fällen kann die Trocknung aber auch durch Filtration und anschließende Trocknung des Filterkuchens erfolgen). Der Partikeldurchmesser $d_{50}$ des resultierenden Sprühpulvers beträgt in typischer Weise 10 bis 50 $\mu$m. War Wasser die Basis des flüssigen Mediums, wird das resultierende Sprühpulver normalerweise nicht mehr als 20 % seines Gewichtes, vorzugsweise nicht mehr als 15% seines Gewichtes und besonders bevorzugt nicht mehr als 10 Gew.-% seines Gewichtes an Wasser enthalten. Diese Prozentsätze gelten in der Regel auch bei Anwendung anderer flüssiger Lösungs- bzw. Suspendierhilfsmittel. Nach Zusatz der erfindungsgemäßen Formungshilfsmittel zur jeweiligen pulverförmig gestalteten Trockenmasse sowie gegebenenfalls weiterer Formungs- und/oder Verstärkungsmittel, kann das pulverförmige Gemisch als feinteiliges Vorläufergemisch erfindungsgemäß zum gewünschten Katalysatorvorläuferformkörper verdichtet (geformt) werden. Die feinteiligen Formungs- und/oder Verstärkungshilfsmittel können aber auch bereits vorab in die Sprühmaische (teilweise oder vollständig) zugesetzt werden.

Eine nur teilweise Entfernung des Lösungs- bzw. Suspendiermittels kann auch dann zweckmäßig sein, wenn seine Mitverwendung als Formungshilfsmittel beabsichtigt ist. Vorab der erfindungsgemäßen Zugabe des feinteiligen Graphits kann auch bereits eine erste thermische Behandlung des Trockenpulvers erfolgen. Nach Zugabe des Graphits erfolgt dann die Formgebung sowie die erfindungsgemäße thermische Weiterbehandlung.

Anstatt das auf dem Sprühpulver fußende feinteilige Vorläufergemisch als solches unmittelbar zur gewünschten Geometrie zu formen, ist es häufig zweckmäßig, als einen ersten Formgebungsschritt zunächst eine Zwischenkompaktierung durchzuführen, um das Pulver zu vergröbern (in der Regel auf Partikeldurchmesser von 100 bis 2000 $\mu$m,

bevorzugt 150 bis 1500 $\mu$m, besonders bevorzugt 400 bis 1250 $\mu$m, oder 400 bis 1000 $\mu$m, oder 400 bis 800 $\mu$m).

Dabei kann bereits vor der Zwischenkompaktierung erfindungsgemäß zu verwendender Graphit als Kompaktierhilfsmittel zugesetzt werden. Anschließend erfolgt auf der Grundlage des vergröberten Pulvers die endgültige (eigentliche) Formgebung, wobei bei Bedarf zuvor nochmals feinteiliger erfindungsgemäßer Graphit (sowie gegebenenfalls weitere Formungs- und/oder Verstärkungshilfsmittel) zugegeben werden kann.

Selbstverständlich können als Quellen der elementaren Konstituenten auch Ausgangsverbindungen eingesetzt werden, die ihrerseits durch thermische Behandlung von Vorläuferformkörpern erzeugt wurden, und multielementoxidischer Natur sind. Insbesondere können die Ausgangsverbindungen der elementaren Konstituenten multimetallischer Natur sein.

Das erfindungsgemäße Verfahren eignet sich insbesondere zur Herstellung von Katalysatorformkörpern, deren Aktivmasse ein Multielementoxid ist, innerhalb dessen das Element Mo (oder die Elemente V und P) das numerisch (molar gerechnet) am häufigsten auftretende Element ist. Insbesondere eignet es sich zur Herstellung von Katalysatorformkörpern, deren Aktivmasse ein Multielementoxid ist, das die Elemente Mo, Fe und Bi, oder die Elemente Mo und V, oder die Elemente Mo, V und P oder die Elemente V und P umfasst. Die ersteren Katalysatorformkörper in der vorgenannten Reihe eignen sich vor allem für heterogen katalysierte partielle Gasphasenoxidationen von Propylen zu Acrolein. Die zweitgenannten Katalysatorformkörper eignen sich vor allem für heterogen katalysierte partielle Gasphasenoxidationen von Acrolein zur Acrylsäure, die vorletztgenannten Katalysatorformkörper in der vorgenannten Reihe eignen sich vor allem für heterogen katalysierte partielle Gasphasenoxidationen von Methacrolein zu Methacrylsäure und die letztgenannten Katalysatorformkörper eignen sich vor allem für heterogen katalysierte partielle Gasphasenoxidationen von n-Butan zu Maleinsäureanhydrid.

Im besonderen umfasst die vorliegende Erfindung ein Verfahren zur Herstellung von ringförmigen Katalysatorformkörpern (auch Vollkatalysatoren genannt, da sie keinen inerten Trägerkörper aufweisen, auf den die Aktivmasse aufgetragen ist) mit gekrümmter und/oder nicht gekrümmter Stirnfläche der Ringe, deren Aktivmasse (in der Aktivmasse verbliebenes Graphit ist dabei wie stets in dieser Schrift außer acht gelassen, da es sich im Normalfall chemisch inert verhält und nicht katalytisch aktiv ist) eine Stöchiometrie der allgemeinen Formel I,

$$Mo_{12}Bi_aFe_bX^1_cX^2_dX^3_eX^4_fO_n \qquad (I),$$

mit

X$^1$ = Nickel und/oder Kobalt,
X$^2$ = Thallium, ein Alkalimetall und/oder ein Erdalkalimetall,
X$^3$ = Zink, Phosphor, Arsen, Bor, Antimon, Zinn, Cer, Blei, Vanadium, Chrom und/oder Wolfram,
X$^4$ = Silizium, Aluminium, Titan und/oder Zirkonium,

a = 0,2 bis 5,
b = 0,01 bis 5,
c = 0 bis 10,
d = 0 bis 2,
e = 0 bis 8,
f = 0 bis 10 und
n = eine Zahl, die durch die Wertigkeit und Häufigkeit der von Sauerstoff verschiedenen Elemente in I bestimmt wird,

oder eine Stöchiometrie der allgemeinen Formel II,

$$[Y^1_aY^2_b{}'O_{x'}]_p[Y^3_cY^4_{d'}Y^5_{e'}Y^6_{f'}Y^7_{g'}Y^8_{h'}O_{y'}]_q \qquad (II),$$

mit

Y$^1$ = nur Wismut oder Wismut und wenigstens eines der Elemente Tellur, Antimon, Zinn und Kupfer,
Y$^2$ = Molybdän oder Wolfram, oder Molybdän und Wolfram,
Y$^3$ = ein Alkalimetall, Thallium und/oder Samarium,
Y$^4$ = ein Erdalkalimetall, Nickel, Kobalt, Kupfer, Mangan, Zink, Zinn, Cadmium und/oder Quecksilber,
Y$^5$ = Eisen oder Eisen und wenigstens eines der Elemente Vanadium, Chrom und Cer,
Y$^6$ = Phosphor, Arsen, Bor und/oder Antimon,
Y$^7$ = ein seltenes Erdmetall, Titan, Zirkonium, Niob, Tantal, Rhenium, Ruthenium, Rhodium, Silber, Gold, Aluminium, Gallium, Indium, Silizium, Germanium, Blei, Thorium und/oder Uran,
Y$^8$ = Molybdän oder Wolfram, oder Molybdän und Wolfram,

a' =	0,01 bis 8,
b' =	0,1 bis 30,
c' =	0 bis 4,
d' =	0 bis 20,
e' >	0 bis 20,
f' =	0 bis 6,
g' =	0 bis 15,
h' =	8 bis 16,

x', y' =	Zahlen, die durch die Wertigkeit und Häufigkeit der von Sauerstoff verschiedenen Elemente in II bestimmt werden und

p, q =	Zahlen, deren Verhältnis p/q 0,1 bis 10 beträgt,

und deren ringförmige Geometrie, ohne Berücksichtigung einer gegebenenfalls bestehenden Krümmung der Stirnfläche, eine Höhe H von 1,5 bis 8 mm, einen Außendurchmesser A von 2 bis 11 mm und eine Wandstärke W von 0,75 mm bis 2,0 mm aufweist.

Bei diesem Verfahren wird man aus Quellen der elementaren Konstituenten der Aktivmasse ein feinteiliges formbares Vorläufergemisch erzeugen und aus diesem Gemisch, nach Zugabe von erfindungsgemäßem Formungshilfsmittel sowie gegebenenfalls weiterer Formungs- und/oder Verstärkungshilfsmittel, ringförmige Vollkatalysatorvorläuferformkörper formen, deren Stirnflächen gekrümmt und/oder nicht gekrümmt sind, und diese durch thermisches Behandeln bei erhöhter Temperatur in die ringförmigen Vollkatalysatoren überführen.

Außerdem betrifft die vorliegende Erfindung die Verwendung der vorstehenden erfindungsgemäß erhältlichen ringförmigen Vollkatalysatoren als Katalysatoren mit erhöhter Selektivität (bei vergleichbarer Aktivität) für die gasphasenkatalytische Partialoxidation von Propen zu Acrolein sowie von iso-Buten bzw. tert.-Butanol bzw. dessen Methylether zu Methacrolein.

Das vorgenannte Verfahren zur Herstellung der ringförmigen Katalysatorformkörper ist dann besonders vorteilhaft, wenn die Formung (Verdichtung) des feinteiligen Vorläufergemisches so erfolgt, dass die Seitendruckfestigkeit der resultierenden ringförmigen Vollkatalysatorvorläuferformkörper $\geq 10$ und $\leq 40$ N, besser $\geq 10$ und $\leq 35$ N, noch besser $\geq 12$ N und $\leq 23$ N beträgt. Vorzugsweise beträgt die Seitendruckfestigkeit der resultierenden ringförmigen Vollkatalysatorvorläuferformkörper $\geq 13$ N und $\leq 22$ N, bzw. $\geq 14$ N und $\leq 21$ N. Ganz besonders bevorzugt beträgt die Seitendruckfestigkeit der resultierenden ringförmig Vollkatalysatorvorläuferformkörper $\geq 16$ N und $\leq 21$ N.

Ferner beträgt für diese Katalysatortypen die Körnung (der Partikeldurchmesser) des feinteiligen Vorläufergemischs (ausgenommen die zuzusetzenden Hilfsmittel) vorteilhaft 200 µm bis 1500 µm, besonders vorteilhaft 400 µm bis 1000 µm (z. B. eingestellt durch Zwischenkompaktierung). In günstiger Weise liegen wenigstens 80 Gew.-%, besser wenigstens 90 Gew.-% und besonders vorteilhaft wenigstens 95 oder 98 oder mehr Gew.-% des feinteiligen Vorläufergemischs in diesem Körnungsbereich.

Als Seitendruckfestigkeit wird dabei in dieser Schrift die Druckfestigkeit bei Stauchung des ringförmigen Vollkatalysatorvorläuferformkörpers senkrecht zur zylindrischen Einhüllenden (d.h., parallel zur Fläche der Ringöffnung) verstanden. Dabei beziehen sich alle Seitendruckfestigkeiten dieser Schrift auf eine Bestimmung mittels einer Material-Prüfmaschine der Firma Zwick GmbH & Co (D-89079 Ulm) des Typs Z 2.5/TS15. Diese Material-Prüfmaschine ist für quasistatische Beanspruchung mit zügigem, ruhendem, schwellendem oder wechselndem Verlauf konzipiert. Sie ist für Zug-, Druck- und Biegeversuche geeignet. Der installierte Kraftaufnehmer des Typs KAF-TC der Firma A.S.T. (D-01307 Dresden) mit der Herstellnummer 03-2038 wurde dabei entsprechend der DIN EN ISO 7500-1 kalibriert und war für den Messbereich 1-500 N einsetzbar (relative Messunsicherheit: $\pm$ 0,2 %).

Die Messungen wurden mit folgenden Parametern durchgeführt:

Vorkraft: 0,5 N.
Vorkraft-Geschwindigkeit: 10 mm/min.
Prüfgeschwindigkeit: 1,6 mm/min.

Dabei wurde der obere Stempel zunächst langsam bis kurz vor die Fläche der zylindrischen Einhüllenden des ringförmigen Vollkatalysatorvorläuferformkörpers abgesenkt. Dann wurde der obere Stempel abgestoppt, um anschließend mit der deutlich langsameren Prüfgeschwindigkeit mit minimaler, zu weiterer Absenkung erforderlicher, Vorkraft abgesenkt zu werden.

Die Vorkraft, bei der der Vollkatalysatorvorläuferformkörper Rissbildung zeigt, ist die Seitendruckfestigkeit (SDF).

Erfindungsgemäß besonders vorteilhafte Vollkatalysatorringgeometrien erfüllen zusätzlich die Bedingung H / A = 0,3 bis 0,7. Besonders bevorzugt ist H / A = 0,4 bis 0,6.

Weiterhin ist es für die relevanten Vollkatalysatorringe vorteilhaft, wenn das Verhältnis I / A (wobei I der Innendurchmesser der Vollkatalysatorringgeometrie ist) 0,3 bis 0,8, vorzugsweise 0,4 bis 0,7 beträgt.

Besonders vorteilhaft sind Vollkatalysatorringgeometrien, die gleichzeitig eines der vorteilhaften H / A-Verhältnisse und eines der vorteilten I / A-Verhältnisse aufweisen. Solche mögliche Kombinationen sind z. B. H / A = 0,3 bis 0,7 und I / A = 0,3 bis 0,8 oder 0,4 bis 0,7. Alternativ kann H / A 0,4 bis 0,6 und I / A gleichzeitig 0,3 bis 0,8 oder 0,4 bis 0,7 betragen.

Ferner ist es für die relevanten Vollkatalysatorringe bevorzugt, wenn H 2 bis 6 mm und besonders bevorzugt, wenn H 2 bis 4 mm beträgt.

Weiterhin ist es vorteilhaft, wenn A 4 bis 8 mm, vorzugsweise 5 bis 7 mm beträgt.

Die Wandstärke der erfindungsgemäß erhältlichen relevanten Vollkatalysatorringgeometrien beträgt mit Vorteil 1 bis 1,5 mm.

D. h., günstige besagte Vollkatalysatorringgeometrien sind z. B. solche mit H = 2 bis 6 mm und A = 4 bis 8 mm oder 5 bis 7 mm. Alternativ kann H 2 bis 4 mm und A gleichzeitig 4 bis 8 mm oder 5 bis 7 mm betragen. In allen vorgenannten Fällen kann die Wandstärke W 0,75 bis 2,0 mm oder 1 bis 1,5 mm betragen.

Besonders bevorzugt sind unter den vorgenannten günstigen Vollkatalysatorgeometrien jene, bei denen gleichzeitig die vorstehend genannten H / A sowie I / A Kombinationen erfüllt sind.

Mögliche relevante Vollkatalysatorringgeometrien sind somit (A x H x I) 5 mm x 2 mm x 2 mm, oder 5 mm x 3 mm x 2 mm, oder 5 mm x 3 mm x 3 mm, oder 5,5 mm x 3 mm x 3,5 mm, oder 6 mm x 3 mm x 4 mm, oder 6,5 mm x 3 mm x 4,5 mm, oder 7 mm x 3 mm x 5 mm, oder 7 mm x 7 mm x 3 mm, oder 7 mm x 7 mm x 4 mm.

Die Stirnflächen der wie beschrieben beschaffenen Ringe können auch entweder beide oder nur eine wie in der EP-A 184 790 beschrieben gekrümmt sein und zwar z. B. so, dass der Radius der Krümmung vorzugsweise das 0,4 bis 5-fache des Außendurchmessers A beträgt. Erfindungsgemäß bevorzugt sind beide Stirnflächen ungekrümmt.

Alle diese Vollkatalysatorringgeometrien eignen sich z. B. sowohl für die gasphasenkatalytische Partialoxidation von Propen zu Acrolein als auch für die gasphasenkatalytische Partialoxidation von iso-Buten oder tert.-Butanol oder dem Methylether des tert.-Butanols zu Methacrolein.

Betreffend die Aktivmassen der Stöchiometrie der allgemeinen Formel I betragen der stöchiometrische Koeffizient b vorzugsweise 2 bis 4, der stöchiometrische Koeffizient c vorzugsweise 3 bis 10, der stöchiometrische Koeffizient d vorzugsweise 0,02 bis 2, der stöchiometrische Koeffizient e vorzugsweise 0 bis 5 und der stöchiometrische Koeffizient a beträgt vorzugsweise 0,4 bis 2. Der stöchiometrische Koeffizient f beträgt vorteilhaft 0,5 oder 1 bis 10. Besonders bevorzugt liegen die vorgenannten stöchiometrischen Koeffizienten gleichzeitig in den genannten Vorzugsbereichen.

Ferner ist $X^1$ vorzugsweise Kobalt, $X^2$ ist vorzugsweise K, Cs und/oder Sr, besonders bevorzugt K, $X^3$ ist bevorzugt Wolfram, Zink und/oder Phosphor und $X^4$ ist bevorzugt Si. Besonders bevorzugt weisen die Variablen $X^1$ bis $X^4$ gleichzeitig die vorgenannten Bedeutungen auf.

Besonders bevorzugt weisen alle stöchiometrischen Koeffizienten a bis f und alle Variablen $X^1$ bis $X^4$ gleichzeitig ihre vorgenannten vorteilhaften Bedeutungen auf.

Innerhalb der Stöchiometrien der allgemeinen Formel II sind jene bevorzugt, die der allgemeinen Formel III

$$[Bi_{a''}Z^2_{b''}O_{x''}]_p[Z^8_{12}Z^3_{c''}Z^4_{d''}Fe_{e''}Z^5_{f''}Z^6_{g''}Z^7_{h''}O_{y''}]_{q''} \qquad (III),$$

mit

| | | |
|---|---|---|
| $Z^2$ = | Molybdän oder Wolfram, oder Molybdän und Wolfram, | |
| $Z^3$ = | Nickel und/oder Kobalt, | |
| $Z^4$ = | Thallium, ein Alkalimetall und/oder ein Erdalkalimetall, vorzugsweise K, Cs und/oder Sr, | |
| $Z^5$ = | Phosphor, Arsen, Bor, Antimon, Zinn, Cer, Vanadium, Chrom und/oder Bi, | |
| $Z^6$ = | Silizium, Aluminium, Titan und/oder Zirkonium, vorzugsweise Si, | |
| $Z^7$ = | Kupfer, Silber und/oder Gold, | |
| $Z^8$ = | Molybdän oder Wolfram, oder Wolfram und Molybdän | |
| a" = | 0,1 bis 1, | |
| b" = | 0,2 bis 2, | |
| c" = | 3 bis 10, | |
| d" = | 0,02 bis 2, | |
| e" = | 0,01 bis 5, vorzugsweise 0,1 bis 3, | |
| f" = | 0 bis 5, | |
| g" = | 0 bis 10, vorzugsweise > 0 bis 10, besonders bevorzugt 0,2 bis 10 und ganz besonders bevorzugt 0,4 bis 3, | |
| h" = | 0 bis 1, | |
| x", y" = | Zahlen, die durch die Wertigkeit und Häufigkeit der von Sauerstoff verschiedenen Elemente in III bestimmt werden und | |

p", q" =     Zahlen, deren Verhältnis p" / q" 0,1 bis 5, vorzugsweise 0,5 bis 2 beträgt,

entsprechen.

Ferner sind Aktivmassen der Stöchiometrie II bevorzugt, die dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Y^1_{a'}Y^2_{b'}O_{x'}$ enthalten, deren Größtdurchmesser (längste durch den Schwerpunkt des Bereichs gehende Verbindungsstrecke zweier auf der Oberfläche (Grenzfläche) des Bereichs befindlicher Punkte) 1 nm bis 100 $\mu$m, häufig 10 nm bis 500 nm oder 1 $\mu$m bis 50 bzw. 25 $\mu$m, beträgt.

Besonders vorteilhafte Aktivmassen der Stöchiometrie II sind solche, in denen $Y^1$ nur Wismut ist.

Innerhalb der Aktivmassen der Stöchiometrie III sind diejenigen erfindungsgemäß bevorzugt, in denen $Z^2_{b"}$ = (Wolfram)$_{b"}$ und $Z^8_{12}$ = (Molybdän)$_{12}$ ist.

Ferner sind für die diskutierten ringförmigen Vollkatalysatoren Aktivmassen der Stöchiometrie III bevorzugt, die dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Bi_{a"}Z^2_{b"}O_{x"}$ enthalten, deren Größtdurchmesser (längste durch den Schwerpunkt des Bereichs gehende Verbindungsstrecke zweier auf der Oberfläche (Grenzfläche) des Bereichs befindlicher Punkte) 1 nm bis 100 $\mu$m, häufig 10 nm bis 500 nm oder 1 $\mu$m bis 50 bzw. 25 $\mu$m, beträgt.

Ferner ist es von Vorteil, wenn wenigstens 25 mol-% (bevorzugt wenigstens 50 mol-% und besonders bevorzugt wenigstens 100 mol-%) des gesamten Anteils $[Y^1_{a'}Y^2_{b'}O_{x'}]_p$ ($[Bi_{a"}Z^2_{b"}O_{x"}]_p$) der wie beschrieben erhältlichen Aktivmassen der Stöchiometrie I (Aktivmassen der Stöchiometrie III) in den Aktivmassen der Stöchiometrie II (Aktivmassen der Stöchiometrie III) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung $Y^1_{a'}Y^2_{b'}O_{x'}$ ($[Bi_{a"}Z^2_{b"}O_{x"}]$) vorliegen, deren Größtdurchmesser im Bereich 1 nm bis 100 $\mu$m liegt.

Als Formungshilfsmittel (Gleitmittel) kommen für das erfindungsgemäße Verfahren der Herstellung der relevanten ringförmigen Katalysatorformkörper neben erfindungsgemäßem Graphit Ruß, Polyethylenglycol, Stearinsäure, Stärke, Polyacrylsäure, Mineral- oder Pflanzenöl, Wasser, Bortrifluorid und/oder Bornitrid in Betracht. Auch Glycerin und Celluloseether können als weitere Gleitmittel eingesetzt werden. Erfindungsgemäß bevorzugt wird ausschließlich erfindungsgemäß zu verwendender Graphit als Formungshilfsmittel zugesetzt. Bezogen auf die zum Vollkatalysatorvorläuferformkörper zu formende Masse werden insgesamt in der Regel $\leq$ 15 Gew.-%, meist $\leq$ 9 Gew.-%, vielfach $\leq$ 5 Gew.-%, oft $\leq$ 4 Gew.-% an erfindungsgemäß zu verwendendem Graphit zugesetzt. Üblicherweise beträgt die vorgenannte Zusatzmenge $\geq$ 0,5 Gew.-%, meist $\geq$ 2,5 Gew.-%. Bevorzugt zugesetzte erfindungsgemäße Graphite sind Asbury 3160 und Asbury 4012.

Ferner können feinteilige Verstärkungsmittel wie Mikrofasern aus Glas, Asbest, Siliciumcarbid oder Kaliumtitanat zugesetzt werden. Die Formung zum ringförmigen Vollkatalysatorvorläuferformkörper kann z. B. mittels einer Tablettierungsmaschine, einer Extrusionsverformungsmaschine oder dergleichen durchgeführt werden.

Die thermische Behandlung des relevanten ringförmigen Vollkatalysatorvorläuferformkörpers erfolgt in der Regel bei Temperaturen, die 350 °C überschreiten. Normalerweise wird im Rahmen der thermischen Behandlung die Temperatur von 650 °C jedoch nicht überschritten. Erfindungsgemäß vorteilhaft wird im Rahmen der thermischen Behandlung die Temperatur von 600 °C, bevorzugt die Temperatur von 550 °C und besonders bevorzugt die Temperatur von 500 °C nicht überschritten. Ferner wird im Rahmen der thermischen Behandlung des ringförmigen Vollkatalysatorvorläuferformkörpers vorzugsweise die Temperatur von 380 °C, mit Vorteil die Temperatur von 400 °C, mit besonderem Vorteil die Temperatur von 420 °C und ganz besonders bevorzugt die Temperatur von 440 °C überschritten. Dabei kann die thermische Behandlung in ihrem zeitlichen Ablauf auch in mehrere Abschnitte gegliedert sein. Beispielsweise kann zunächst eine thermische Behandlung bei einer Temperatur von 150 bis 350 °C, vorzugsweise 220 bis 290 °C, und daran anschließend eine thermische Behandlung bei einer Temperatur von 400 bis 600 °C, vorzugsweise 430 bis 550 °C durchgeführt werden.

Normalerweise nimmt die thermische Behandlung des ringförmigen Vollkatalysatorvorläuferformkörpers mehrere Stunden (meist mehr als 5 h) in Anspruch. Häufig erstreckt sich die Gesamtdauer der thermischen Behandlung auf mehr als 10 h. Meist werden im Rahmen der thermischen Behandlung des ringförmigen Vollkatalysatorvorläuferformkörpers Behandlungsdauern von 45 h bzw. 25 h nicht überschritten. Oft liegt die Gesamtbehandlungsdauer unterhalb von 20 h. Erfindungsgemäß vorteilhaft werden im Rahmen der thermischen Behandlung des relevanten ringförmigen Vollkatalysatorvorläuferformkörpers 500°C (460°C) nicht überschritten und die Behandlungsdauer im Temperaturfenster von $\geq$ 400°C ($\geq$ 440°C) erstreckt sich auf 5 bis 20 h.

Die thermische Behandlung (auch die nachfolgend angesprochene Zersetzungsphase) der ringförmigen Vollkatalysatorvorläuferformkörper kann sowohl unter Inertgas als auch unter einer oxidativen Atmosphäre wie z. B. Luft (Gemisch aus Inertgas und Sauerstoff) sowie auch unter reduzierender Atmosphäre (z.B. Gemisch aus Inertgas, NH$_3$, CO und/oder H$_2$ oder Methan, Acrolein, Methacrolein) erfolgen. Selbstredend kann die thermische Behandlung auch unter Vakuum ausgeführt werden.

Prinzipiell kann die thermische Behandlung der relevanten ringförmigen Vollkatalysatorvorläuferformkörper in den unterschiedlichsten Ofentypen wie z. B. beheizbare Umluftkammern, Hordenöfen, Drehrohröfen, Bandkalzinierern oder Schachtöfen durchgeführt werden. Bevorzugt erfolgt die thermische Behandlung der ringförmigen Vollkatalysatorvorläuferformkörper in einer Bandkalziniervorrichtung, wie sie die DE-A 100 46 957 und die WO 02/24620 empfehlen.

Die thermische Behandlung der relevanten ringförmigen Vollkatalysatorvorläuferformkörper unterhalb von 350 °C verfolgt in der Regel das Ziel der thermischen Zersetzung der in den Vollkatalysatorvorläuferformkörpern enthaltenen Quellen der elementaren Konstituenten des angestrebten ringförmigen Vollkatalysators. Häufig erfolgt diese Zersetzungsphase im Rahmen des Aufheizens auf Temperaturen $\geq$ 350 °C.

Die ringförmigen Vollkatalysatorvorläuferformkörper von angestrebten ringförmigen Vollkatalysatoren, deren Aktivmasse eine Stöchiometrie der allgemeinen Formel I, oder der allgemeinen Formel II, oder der allgemeinen Formel III aufweist, können dadurch hergestellt werden, dass man von Quellen der elementaren Konstituenten der Aktivmasse des gewünschten ringförmigen Vollkatalysators ein (möglichst inniges) feinteiliges, der Stöchiometrie der gewünschten Aktivmasse entsprechend zusammengesetztes, formbares Gemisch erzeugt und aus diesem, nach Zusatz von (u.a. erfindungsgemäßen) Formungs- und gegebenenfalls Verstärkungshilfsmitteln, einen ringförmigen Vollkatalysatorvorläuferformkörper (mit gekrümmter und/oder nicht gekrümmter Stirnfläche) formt, dessen Seitendruckfestigkeit $\geq$ 12 N und $\leq$ 23 N beträgt. Die Geometrie des ringförmigen Vollkatalysatorvorläuferformkörpers wird dabei im wesentlichen derjenigen des gewünschten ringförmigen Vollkatalysators entsprechen.

Als Quellen für die elementaren Konstituenten der gewünschten Aktivmasse kommen solche Verbindungen in Betracht, bei denen es sich bereits um Oxide handelt und/oder um solche Verbindungen, die durch Erhitzen, wenigstens in Anwesenheit von molekularem Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, AmmoniumSalze und/oder Hydroxide in Betracht (Verbindungen wie $NH_4OH$, $(NH_4)_2CO_3$, $NH_4NO_3$, $NH_4CHO_2$, $CH_3COOH$, $NH_4CH_3CO_2$, Ammoniumoxalat und/oder organische Komponenten wie z. B. Stearinsäure, die beim thermischen Behandeln zu vollständig gasförmig entweichenden Verbindungen zerfallen und/oder zersetzt werden können, können in das feinteilige formbare Gemisch (vorzugsweise ein Trockengemisch) zusätzlich eingearbeitet werden).

Das, vorzugsweise innige, Vermischen der Ausgangsverbindungen (Quellen) zur Herstellung des feinteiligen formbaren Gemischs kann beim erfindungsgemäßen Verfahren in trockener oder in nasser Form erfolgen. Erfolgt es in trockener Form, so werden die Ausgangsverbindungen zweckmäßigerweise als feinteilige Pulver (der Partikeldurchmesser $d_{50}$ sollte vorteilhaft $\leq$ 100 $\mu$m, vorzugsweise $\leq$ 50 $\mu$m betragen; in der Regel wird der Partikeldurchmesser $d_{50}$ $\geq$ 1 $\mu$m betragen) eingesetzt. Nach Zusatz von (u.a. erfindungsgemäßen) Formungs- und gegebenenfalls Verstärkungshilfsmitteln kann anschließend die Formgebung zum ringförmigen Vollkatalysatorvorläuferformkörper erfolgen.

Bevorzugt erfolgt das innige Vermischen jedoch in nasser Form. Üblicherweise werden dabei die Ausgangsverbindungen in Form einer wässrigen Lösung und/oder Suspension miteinander vermischt. Besonders innige formbare Gemische werden dabei dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene Lösung oder Suspension getrocknet, wobei der Trocknungsprozess vorzugsweise durch Sprühtrocknung mit Austrittstemperaturen von 100 bis 150 °C erfolgt. Der Partikeldurchmesser $d_{50}$ des resultierenden Sprühpulvers beträgt in typischer Weise 10 bis 50 $\mu$m, bevorzugt 15 bis 40 $\mu$m.

Das Sprühpulver kann nun nach Zusatz von (u.a. erfindungsgemäßen) Formungs- und gegebenenfalls Verstärkungshilfsmitteln zu den ringförmigen Vollkatalysatorvorläuferformkörpern verdichtet (geformt) werden. Die feinteiligen Formungs- und gegebenenfalls Verstärkungshilfsmittel können aber auch bereits vorab der Sprühtrocknung (teilweise oder vollständig) zugesetzt werden. Auch kann bei der Trocknung das Lösungs- bzw. Suspendiermittel nur teilweise entfernt werden, falls seine Mitverwendung als Formungshilfsmittel beabsichtigt ist.

Anstatt das Sprühpulver nach Zusatz von (u.a. erfindungsgemäßen) Formungs- und gegebenenfalls Verstärkungshilfsmitteln, unmittelbar zu den ringförmigen Vollkatalysatorvorläuferformkörpern (mit gekrümmter und/oder nicht gekrümmter Stirnfläche der Ringe) zu formen, ist es häufig zweckmäßig, zunächst eine Zwischenkompaktierung durchzuführen, um das Pulver zu vergröbern (in der Regel auf eine Körnung von 400 $\mu$m bis 1 mm). Anschließend erfolgt mit dem vergröberten Pulver die eigentliche Ringformung, wobei bei Bedarf zuvor nochmals feinteiliges erfindungsgemäßes Gleitmittel zugegeben werden kann.

Eine solche Zwischenkompaktierung zum Zweck der Kornvergröberung kann beispielsweise mittels eines Kompaktierers der Fa. Hosokawa Bepex GmbH (D-74211 Leingarten), vom Typ Kompaktor K 200/100 erfolgen. Die Härte des Zwischenkompaktats liegt häufig bereits im Bereich von 10 N. Für die Ringformung zum Vollkatalysatorvorläuferformkörper kommt z.B. ein Kilian Rundläufer (der Fa. Kilian in D-50735 Köln) vom Typ RX 73 oder S 100 in Betracht. Alternativ kann eine Tablettenpresse der Fa. Korsch (D-13509 Berlin) vom Typ PH 800-65 eingesetzt werden. Insbesondere zur Herstellung von Aktivmassen der Stöchiometrie der allgemeinen Formel II oder III ist es vorteilhaft, ein Mischoxid $Y^1{}_a{}'Y^2{}_b{}'O_x{}'$ bzw. $Bi_a{}''Z^2{}_b{}''O_x{}''$ als Quelle der Elemente $Y^1$, $Y^2$ bzw. Bi, $Z^2$ in Abwesenheit der übrigen Konstituenten der Aktivmassen der Stöchiometrie der allgemeinen Formel II bzw. III vorzubilden und damit nach seiner Vorbildung, wie bereits beschrieben, mit Quellen der übrigen Konstituenten der Aktivmassen der Stöchiometrie der allgemeinen Formel II bzw. III ein feinteiliges formbares Gemisch zu erzeugen, um daraus, nach Zusatz von (u.a. erfindungsgemäßen) Formungs- und gegebenenfalls Verstärkungshilfsmitteln den ringförmigen Vollkatalysatorvorläuferformkörper zu formen.

Bei einer solchen Vorgehensweise ist lediglich darauf zu achten, dass für den Fall, dass die Herstellung des feinteiligen formbaren Gemischs nass erfolgt (in Suspension), die vorgebildeten Mischoxide $Y^1_{a'}Y^2_{b'}O_{x'}$ bzw. $Bi_{a''}Z^2_{b''}O_{x''}$ nicht in nennenswertem Umfang in Lösung gehen.

Ausführlich wird eine wie vorstehend beschriebene Herstellweise in den Schriften DE-A 44 07 020, EP-A 835, EP-A 575 897 und DE-C 33 38 380 beschrieben.

Beispielsweise kann man wasserlösliche Salze von $Y^1$ wie Nitrate, Carbonate, Hydroxide oder Acetate mit $Y^2$-Säuren oder deren Ammoniumsalzen in Wasser mischen, die Mischung trocknen (vorzugsweise sprühtrocknen) und die getrocknete Masse anschließend thermisch behandeln. Die thermisch behandelte Masse wird nachfolgend zweckmäßig zerkleinert (z. B. in einer Kugelmühle oder durch Strahlmahlen) und aus dem dabei erhältlichen, in der Regel aus im wesentlichen kugelförmigen Partikeln bestehenden, Pulver die Kornklasse mit einem im für die Aktivmasse der Stöchiometrie der allgemeinen Formel II bzw. III gewünschten Größtdurchmesserbereich liegenden Korngrößtdurchmesser durch in an sich bekannter Weise durchzuführendes Klassieren (z. B. Nass- oder Trockensiebung) abgetrennt und vorzugsweise mit, bezogen auf die Masse dieser abgetrennten Kornklasse, 0,1 bis 3 Gew.-%, feinteiligem $SiO_2$ (der Partikeldurchmesser $d_{50}$ der üblicherweise im wesentlichen kugelförmigen $SiO_2$-Partikel beträgt zweckmäßigerweise 100 nm bis 15 μm) vermischt und so eine Ausgangsmasse 1 hergestellt. Die thermische Behandlung erfolgt zweckmäßig bei Temperaturen von 400 bis 900 °C, vorzugsweise bei 600 bis 900 °C. Letzteres gilt insbesondere dann, wenn es sich bei dem vorgebildeten Mischoxid um ein solches der Stöchiometrie $BiZ^2O_6$, $Bi_2Z^2_2O_9$ und/oder $Bi_2Z^2_3O_{12}$ handelt, unter denen das $Bi_2Z^2_2O_9$ bevorzugt ist, insbesondere wenn $Z^2$ = Wolfram.

Üblicherweise erfolgt die thermische Behandlung im Luftstrom (z. B. in einem Drehrohrofen, wie er in der DE-A 103 25 487 beschrieben ist). Die Dauer der thermischen Behandlung erstreckt sich in der Regel auf einige Stunden.

Von den übrigen Bestandteilen der gewünschten Aktivmasse der allgemeinen Formel II bzw. III wird normalerweise ausgehend von in an sich bekannter Weise geeigneten Quellen (vgl. EP-A 835 und DE-C 33 38 380 sowie DE-A 44 07 020) in erfindungsgemäß zweckmäßiger Weise z. B. ein möglichst inniges, vorzugsweise feinteiliges Trockengemisch hergestellt (z. B. wasserlösliche Salze wie Halogenide, Nitrate, Acetate, Carbonate oder Hydroxide in einer wässrigen Lösung vereinen und anschließend die wässrige Lösung z. B. sprühtrocknen oder nicht wasserlösliche Salze, z. B. Oxide, in wässrigem Medium suspendieren und anschließend die Suspension z. B. sprühtrocknen), das hier als Ausgangsmasse 2 bezeichnet wird. Wesentlich ist nur, dass es sich bei den Bestandteilen der Ausgangsmasse 2 entweder bereits um Oxide handelt, oder um solche Verbindungen, die durch Erhitzen, gegebenenfalls in Anwesenheit von Sauerstoff, in Oxide überführbar sind. Anschließend werden die Ausgangsmasse 1 und die Ausgangsmasse 2 im gewünschten Mengenverhältnis in erfindungsgemäßer Weise, d.h. nach Zusatz von (u.a. erfindungsgemäßen) Formungs- und gegebenenfalls Verstärkungshilfsmitteln, zum ringförmigen Vollkatalysatorvorläuferformkörper formbaren Gemisch vermischt. Die Formung kann, wie bereits beschrieben, anwendungstechnisch zweckmäßig über die Stufe einer Zwischenkompaktierung erfolgen.

In einer weniger bevorzugten Ausführungsform kann das vorgebildete Mischoxid $Y^1_{a'}Y^2_{b'}O_{x'}$ bzw. $Bi_{a''}Z^2_{b''}O_{x''}$ mit Quellen der übrigen Bestandteile der gewünschten Aktivmasse auch in flüssigem, vorzugsweise wässrigem, Medium innig vermischt werden. Dieses Gemisch wird anschließend z. B. zu einem innigen Trockengemisch getrocknet und sodann, wie bereits beschrieben, geformt und thermisch behandelt. Dabei können die Quellen der übrigen Konstituenten in diesem flüssigen Medium gelöst und/oder suspendiert vorliegen, wohingegen das vorgebildete Mischoxid in diesem flüssigen Medium im wesentlichen unlöslich sein sollte, d. h., suspendiert vorliegen muss.

Die vorgebildeten Mischoxidpartikel sind im fertiggestellten ringförmigen Vollkatalysator in der durch die Klassierung eingestellten Längsausdehnung im wesentlichen unverändert enthalten.

Bevorzugt beträgt die spezifische Oberfläche von solchermaßen vorgebildeten Mischoxiden $Y^1_{a'}Y^2_{b'}O_{x'}$ bzw. $Bi_{a''}Z^2_{b''}O_{x''}$ 0,2 bis 2, besonders bevorzugt 0,5 bis 1,2 m²/g. Ferner resultiert das Porengesamtvolumen von solchermaßen vorgebildeten Mischoxiden vorteilhaft überwiegend von Mikroporen.

Vorteilhafte relevante ringförmige Vollkatalysatoren sind solche, deren spezifische Oberfläche O 5 bis 20 bzw. 15 m²/g, häufig 5 bis 10 m²/g beträgt. Das Porengesamtvolumen V solcher ringförmigen Vollkatalysatoren liegt dabei vorteilhaft im Bereich von 0,1 bis 1 bzw. 0,8 cm³/g, häufig im Bereich 0,2 bis 0,4 cm³/g.

In der Regel weisen erfindungsgemäß hergestellte Katalysatorformkörper eine begrenzte Erhöhung desjenigen Porendurchmessers $d^{max}$ auf, dessen diesen Durchmesser aufweisende Poren in ihrer Gesamtzahl zum Porengesamtvolumen des erfindungsgemäß hergestellten Katalysatorformkörpers den prozentual größten Beitrag leisten. Dies könnte ursächlich für die erfindungsgemäß beobachtete Erhöhung der mit erfindungsgemäß hergestellten Katalysatorformkörpern erzielten Zielproduktselektivität sein.

In typischer Weise betragen die Seitendruckfestigkeiten von wie beschrieben erhältlichen ringförmigen Vollkatalysatoren 5 bis 13 N, häufig 8 bis 11 N. Diese Seitendruckfestigkeiten von wie beschrieben erhältlichen ringförmigen Vollkatalysatoren liegen normalerweise auch dann vor, wenn die übrigen als vorteilhaft beschriebenen physikalischen Eigenschaften (z. B. O, V und Porendurchmesserverteilung) von wie beschrieben erhältlichen ringförmigen Vollkatalysatoren vorliegen.

Wie bereits erwähnt, eignen sich die wie beschrieben erhältlichen ringförmigen Vollkatalysatoren insbesondere als Katalysatoren für die Partialoxidation von Propen zu Acrolein bzw. von iso-Buten und/oder tert. Butanol zu Methacrolein. Die Partialoxidation kann dabei z. B. wie in den Schriften WO 00/53557, WO 00/53558, DE-A 199 10 506, EP-A 1 106 598, WO 01/36364, DE-A 199 27 624, DE-A 199 48 248, DE-A 199 48 523, DE-A 199 48 241, EP-A 700 714, DE-A 10313213, DE-A 103 13 209, DE-A 102 32 748, DE-A 103 13 208, WO 03/039744. EP-A 279 374, DE-A 33 38 380, DE-A 33 00 044, EP-A 575 897, DE-A 10 2004 003 212, DE-A 10 2005 013 039, DE-A 10 2005 009 891, DE-A 10 2005 010 111, DE-A 10 2005 009 885 sowie DE-A 44 07 020 beschrieben durchgeführt werden, wobei die Katalysatorbeschickung z. B. nur wie beschrieben erhältliche ringförmige Vollkatalysatoren oder z. B. mit inerten Formkörpern verdünnte ringförmige Vollkatalysatoren umfassen kann. Im letzteren Fall wird die Katalysatorbeschickung vorteilhaft in der Regel so gestaltet, dass ihre volumenspezifische Aktivität in Strömungsrichtung des Reaktionsgasgemisches kontinuierlich, sprunghaft und/oder stufenförmig zunimmt.

Dabei erweisen sich insbesondere die in dieser Schrift individualisiert hervorgehobenen Ringgeometrien der wie beschrieben erhältlichen Vollkatalysatoren dann als vorteilhaft, wenn die Belastung der Katalysatorbeschickung mit im Reaktionsgasausgangsgemisch enthaltenem Propen, iso-Buten und/oder tert. Butanol (bzw. dessen Methylether) $\geq$ 130 Nl/l Katalysatorbeschickung ·h beträgt (Vor- und/oder Nachschüttungen aus reinem Inertmaterial werden bei Belastungsbetrachtungen nicht als zur Katalysatorbeschickung gehörig betrachtet). Dies insbesondere dann, wenn auch die anderen in dieser Schrift als vorteilhaft beschriebenen physikalischen Eigenschaften von wie beschrieben erhältlichen ringförmigen Vollkatalysatoren gegeben sind.

Dieses vorteilhafte Verhalten von wie beschrieben erhältlichen ringförmigen Vollkatalysatoren, insbesondere den vorgenannten, liegt aber auch dann vor, wenn die vorgenannte Belastung der Katalysatorbeschickung $\geq$ 140 Nl/l·h, oder $\geq$ 150 Nl/l·h, oder $\geq$ 160 Nl/l·h beträgt. Im Normalfall wird die vorgenannte Belastung der Katalysatorbeschickung $\leq$ 600 Nl/l·h, häufig $\leq$ 500 Nl/l·h, vielfach $\leq$ 400 Nl/l·h oder $\leq$ 350 Nl/l·h betragen. Belastungen im Bereich von 160 Nl/l·h bis 300 bzw. 250 oder 200 Nl/l·h sind besonders typisch.

Selbstverständlich können die wie beschrieben erhältlichen ringförmigen Vollkatalysatoren als Katalysatoren für die Partialoxidation von Propen zu Acrolein bzw. von iso-Buten und/oder tert. Butanol (bzw. dessen Methylether) zu Methacrolein auch bei Belastungen der Katalysatorbeschickung mit der partiell zu oxidierenden Ausgangsverbindung von < 130 Nl/l·h, oder < 120 Nl/l·h, oder < 110 Nl/l·h, betrieben werden. In der Regel wird diese Belastung jedoch bei Werten > 60 Nl/l·h, oder > 70 Nl/l·h, oder $\geq$ 80 Nl/l·h liegen.

Prinzipiell kann die Belastung der Katalysatorbeschickung mit der partiell zu oxidierenden Ausgangsverbindung (Propen, iso-Buten und/oder tert. Butanol (bzw. dessen Methylether)) über zwei Stellschrauben eingestellt werden:

a) die Belastung der Katalysatorbeschickung mit Reaktionsgasausgangsgemisch; und/oder

b) den Gehalt des Reaktionsgasausgangsgemischs mit der partiell zu oxidierenden Ausgangsverbindung.

Die erfindungsgemäß erhältlichen ringförmigen Vollkatalysatoren eignen sich insbesondere auch dann, wenn bei oberhalb von 130 Nl/l·h liegenden Belastungen der Katalysatorbeschickung mit der partiell zu oxidierenden organischen Verbindung die Belastungseinstellung vor allem über die vorgenannte Stellschraube a) erfolgt.

Der Propenanteil (iso-Butenanteil bzw. tert. Butanolanteil (bzw. dessen Methyletheranteil)) im Reaktionsgasausgangsgemisch wird im Regelfall (d.h. im wesentlichen unabhängig von der Belastung) 4 bis 20 Vol.-%, häufig 5 bis 15 Vol.-%, oder 5 bis 12 Vol.-%, oder 5 bis 8 Vol.-% betragen (jeweils bezogen auf das Gesamtvolumen).

Häufig wird man das Verfahren der mit den wie beschrieben erhältlichen ringförmigen Vollkatalysatoren katalysierten Partialoxidation (im wesentlichen unabhängig von der Belastung) bei einem partiell zu oxidierende (organische) Verbindung (z.B. Propen): Sauerstoff: indifferente Gase (einschließlich Wasserdampf) Volumenverhältnis im Reaktionsgasausgangsgemisch von 1:(1,0 bis 3,0):(5 bis 25), vorzugsweise 1 :(1,5 bis 2,3):(10 bis 20) durchführen.

Unter indifferenten Gasen (oder auch Inertgasen) werden dabei solche Gase verstanden, die im Verlauf der Partialoxidation zu wenigstens 95 mol-%, vorzugsweise zu wenigstens 98 mol-% chemisch unverändert erhalten bleiben.

Bei den vorstehend beschriebenen Reaktionsgasausgangsgemischen kann das indifferente Gas zu $\geq$ 20 Vol.-%, oder zu $\geq$ 30 Vol.-%, oder zu $\geq$ 40 Vol.-%, oder zu $\geq$ 50 Vol.-%, oder zu $\geq$ 60 Vol.-%, oder zu $\geq$ 70 Vol.-%, oder zu $\geq$ 80 Vol.-%, oder zu $\geq$ 90 Vol.-%, oder zu $\geq$ 95 Vol.-% aus molekularem Stickstoff bestehen.

Bei Belastungen der Katalysatorbeschickung mit der partiell zu oxidierenden organischen Verbindung von $\geq$ 150 Nl/l·h ist jedoch die Mitverwendung von inerten Verdünnungsgasen wie Propan, Ethan, Methan, Pentan, Butan, $CO_2$, CO, Wasserdampf und/oder Edelgasen für das Rektionsgasausgangsgemisch empfehlenswert. Generell können diese inerten Gase und ihre Gemische aber auch bereits bei geringeren erfindungsgemäßen Belastungen der Katalysatorbeschickung mit der partiell zu oxidierenden organischen Verbindung eingesetzt werden. Auch kann Kreisgas als Verdünnungsgas mitverwendet werden. Unter Kreisgas wird das Restgas verstanden, das verbleibt, wenn man aus dem Produktgasgemisch der Partialoxidation die Zielverbindung im wesentlichen selektiv abtrennt. Dabei ist zu berücksichtigen, dass die Partialoxidationen zu Acrolein oder Methacrolein mit den erhältlichen ringförmigen Vollkatalysatoren nur die erste Stufe einer zweistufigen Partialoxidation zu Acrylsäure oder Methacrylsäure als den eigentlichen Zielverbindungen sein können, so dass die Kreisgasbildung dann meist erst nach der zweiten Stufe erfolgt. Im Rahmen einer solchen zweistufigen Partialoxidation wird in der Regel das Produktgasgemisch der ersten Stufe als solches, gegebenenfalls nach Abkühlung und/oder Sekundärsauerstoffzugabe, der zweiten Partialoxidationsstufe zugeführt.

Bei der Partialoxidation von Propen zu Acrolein, unter Anwendung der wie beschrieben erhältlichen ringförmigen Vollkatalysatoren, kann eine typische Zusammensetzung des Reaktionsgasausgangsgemischs gemessen am Reaktoreingang (unabhängig von der gewählten Belastung) z.B. die nachfolgenden Komponenten enthalten:

| | |
|---|---|
| 6 bis 6,5 Vol.-% | Propen, |
| 1 bis 3,5 Vol.-% | $H_2O$, |
| 0,2 bis 0,5 Vol.-% | CO, |
| 0,6 bis 1,2 Vol.-% | $CO_2$, |
| 0,015 bis 0,04 Vol.-% | Acrolein, |
| 10,4 bis 11,3 Vol.-% | $O_2$ und |

als Restmenge ad 100% molekularer Stickstoff, oder:

| | |
|---|---|
| 5,6 Vol.-% | Propen, |
| 10,2 Vol.-% | Sauerstoff, |
| 1,2 Vol.-% | $CO_x$, |
| 81,3 Vol.-% | $N_2$ und |
| 1,4 Vol.-% | $H_2O$. |

Erstere Zusammensetzungen eignen sich insbesondere bei Propenbelastungen von $\geq$ 130 Nl/l·h und letztere Zusammensetzung insbesondere bei Propenbelastungen < 130 Nl/l·h, insbesondere $\leq$ 100 Nl/l·h.

Als alternativen Zusammensetzungen des Reaktionsgasausgangsgemischs kommen (unabhängig von der gewählten Belastung) solche in Betracht, die nachfolgende Komponentengehalte aufweisen:

| | |
|---|---|
| 4 bis 25 Vol.-% | Propylen, |
| 6 bis 70 Vol.-% | Propan, |
| 5 bis 60 Vol.-% | $H_2O$, |
| 8 bis 65 Vol.-% | $O_2$, und |
| 0,3 bis 20 Vol.-% | $H_2$; |

oder

| | |
|---|---|
| 4 bis 25 Vol.-% | Propylen, |
| 6 bis 70 Vol.-% | Propan, |
| 0 bis 60 Vol.-% | $H_2O$, |
| 8 bis 16 Vol.-% | $O_2$, |
| 0 bis 20 Vol.-% | $H_2$, |
| 0 bis 0,5 Vol.-% | CO, |
| 0 bis 1,2 Vol.-% | $CO_2$, |
| 0 bis 0,04 Vol.-% | Acrolein, |

und als Restmenge ad 100 Vol.-% im wesentlichen $N_2$;
oder

| | |
|---|---|
| 50 bis 80 Vol.-% | Propan, |
| 0,1 bis 20 Vol.-% | Propylen, |
| 0 bis 10 Vol.-% | $H_2$, |
| 0 bis 20 Vol.-% | $N_2$, |

|  |  |
|---|---|
| 5 bis 15 Vol.-% | H$_2$O, |

soviel molekularer Sauerstoff, dass das molare Verhältnis von Sauerstoffgehalt zu Propylengehalt 1,5 bis 2,5 beträgt. oder

| 6 bis 9 Vol.-% | Propylen, |
|---|---|
| 8 bis 18 Vol.-% | molekularer Sauerstoff, |
| 6 bis 30 Vol.-% | Propan, und |
| 32 bis 72 Vol.-% | molekularer Stickstoff. |

Das Reaktionsgasausgangsgemisch kann aber auch wie folgt zusammengesetzt sein:

| 4 bis 15 Vol.-% | Propen, |
|---|---|
| 1,5 bis 30 Vol.-% | (häufig 6 bis 15 Vol.-%) Wasser, |
| ≥ 0 bis 10 Vol.-% | (vorzugsweise ≥ 0 bis 5 Vol.-%) von Propen, |

Wasser, Sauerstoff und Stickstoff verschiedenen Bestandteilen, soviel molekularem Sauerstoff, dass das molare Verhältnis von enthaltenem molekularem Sauerstoff zu enthaltenem molekularem Propen 1,5 bis 2,5 beträgt und als Restmenge bis zur 100 Vol.-% Gesamtmenge aus molekularem Stickstoff.

Eine andere mögliche Reaktionsgasausgangsgemischzusammensetzung kann enthalten:

| 6,0 Vol.-% | Propen, |
|---|---|
| 60 Vol.-% | Luft und |
| 34 Vol.-% | H$_2$O. |

Alternativ können auch Reaktionsgasausgangsgemische der Zusammensetzung gemäß Example 1 der EP-A 990 636, oder gemäß Example 2 der EP-A 990 636, oder gemäß Example 3 der EP-A 1 106 598, oder gemäß Example 26 der EP-A 1 106 598, oder gemäß Example 53 der EP-A 1 106 598 verwendet werden.

Auch eignen sich die wie beschrieben erhältlichen ringförmigen Katalysatoren für die Verfahren der DE-A 10246119 bzw. DE-A 10245585.

Weitere erfindungsgemäß geeignete Reaktionsgasausgangsgemische können im nachfolgenden Zusammensetzungsraster liegen:

| 7 bis 11 Vol.-% | Propen, |
|---|---|
| 6 bis 12 Vol.-% | Wasser, |
| ≥ 0 bis 5 Vol.-% | von Propen, Wasser, Sauerstoff und Stickstoff verschiedenen Bestandteilen, |

soviel molekularem Sauerstoff, dass das molare Verhältnis von enthaltenem Sauerstoff zu enthaltenem molekularem Propen 1,6 bis 2,2 beträgt, und als Restmenge bis zur 100 Vol.-% Gesamtmenge molekularer Stickstoff.

Im Fall von Methacrolein als Zielverbindung kann das Reaktionsgasausgangsgemisch insbesondere auch wie in der DE-A 44 07 020 beschrieben zusammengesetzt sein.

Die Reaktionstemperatur für die Propenpartialoxidation liegt bei Verwendung der wie beschrieben erhältlichen ringförmigen Vollkatalysatoren häufig bei 300 bis 380°C. Das gleiche trifft im Fall von Methacrolein als Zielverbindung zu.

Der Reaktionsdruck liegt für die vorgenannten Partialoxidationen in der Regel bei 0,5 bzw. 1,5 bis 3 bzw. 4 bar (gemeint sind in dieser Schrift, soweit nicht ausdrücklich anders erwähnt, Absolutdruckes).

Die Gesamtbelastung der Katalysatorbeschickung mit Reaktionsgasausgangsgemisch beläuft sich bei den vorgenannten Partialoxidationen typisch auf 1000 bis 10000 Nl/l·h, meist auf 1500 bis 5000 Nl/l·h und oft auf 2000 bis 4000 Nl/l·h.

Als im Reaktionsgasausgangsgemisch zu verwendendes Propen kommen vor allem polymer grade Propen und chemical grade Propen in Betracht, wie es z. B. die DE-A 102 32 748 beschreibt.

Als Sauerstoffquelle wird normalerweise Luft eingesetzt.

Die Partialoxidation in Anwendung der wie beschrieben erhältlichen ringförmigen Vollkatalysatoren kann im einfachsten Fall z. B. in einem Einzonen-Vielkontaktrohr-Festbettreaktor durchgeführt werden, wie ihn die DE-A 44 31 957, die EP-A 700 714 und die EP-A 700 893 beschreiben.

Üblicherweise sind in den vorgenannten Rohrbündelreaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Eine typische Kontaktrohrlänge beläuft sich z. B. auf 3,20 m. Anwendungstechnisch zweckmäßig beläuft sich die im Rohrbündelbehälter untergebrachte Anzahl an Kontaktrohren auf wenigstens 5000, vorzugsweise auf wenigstens 1000. Häufig beträgt die Anzahl der im Reaktionsbehälter untergebrachten Kontaktrohre 15000 bis 35000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. EP-B 468 290).

Die Partialoxidation kann aber auch in einem Mehrzonen (z. B. "Zwei-Zonen")-Vielkontaktrohr-Festbettreaktor durchgeführt werden, wie es die DE-A 199 10 506, die DE-A 103 13 213, die DE-A 103 13 208 und die EP-A 1 106 598 insbesondere bei erhöhten Belastungen der Katalysatorbeschickung mit der partiell zu oxidierenden organischen Verbindung empfehlen. Eine typische Kontaktrohrlänge im Fall eines Zweizonen-Vielkontaktrohr-Festbettreaktors beträgt 3,50 m. Alles andere gilt im wesentlichen wie beim Einzonen-Vielkontaktrohr-Festbettreaktor beschrieben. Um die Kontaktrohre, innerhalb derer sich die Katalysatorbeschickung befindet, wird in jeder Temperierzone ein Wärmeaustauschmittel geführt. Als solche eignen sich z. B. Schmelzen von Salzen wie Kaliumnitrat, Kaliumnitrit, Natriumnitrit und/oder Natriumnitrat, oder von niedriger schmelzenden Metallen wie Natrium, Quecksilber sowie Legierungen verschiedener Metalle. Die Fließgeschwindigkeit des Wärmeaustauschmittels innerhalb der jeweiligen Temperierzone wird in der Regel so gewählt, dass die Temperatur des Wärmeaustauschmittels von der Eintrittsstelle in die Temperaturzone bis zur Austrittsstelle aus der Temperaturzone um 0 bis 15 °C, häufig 1 bis 10 °C, oder 2 bis 8 °C, oder 3 bis 6 °C ansteigt.

Die Eingangstemperatur des Wärmeaustauschmittels, das, über die jeweilige Temperierzone betrachtet, im Gleichstrom oder im Gegenstrom zum Reaktionsgasgemisch geführt werden kann, wird vorzugsweise wie in den Schriften EP-A 1 106 598, DE-A 199 48 523, DE-A 199 48 248, DE-A 103 13 209, EP-A 700 714, DE-A 103 13 208, DE-A 103 13 213, WO 00/53557, WO 00/53558, WO 01/36364, WO 00/53557 sowie den anderen in dieser Schrift als Stand der Technik zitierten Schriften empfohlen gewählt. Innerhalb der Temperierzone wird das Wärmeaustauschmittel bevorzugt mäanderförmig geführt. In der Regel verfügt der Vielkontaktrohr-Festbettreaktor zusätzlich über Thermorohre zur Bestimmung der Gastemperatur im Katalysatorbett. In zweckmäßiger Weise wird der Innendurchmesser der Thermorohre und der Durchmesser der innenliegenden Aufnahmehülse für das Thermoelement so gewählt, dass das Verhältnis von Reaktionswärme entwickelndem Volumen zu Wärme abführender Oberfläche bei Thermorohren und Arbeitsrohren gleich oder nur geringfügig unterschiedlich ist.

Der Druckverlust sollte bei Arbeitsrohren und Thermorohren, bezogen auf gleiche GHSV, gleich sein. Ein Druckverlustangleich beim Thermorohr kann durch Zusatz von versplittetem Katalysator zu den Katalysatorformkörpern erfolgen. Dieser Ausgleich erfolgt zweckmäßig über die gesamte Thermorohrlänge homogen.

Zur Bereitung der Katalysatorbeschickung in den Kontaktrohren können, wie bereits erwähnt, nur wie beschrieben erhältliche ringförmige Vollkatalysatoren oder z.B. auch weitgehend homogene Gemische aus wie beschrieben erhältlichen ringförmigen Vollkatalysatoren und keine Aktivmasse aufweisenden, sich bezüglich der heterogen katalysierten partiellen Gasphasenoxidation im wesentlichen inert verhaltenden Formkörpern verwendet werden. Als Materialien für solche inerten Formkörper kommen z. B. poröse oder unporöse Aluminiumoxide, Siliciumdioxid, Zirkondioxid, Siliciumcarbid, Silikate wie Magnesium- oder Aluminiumsilikat oder Steatit (z.B. vom Typ C220 der Fa. CeramTec, DE) in Betracht.

Die Geometrie solcher inerter Verdünnungsformkörper kann im Prinzip beliebig sein. D.h., es können beispielsweise Kugeln, Polygone, Vollzylinder oder auch, wie die Katalysatorformkörper, Ringe sein. Häufig wird man als inerte Verdünnungsformkörper solche wählen, deren Geometrie derjenigen der mit ihnen zu verdünnenden Katalysatorformkörper entspricht. Längs der Katalysatorbeschickung können aber auch die Geometrie des Katalysatorformkörpers gewechselt oder Katalysatorformkörper unterschiedlicher Geometrie in weitgehend homogener Abmischung eingesetzt werden. In einer weniger bevorzugten Vorgehensweise kann auch die Aktivmasse des Katalysatorformkörpers längs der Katalysatorbeschickung verändert werden.

Ganz generell wird, wie bereits erwähnt, die Katalysatorbeschickung mit Vorteil so gestaltet, dass die volumenspezifische (d. h., die auf die Einheit des Volumens normierte) Aktivität in Strömungsrichtung des Reaktionsgasgemisches entweder konstant bleibt oder zunimmt (kontinuierlich, sprunghaft oder stufenförmig).

Eine Verringerung der volumenspezifischen Aktivität kann in einfacher Weise z. B. dadurch erzielt werden, dass man eine Grundmenge von erfindungsgemäß einheitlich hergestellten ringförmigen Vollkatalysatoren mit inerten Verdünnungsformkörpern homogen verdünnt. Je höher der Anteil der Verdünnungsformkörper gewählt wird, desto geringer ist die in einem bestimmten Volumen der Beschickung enthaltene Aktivmasse bzw. Katalysatoraktivität. Eine Verringerung kann aber auch dadurch erzielt werden, dass man die Geometrie der erfindungsgemäß erhältlichen ringförmigen Vollkatalysatoren so verändert, dass die in der Einheit des Ringgesamtvolumens (einschließlich der Ringöffnung) enthaltene Aktivmassenmenge kleiner wird.

Für die heterogen katalysierten Gasphasenpartialoxidationen mit den wie beschrieben erhältlichen ringförmigen Vollkatalysatoren wird die Katalysatorbeschickung vorzugsweise entweder auf der gesamten Länge einheitlich mit nur einem Vollkatalysatorring gestaltet oder wie folgt strukturiert. Am Reaktoreingang wird auf einer Länge von 10 bis 60 %, bevorzugt 10 bis 50 %, besonders bevorzugt 20 bis 40 % und ganz besonders bevorzugt 25 bis 35 % (d. h., z. B. auf einer Länge von 0,70 bis 1,50 m, bevorzugt 0,90 bis 1,20 m), jeweils der Gesamtlänge der Katalysatorbeschickung, ein im wesentlichen homogenes Gemisch aus erfindungsgemäß erhältlichem ringförmigem Vollkatalysator und inertem Verdünnungsformkörper (wobei beide vorzugsweise im wesentlichen die gleiche Geometrie aufweisen) platziert, wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diesen ersten Beschickungsabschnitt befindet sich dann vorteilhaft bis zum Ende der Länge der Katalysatorbeschickung (d. h., z. B. auf einer Länge von 1,00 bis 3,00 m oder von 1,00 bis 2,70 m, bevorzugt 1,40 bis 3,00 m, oder 2,00 bis 3,00 m) entweder eine nur in geringerem Umfang (als im ersten Abschnitt) verdünnte Schüttung des wie beschrieben erhältlichen ringförmigen Vollkatalysators, oder, ganz besonders bevorzugt, eine alleinige (unverdünnte) Schüttung desselben ringförmigen Vollkatalysators, der auch im ersten Abschnitt verwendet worden ist. Natürlich kann über die gesamte Beschickung auch eine konstante Verdünnung gewählt werden. Auch kann im ersten Abschnitt nur mit einem erfindungsgemäß erhältlichen ringförmigen Vollkatalysator von geringer, auf seinen Raumbedarf bezogener, Aktivmassendichte und im zweiten Abschnitt mit einem erfindungsgemäß erhältlichen ringförmigen Vollkatalysator mit hoher, auf seinen Raumbedarf bezogener, Aktivmassendichte beschickt werden (z.B. 6,5 mm x 3 mm x 4,5 mm [A x H x I] im ersten Abschnitt, und 5 x 2 x 2 mm im zweiten Abschnitt).

Insgesamt werden bei einer mit den wie beschrieben erhältlichen ringförmigen Vollkatalysatoren als Katalysatoren durchgeführten Partialoxidation zur Herstellung von Acrolein oder Methacrolein die Katalysatorbeschickung, das Reaktionsgasausgangsgemisch, die Belastung und die Reaktionstemperatur in der Regel so gewählt, dass beim einmaligen Durchgang des Reaktionsgasgemischs durch die Katalysatorbeschickung ein Umsatz der partiell zu oxidierenden organischen Verbindung (Propen, iso-Buten, tert-Butanol bzw. dessen Methylether) von wenigstens 90 mol-%, oder 92 mol-%, vorzugsweise von wenigstens 94 mol-% resultiert. Die Selektivität der Acrolein- bzw. Methacroleinbildung wird dabei regelmäßig ≥ 80 mol-%, bzw. ≥85 mol-% betragen. In natürlicher Weise werden dabei möglichst geringe Heißpunkttemperaturen angestrebt.

Insgesamt bedingen dabei die wie beschrieben erhältlichen ringförmigen Vollkatalysatoren eine erhöhte Selektivität der Zielproduktbildung.

Abschließend sei festgehalten, das die wie beschrieben erhältlichen ringförmigen Vollkatalysatoren auch ein vorteilhaftes Bruchverhalten bei der Reaktorbefüllung aufweisen. Auch ihr Druckverlustverhalten ist vorteilhaft. Im übrigen eignen sich die wie beschrieben erhältlichen ringförmigen Vollkatalysatoren ganz generell als Katalysatoren mit erhöhter Selektivität für gasphasenkatalytische Partialoxidationen organischer Verbindungen wie niederer (z. B. 3 bis 6 (d. h. 3, 4, 5, oder 6) C-Atome enthaltender) Alkane (insbesondere Propan), Alkanole, Alkanale, Alkene und Alkenale zu olefinisch ungesättigten Adehyden und/oder Carbonsäuren, sowie den entsprechenden Nitrilen (Ammoxidation, vor allem von Propen zu Acrylnitril und von 2-Methylpropen bzw. tert.-Butanol (bzw. dessen Methylether) zu Methacrylnitril) sowie für gasphasenkatalytisch oxidative Dehydrierungen organischer Verbindungen (z. B. 3, 4, 5, oder 6 C-Atome enthaltender).

Für das Verfahren der Propylenpartialoxidation zu Acrolein besonders vorteilhafte Stöchiometrien sind:

a) $[Bi_2W_2O_9 \times 2WO_3]_{0,5}[Mo_{12}Co_{5,5}Fe_{2,94}Si_{1,59}K_{0,08}O_x]_1$;
b) $Mo_{12}Ni_{6,5}Zn_2Fe_2Bi_1P_{0,0065}K_{0,06}O_x \cdot 10SiO_2$;
c) $MO_{12}Co_7Fe_{2,94}Bi_{0,6}Si_{1,59}K_{0,08}O_x$;
d) wie Multimetalloxid II-Vollkatalysator gemäß Beispiel 1 der DE-A 197 46 210; und
e) Wie Beispiel 1c aus der EP-A 015 565.

Der Wismutgehalt der beschriebenen Aktivmassen kann auch wie in der DE-A 100 63 162 beschrieben eingestellt werden. Dabei wird aus Ausgangsverbindungen der elementaren Konstituenten der angestrebten Aktivmasse eine Lösung oder Suspension erzeugt, die zwar die zur Herstellung der Aktivmasse erforderliche Gesamtmenge der von Bi verschiedenen elementaren Konstituenten, aber nur eine Teilmenge des zur Herstellung der Aktivmasse erforderlichen Bi enthält, die Lösung oder Suspension unter Erhalt einer Trockenmasse getrocknet und die zur Herstellung der Aktivmasse zusätzlich benötigte Restmenge an Bi in Form einer Ausgangsverbindung des Bi wie in der DE-A 100 63 162 beschrieben unter Erhalt eines formbaren Gemischs in diese Trockenmasse eingearbeitet (z. B. wie im Beispiel der DE-A 100 63 162), das formbare Gemisch in erfindungsgemäßer Weise (d. h. nach Zugabe von Formungs- und gegebenenfalls Verstärkungshilfsmitteln) zu einem ringförmigen Vollkatalysatorformkörper geformt und dieser durch thermisches Behandeln (z. B. wie im Beispiel der DE-A 100 63 162) in den gewünschten ringförmigen Vollkatalysator überführt. Die Stöchiometrien (insbesondere der Ausführungsbeispiele) und thermischen Behandlungsbedingungen dieser (vorgenannten) Schrift sind für die Propylenpartialoxidation zu Acrolein gleichfalls besonders geeignet. Dies gilt insbesondere für die Stöchiometrie $Mo_{12}Bi_{1,0}Fe_3Co_7Si_{1,6}K_{0,08}$.

Die Inbetriebnahme einer frischen Katalysatorbeschickung mit wie beschrieben erhältlichen ringförmigen Vollkatalysatoren kann wie in der DE-A 10337788 beschrieben erfolgen. In der Regel nehmen Aktivität und Selektivität der Zielproduktbildung anfänglich mit der Betriebsdauer der Katalysatorbeschickung zu. Diese Formierung kann dadurch beschleunigt werden, dass man sie bei im wesentlichen gleichbleibendem Umsatz unter erhöhter Belastung der Katalysatorbeschickung mit Reaktionsgasausgangsgemisch durchführt und nach weitgehend vollzogener Formierung die Belastung auf ihren Zielwert zurücknimmt.

Weiterhin umfasst die vorliegende Erfindung im besonderen ein Verfahren zur Herstellung von ringförmigen Katalysatorformkörpern mit gekrümmter und/oder nicht gekrümmter Stirnfläche der Ringe, deren Aktivmasse eine Stöchiometrie der allgemeinen Formel IV,

$$Mo_{12}P_aV_bX_c^1X_d^2X_e^3Sb_fRe_gS_hO_n \qquad (IV),$$

aufweist, in der Variablen folgende Bedeutung haben:

$X^1 =$ Kalium, Rubidium und/oder Cäsium,
$X^2 =$ Kupfer und/oder Silber,
$X^3 =$ Cer, Bor, Zirkonium, Mangan und/oder Wismut,
$a =$ 0,5 bis 3,
$b =$ 0,01 bis 3,
$c =$ 0,2 bis 3,
$d =$ 0,01 bis 2,
$e =$ 0 bis 2,
$f =$ 0,01 bis 2,
$g =$ 0 bis 1,
$h =$ 0,001 bis 0,5 und
$n =$ eine Zahl, die durch die Wertigkeit und Häufigkeit der von Sauerstoff verschiedenen Elemente in IV bestimmt wird,

und deren ringförmige Geometrie jener der bereits beschriebenen ringförmigen Katalysatorformkörper mit Aktivmassen einer Stöchiometrie der allgemeinen Formel (I), (II), oder (III) entspricht.

Bevorzugt sind Aktivmassen IV, in denen h 0,03 bis 0,5 ist.

Besonders bevorzugte Stöchiometrie der allgemeinen Formel IV sind jene der Ausführungsbeispiele B1 bis B15 aus der EP-A 467 144 und dies auch dann, wenn diese beispielhaften Aktivmassen kein K und/oder kein Re enthalten.

Vorgenannte EP-A 467 144 beschreibt auch die Herstellung solcher ringförmigen Katalysatorformkörper und ihre Verwendung als Katalysatoren für die heterogen katalysierte Gasphasenpartialoxidation von Methacrolein zu Methacrylsäure. Diese Beschreibungen sind auch im in der vorliegenden Anmeldung gegebenen Kontext zutreffend, sieht man davon ab, dass bei der Herstellung der ringförmigen Katalysatorformkörper erfindungsgemäß zu verwendender Graphit als Gleitmittel einzusetzen ist.

D.h., ringförmige Katalysatorformkörper mit Aktivmassen der allgemeinen Stöchiometrie IV können dadurch hergestellt werden, dass man als Ausgangsverbindungen geeignete Salze der sie konstituierenden elementaren Bestandteile, gegebenenfalls bei erhöhter Temperatur und unter Zusatz von Säuren oder Basen, in wässrigem Medium durch Lösen und/oder Suspendieren fein verteilt und, zur Vermeidung unerwünschter Oxidationsprozesse ggf. unter Inertgas, mischt, das Gemisch trocknet (z. B. eindampft oder sprühtrocknet), der resultierenden, feinteilige Form aufweisenden oder in feinteilige Form überführten Trockenmasse das erfindungsgemäß erforderliche Graphit sowie gegebenenfalls sonstige der genannten Formungshilfsmittel und Verstärkungsmittel zusetzt, die dabei erhaltene feinteilige Masse zur gewünschten Ringgeometrie formt (verdichtet) und die dabei resultierenden Katalysatorvorläuferformkörper anschließend thermisch behandelt. Bevorzugt wird die thermische Behandlung bei Temperaturen von 180 bis 480°C, besonders bevorzugt bei Temperaturen von 250 bis 450 °C durchgeführt. Die thermische Behandlung kann dabei unter den bereits beschriebenen Gasatmosphären erfolgen. Beispielhaft erwähnt seien nochmals strömende Luft, strömende Inertatmosphäre (z. B. $N_2$, oder $CO_2$, oder Edelgase) oder Vakuum. Die thermische Behandlung kann in mehreren Temperaturstufen und/oder in verschiedenen Atmosphären durchgeführt werden. So kann z. B. in einer ersten Stufe bei 200 bis 260 °C in Luft, in einer zweiten Stufe bei 420 bis 460 °C in Stickstoff und in einer dritten Stufe bei 350 bis 410 °C wiederum in Luft thermisch behandelt werden. Im Regelfall ist strömende Luft die bevorzugte Atmosphäre für die thermische Behandlung.

Im übrigen gilt das bei der Herstellung von ringförmigen Katalysatorformkörpern der Aktivmassen (I), (II) und (III) gesagte hier in entsprechender Weise, jedoch mit dem Unterschied, dass hier die erhöhten Seitendruckfestigkeiten für die ringförmigen Vollkatalysatorvorläuferformkörper bevorzugt werden.

D. h., z. B. bevorzugtes Trocknungsverfahren für die wässrige Lösung oder Suspension der Quellen der elementaren Konstituenten der gewünschten Aktivmasse ist die Sprühtrocknung. Das resultierende Sprühpulver mit einem Partikeldurchmesser $d_{50}$ zwischen 10 bis 50 $\mu$m wird erfindungsgemäß vorteilhaft und erfindungsgemäß zweckmäßig nach Zusatz von feinteiligem erfindungsgemäßem Graphit als Hilfsmittel, zwischenkompaktiert, um das Pulver zu vergröbern. Bevorzugt erfolgt die Zwischenkompaktierung hier auf Partikeldurchmesser von 100 bis 2000 $\mu$m, bevorzugt von 150 bis 1500 $\mu$m und besonders bevorzugt von 400 bis 1000 $\mu$m. Anschließend erfolgt auf der Grundlage des vergröberten Pulvers die eigentliche Formgebung, wobei bei Bedarf zuvor nochmals feinteiliges erfindungsgemäßes Graphit (sowie gegebenenfalls weiterer Formungs- und/oder Verstärkungshilfsmittel) zugegeben werden kann. Das bei der Herstellung der ringförmigen Katalysatorformkörper der Aktivmassen (I), (II) und (III) zu den Seitendruckfestigkeiten gesagte gilt hier analog.

Bei der beschriebenen Herstellungsweise von ringförmigen Katalysatorformkörpern aus Aktivmassen der allgemeinen Formel IV wird Antimon üblicherweise als Antimontrioxid, Rhenium z. B. als Rhenium(VII)oxid, Molybdän vorzugsweise als Ammoniumsalz der Molybdän- oder Phosphormolybdänsäure, Bor z. B. als Borsäure, Vanadin in der Regel als Ammoniumvanadat oder Vanadinoxalat, Phosphor mit Vorteil als orthoPhosphorsäure oder Di-Ammonium-Phosphat, Schwefel z.B. als Ammoniumsulfat und die kationischen Metalle normalerweise als Nitrate, Oxide, Hydroxide, Carbonate, Chloride, Formiate, Oxalate und/oder Acetate bzw. deren Hydrate eingesetzt. Bevorzugte Ringgeometrie des fertiggestellten Katalysatorformkörpers ist hier die Geometrie 7mm x 7mm x 3mm (Außendurchmesser x Höhe x Innendurchmesser). Die katalytische Gasphasenoxidation von Methacrolein zu Methacrylsäure unter Anwendung der wie beschrieben erhältlichen ringförmigen Katalysatorformkörper kann in an sich bekannter, z. B. in der EP-A 467 144 beschriebener, Weise erfolgen. Das Oxidationsmittel Sauerstoff kann z.B. in Form von Luft, aber auch in reiner Form eingesetzt werden. Aufgrund der hohen Reaktionswärme werden die Reaktionspartner vorzugsweise mit Inertgasen wie $N_2$, CO, $CO_2$ und/oder mit Wasserdampf verdünnt. Vorzugsweise wird bei einem Methacrolein : Sauerstoff: Wasserdampf: Inertgas Verhältnis von 1 : (1 bis 3) : (2 bis 20) : (3 bis 30), besonders bevorzugt von 1 : (1 bis 3) : (3 bis 10) : (7 bis 18) gearbeitet. Der Anteil des Methacroleins am Reaktionsgasausgangsgemisch beläuft sich dabei in der Regel auf 4 bis 11, vielfach 4,5 bis 9 Vol.-%. Der Sauerstoffgehalt wird zur Vermeidung explosiver Gemische bevorzugt auf $\leq$ 12,5 Vol.-% beschränkt. Dies wird besonders bevorzugt durch Rückführung eines Teilstroms des vom Reaktionsprodukt abgetrennten Abgases erreicht. Im übrigen erfolgt die Gasphasenpartialoxidation zur Methacrylsäure in typischer Weise bei Gesamtraumbelastungen des Katalysatorfestbetts von 600 bis 1800 Nl/l•h, bzw. bei Methacroleinbelastungen von 40 bis 100 Nl/l•h. Als Reaktor wird in der Regel ein Rohrbündelreaktor eingesetzt. Reaktionsgas und Salzbad können dabei über den Reaktor betrachtet sowohl im Gleichstrom als auch im Gegenstrom geführt werden. Das Salzbad selbst wird normalerweise in mäandrierender Form durch den Reaktor geführt. Bevorzugtes Graphit zur Herstellung von ringförmigen Katalysatorformkörpern aus Aktivmassen der allgemeinen Stöchiometrie IV ist Asbury 3160 und/oder Asbury 4012.

Das erfindungsgemäße Verfahren umfasst weiterhin insbesondere ein Verfahren zur Herstellung von ringförmigen Katalysatorformkörpern mit gekrümmter und/oder nicht gekrümmter Stirnfläche der Ringe, deren Aktivmasse ein Vanadium, Phosphor und Sauerstoff enthaltendes Multielementoxid ist, und die sich als Katalysatoren für die heterogen katalysierte Gasphasenoxidation wenigstens eines Kohlenwasserstoffs mit mindestens vier Kohlenstoffatomen (insbesondere n-Butan, n-Butene und/oder Benzol) zu Maleinsäureanhydrid eignen. Die Stöchiometrie der Multielementoxidmasse kann dann z. B. eine solche der allgemeinen Formel V sein,

$$V_1 P_b Fe_c X^1_d X^2_e O_n \qquad (V),$$

in der die Variablen folgende Bedeutung aufweisen:

$X^1$ = Mo, Bi, Co, Ni, Si, Zn, Hf, Zr, Ti, Cr, Mn, Cu, B, Sn und/oder Nb,
$X^2$ = K, Na, Rb, Cs und/oder TI,
b = 0,9 bis 1,5,
c = 0 bis 0,1,
d = 0 bis 0,1,
e = 0 bis 0,1, und
n = eine Zahl, die durch die Wertigkeit und Häufigkeit der von Sauerstoff verschiedenen Elemente in V bestimmt wird.

Die Herstellung entsprechender ringförmiger Katalysatorformkörper ist in der WO 03/078310 unter Zusatz von nicht näher spezifiziertem Graphit als Formungshilfsmittel beschrieben. Alle Ausführungen der WO 03/078310 und alle in der WO 03/078310 angesprochenen Katalysatoren besitzen auch dann Tragfähigkeit und die in der WO 03/078310 angesprochene Anwendbarkeit, wenn die in der WO 03/078310 offenbarten Herstellmaßnahmen beibehalten werden, und das im Rahmen der Herstellung mitverwendete Graphit durch gewichtsidentische Mengen an erfindungsgemäßem Graphit ersetzt wird. Allerdings ist die Selektivität der Zielproduktbildung eine erhöhte. D.h. auch im vorgenannten Fall der Vanadium, Phosphor und Sauerstoff enthaltenden ringförmigen Multielementoxidkatalysatoren stellen sich die erfindungsgemäßen Vorteile ein. Dies gilt im Besonderen für alle Ausführungsbeispiele der WO 03/078310. Durch Erhöhung des Graphitanteils (der Graphiteinsatzmenge) auf 5 bis 35 Gew.-% können dessen porenbildende Eigenschaften gegebenenfalls verstärkt und die Selektivität oder Zielproduktbildung weiter erhöht werden.

Das vorstehend für die Vanadium, Phosphor und Sauerstoff enthaltenden Multimetalloxidkatalysatoren der WO 03/078310 und deren Verwendung gesagte gilt in entsprechender Weise für jene der WO 01/68245 sowie für jene der DE-A 10 2005 035 978. Letztere sind erfindungsgemäß damit wie folgt herstellbar:

a) Umsetzung einer fünfwertigen Vanadiumverbindung (z. B. $V_2O_5$) mit einem organischen, reduzierenden Lösungsmittel (z. B. iso-Butanol) in Gegenwart einer fünfwertigen Phosphorverbindung (z. B. Ortho- und/oder Pyrophosphorsäure) unter Erwärmen auf 75 bis 205 °C, bevorzugt auf 100 bis 120 °C;

b) Abkühlen des Reaktionsgasgemisches auf vorteilhaft 40 bis 90 °C;

c) Zugabe von Eisen(III)phosphat;

d) Erneutes Erwärmen auf 75 bis 205°C, bevorzugt 100 bis 120 °C;

e) Isolierung der gebildeten Vanadium-, Phosphor-, Eisen- und Sauerstoff enthaltenden festen Vorläufermasse (z. B. durch Filtrieren);

f) Trocknung und/oder thermische Vorbehandlung der Vorläufermasse (gegebenenfalls bis zu beginnender Vorformierung durch Wasserabspaltung aus der Vorläufermasse);

g) Erfindungsgemäße Zugabe von Graphit und Formgebung durch Überführung in eine beispielsweise kugelförmige, ringförmige oder zylinderförmige Struktur;

h) Thermische Behandlung der gebildeten Katalysatorvorläuferformkörper durch Erhitzen in einer Atmosphäre, welche Sauerstoff, Stickstoff, Edelgase, Kohlendioxid, Kohlenmonoxid und/oder Wasserdampf enthält, z. B. wie in der WO 03078310 auf Seite 20, Zeile 16 bis Seite 21, Zeile 35 beschrieben.

Das erfindungsgemäße Verfahren umfasst weiterhin insbesondere Verfahren zur Herstellung von erfindungsgemäßen, z. B. kugelförmigen, vollzylinderförmigen oder ringförmigen Katalysatorformkörpern mit gekrümmter und/oder nicht gekrümmter Stirnfläche der Ringe, deren Aktivmasse ein Mo, V und wenigstens eines der Elemente Te und Sb enthaltendes Multimetalloxid ist, wie es z.B. die Schriften EP-A 962 253, DE-A 101 22 027, EP-A 608 838, DE-A 198 35 247, EP-A 895 809, EP-A 1 254 709, EP-A 1 192 987, EP-A 1 262 235, EP-A 1 193 240, JP-A 11-343261, JP-A 11-343262, EP-A 1 090 684, EP-A 1 301 457, EP-A 1 254 707, EP-A 1 335 793, DE-A 100 46 672, DE-A 100 34 825, EP-A 1 556 337, DE-A 100 33 121, WO 01/98246, EP-A 1 558 569 beschreiben.
Häufig enthalten die vorgenannten Multimetalloxidmassen noch das Element Nb. Die vorgenannten Multimetalloxidkatalysatoren eignen sich bei erfindungsgemäßer Herstellung für alle in den vorgenannten Schriften durchgeführten heterogen katalysierten Gasphasenreaktionen. Dies sind im besonderen die heterogen katalysierte partielle Gasphasenoxidation von Propan zu Acrylsäure sowie von Acrolein zu Acrylsäure, von Methacrolein zu Methacrylsäure und von iso-Butan zu Methacrylsäure.

Abschließend sei an dieser Stelle noch festgehalten, dass erfindungsgemäß hergestellte Katalysatorformkörper nicht in notwendiger Weise als solche als Katalysatoren für heterogen katalysierte Gasphasenreaktionen eingesetzt werden müssen. Vielmehr können sie einer Mahlung unterworfen werden und nach Klassieren des dabei resultierenden feinteiligen Materials mit Hilfe eines geeigneten flüssigen Bindemittels auf die Oberfläche eines geeigneten Trägerkörpers aufgebracht werden. Nach Trocknung oder unmittelbar nach Auftragung der Aktivmassenschale auf den Trägerkörper kann der resultierende Schalenkatalysator als Katalysator für heterogen katalysierte Gasphasenreaktionen eingesetzt werden, wie es z. B. die DE-A 101 22 027 beschreibt.

Zusammenfassend sei nochmals festgehalten, dass die erfindungsgemäß erhältlichen Katalysatorformkörper in hervorragender Weise als Katalysatoren für heterogen katalysierte Reaktionen in der Gasphase mit erhöhter Selektivität der Zielproduktbildung geeignet sind. Diese Gasphasenreaktionen sind die partiellen Oxidationen organischer Verbindungen, die partiellen Ammoxidationen organischer Verbindungen sowie die Oxidehydrierungen organischer Verbindungen. Als partielle heterogen katalysierte Oxidationen organischer Verbindungen kommen vor allem die in der DE-A 10 2004 025 445 genannten in Betracht. Beispielhaft seien nochmals genannt die Umsetzung von Propylen zu Acrolein und/oder Acrylsäure (vgl. z.B. DE-A 23 51 151), die Umsetzung von tert-Butanol, iso-Buten, iso-Butan, iso-Butyraldehyd oder dem Methylether des tert.-Butanols zu Methacrolein und/oder Methacrylsäure (vgl. z.B. DE-A 25 26 238, EP-A 092 097, EP-A 58927, DE-A 41 32 263, DE-A 41 32 684 und DE-A 40 22 212), die Umsetzung von Acrolein zu Acrylsäure, die Umsetzung von Methacrolein zu Methacrylsäure (vgl. z.B. die DE-A 25 26 238), die Umsetzung von o-Xylol, p-Xylol oder Naphtalin zu Phthalsäureanhydrid (vgl. z.B. EP-A 522 871) oder den entsprechenden Säuren sowie die Umsetzung von Butadien zu Maleinsäureanhydrid (vgl. z.B. DE-A 21 06 796 und DE-A 16 24 921), die Umsetzung von n-Butan zu Maleinsäureanhydrid (vgl. z.B. GB-A 1 464 198 und GB-A 1 291 354), die Umsetzung von Indenen zu z.B. Anthrachinon (vgl. z.B. DE-A 20 25 430), die Umsetzung von Ethylen zu Ethylenoxid oder von Propylen zu Propylenoxid (vgl. z.B. DE-AS 12 54 137, DE-A 21 59 346, EP-A 372 972, WO 89/07101, DE-A 43 11 608 und Beyer, Lehrbuch der organischen Chemie, 17. Auflage (1973), Hirzel Verlag, Stuttgart, S. 261), die Umsetzung von Propylen und/oder Acrolein zu Acrylnitril (vgl. z.B. DE-A 23 51 151), die Umsetzung von iso-Buten und/oder Methacrolein zu Methacrylnitril (d. h., der Begriff der partiellen Oxidation soll, wie bereits gesagt, in dieser Schrift auch die partielle Ammoxidation, d.h., eine partielle Oxidation im Beisein von Ammoniak umfassen), die oxidative Dehydrierung von Kohlenwasserstoffen (vgl. z. B. DE-A 23 51 151), die Umsetzung von Propan zu Acrylnitril oder zu Acrolein und/oder Acrylsäure (vgl. z. B. DE-A 101 31 297, EP-A 1 090 684, EP-A 608 838, DE-A 100 46 672, EP-A 529 853, WO 01/96270 und DE-A 100 28 582), so wie die Reaktionen von Ethan zu Essigsäure, von Benzol zu Phenol sowie von Kohlenwasserstoffen mit 4 Kohlenstoffatomen zu den entsprechenden Butandiolen oder zu Butadien.

Beispiele B und Vergleichsbeispiele V

A) Herstellung von ringförmigen Vollkatalysatorformkörpern mit der nachfolgenden Stöchiometrie der Aktivmasse und unter Verwendung verschiedener Graphite als Fomungshilfsm ittel:

$$[Bi_2W_2O_9 \cdot 2WO_3]_{0,48}[Mo_{12}Co_{5,4}Fe_{3,1}Si_{1,5}K_{0,08}O_x]_1.$$

1. Herstellung einer Ausgangsmasse 1

In 780 kg einer 25 °C aufweisenden wässrigen salpetersauren Wismutnitratlösung (11,2 Gew.-% Bi; freie Salpetersäure 3 bis 5 Gew.-%; Massendichte: 1,22 bis 1,27 g/ml, hergestellt mit Salpetersäure aus Wismutmetall der Firma Sidech S.A., 1495 Tilly, Belgien, Reinheit: > 99,997 Gew.-% Bi, < 7 mg/kg Pb, je < 5 mg/kg Ni, Ag, Fe, je < 3 mg/kg Cu, Sb und < 1 mg/kg Cd, Zn) wurden bei 25°C innerhalb von 20 min portionsweise 214,7 kg einer 25 °C aufweisenden Wolframsäure (74,1 Gew.-% W, H.C. Starck, D-38615 Goslar, Reinheit > 99,9 Gew.-% $WO_3$ nach Glühen bei 750 °C, 0,4 $\mu$m < $d_{50}$ < 0,8 $\mu$m) eingerührt (70 U/min). Das resultierende wässrige Gemisch wurde anschließend noch 3 h bei 25°C gerührt und dann sprühgetrocknet.

Die Sprühtrocknung erfolgte in einem Drehscheibensprühturm im Heißluftgegenstrom bei einer Gaseintritts-temperatur von 300 $\pm$ 10 °C, einer Gasaustrittstemperatur von 100 $\pm$ 10°C, einer Scheibendrehzahl von 18000 U/min und einem Durchsatz von 200 l/h. Das erhaltene Sprühpulver, dessen Körnung im wesentlichen zwischen 5 und 30 $\mu$m betrug und das einen Glühverlust von 12,8 Gew.-% aufwies (3 h bei 600°C unter Luft glühen), wurde anschließend mit 16,7 Gew.-% (bezogen auf das Pulver) an 25 °C aufweisendem Wasser in einem Kneter (20 U/min) für 30 min angeteigt und mittels eines Extruders (Drehmoment: $\leq$ 50 Nm) zu Strängen des Durchmessers 6 mm extrudiert. Diese wurden in Abschnitte von 6 cm geschnitten, auf einem 3-zonigen Bandtrockner bei einer Verweilzeit von 120 min je Zone bei Temperaturen von 90-95°C (Zone 1), 115 °C (Zone 2) und 125 °C (Zone 3) an Luft getrocknet und dann bei einer Temperatur im Bereich um 830°C thermisch behandelt (kalziniert; im luftdurchströmten Drehrohrofen (0,3 mbar Unter-druck, 1,54 $m^3$ Innenvolumen, 200 $Nm^3$/h Luft, 50 kg/h Extrudat, Drehzahl: 1 U/min)). Wesentlich bei der genauen Einstellung der Kalzinationstemperatur ist, dass sie an der angestrebten Phasenzusammensetzung des Kalzinationsprodukts orientiert zu erfolgen hat. Gewünscht sind die Phasen $WO_3$ (monoklin) und $Bi_2W_2O_9$ (orthorhombisch), uner-wünscht ist das Vorhandensein von $\gamma$-$Bi_2WO_6$ (Russellit). Sollte daher nach der Kalzination die Verbindung $\gamma$-$Bi_2WO_6$ anhand eines Reflexes im Röntgenpulverdiffraktogramm bei einem Reflexwinkel von 2$\Theta$ = 28,4° (CuK$\alpha$-Strahlung) noch nachweisbar sein, so ist die Präparation zu wiederholen und die Kalzinationstemperatur innerhalb des angegebenen Temperaturbereichs oder die Verweilzeit bei gleichbleibender Kalzinationstemperatur zu erhöhen, bis das Verschwinden des Reflexes erreicht wird. Das so erhaltene vorgebildete kalzinierte Mischoxid wurde mit einer Biplexmühle BQ500 mit 2500 U/min gemahlen, so dass der $d_{50}$-Wert 2,45 $\mu$m ($d_{10}$ = 1,05 $\mu$m, $d_{90}$ = 5,92 $\mu$m) und die BET-Oberfläche 0,8 $m^2$/g betrug.

Das Mahlgut wurde dann in Portionen von 5 kg in einem Eirich-Intensivmischer (Typ R02, Füllvolumen: 3-5 l, 1,9 kW, Maschinenfabrik Gustav Eirich GmbH & Co KG, D-74736 Hardheim) mit gegenläufig zum Teller rotierenden Messerköpfen (Drehzahl Teller: 50 U/min, Drehzahl Messerköpfe: 2500 U/min) innerhalb von 5 min homogen mit 0,5 Gew.-% (bezogen auf das Mahlgut) feinteiligem $SiO_2$ der Fa. Degussa vom Typ Sipernat® D17 (Rüttelgewicht 150 g/l; $d_{50}$-Wert der $SiO_2$-Partikel (Laserbeugung nach ISO 13320-1) betrug 10 $\mu$m, die spezifische Oberfläche (Stickstoffad-sorption nach ISO 5794-1, Annex D) betrug 100 $m^2$/g) vermischt.

2. Herstellung einer Ausgangsmasse 2

Eine Lösung A wurde hergestellt, indem man bei 60°C unter Rühren (70 U/min) zu 660 l eine Temperatur von 60°C aufweisendem Wasser innerhalb einer Minute 1,075 kg einer eine Temperatur von 60°C aufweisenden wässrigen Kaliumhydroxidlösung (47,5 Gew.-% KOH) und anschließend mit einer Dosiergeschwindigkeit von 600 kg/h 237,1 kg Ammoniumheptamolybdat-tetrahydrat (weiße Kristalle mit einer Körnung d < 1 mm, 81,5 Gew.-% $MoO_3$, 7,0-8,5 Gew.-% $NH_3$, max. 150 mg/kg Alkalimetalle, H.C. Starck, D-38642 Goslar) dosierte und die resultierende leicht trübe Lösung bei 60°C 60 min rührte.

Eine Lösung B wurde hergestellt, indem man bei 60°C in 282,0 kg einer eine Temperatur von 60 °C aufweisenden wässrigen Kobalt(-II)-nitratlösung (12,5 Gew.-% Co, hergestellt mit Salpetersäure aus Kobaltmetall der Firma MFT Metals & Ferro-Alloys Trading GmbH, D-41747 Viersen, Reinheit, >99,6 Gew.-%, < 0,3 Gew.-% Ni, < 100 mg/kg Fe, < 50 mg/kg Cu) vorlegte und zu dieser unter Rühren (70 U/min) 142,0 kg einer 60 °C warmen Eisen-(III)-nitrat-nonahydrat-Schmelze (13,8 Gew.-% Fe, < 0,4 Gew.-% Alkalimetalle, < 0,01 Gew.-% Chlorid, < 0,02 Gew.-% Sulfat, Dr. Paul Lohmann GmbH, D-81857 Emmerthal) dosierte. Anschließend wurde unter Aufrechterhaltung der 60°C 30 Minuten nachgerührt. Dann wurde unter Beibehalt der 60 °C die Lösung B in die vorgelegte Lösung A abgelassen und weitere 15 Minuten bei 60°C gerührt. Anschließend wurden dem resultierenden wässrigen Gemisch 19,9 kg eines Kieselgels der Fa. Du Pont vom Typ Ludox (49,1 Gew.-% $SiO_2$, Dichte: 1,29 g/ml, pH 8,5 bis 9,5, Alkaligehalt max. 0,5 Gew.-%) zugegeben und danach noch weitere 15 Minuten bei 60°C gerührt.

Anschließend wurde in einem Drehscheibensprühturm im Heißluftgegenstrom sprühgetrocknet (Gaseintrittstemperatur: 350 ± 10°C, Gasaustrittstemperatur: 140 ± 5°C, Scheibendrehzahl: 18000 U/min). Das resultierende Sprühpulver wies einen Glühverlust von 31,5 Gew.-% (3 h bei 600°C unter Luft glühen) und einen $d_{50}$ von 19,7 $\mu$m ($d_{10}$ = 3,19 $\mu$m, $d_{90}$ = 51,49 $\mu$m) auf.

3. Herstellung der Multimetalloxidkatalysatorformkörper

Die Ausgangsmasse 1 wurde mit der Ausgangsmasse 2 in den für eine Multimetalloxidaktivmasse der Stöchiometrie

$$[Bi_2W_2O_9 \cdot 2WO_3]_{0,48}[Mo_{12}Co_{5,4}Fe_{3,1}Si_{1,5}K_{0,08}O_x]_1$$

erforderlichen Mengen (Gesamtmenge: 3 kg) in einem Eirich-Intensivmischer (Typ R02, Füllvolumen: 3-5 l, Leistung: 1,9 kW, Maschinenfabrik Gustav Eirich GmbH & Co KG, D-74736 Hardheim) mit gegenläufig zum Teller rotierenden Messerköpfen (Drehzahl Teller: 50 U/min, Drehzahl Messerköpfe: 2500 U/min) innerhalb von 5 min homogen vermischt.

Bezogen auf die vorgenannte Gesamtmasse wurden hierzu in einem Rhönradmischer (Raddurchmesser: 650 mm, Trommelvolumen: 5 l) zusätzlich 1 Gew.-% des jeweiligen Graphits innerhalb von 30 min bei einer Drehzahl von ca. 30 U/min homogen untergemischt. Das resultierende Gemisch wurde dann in einem Laborkalander mit 2 gegenläufigen Stahlwalzen bei einem Pressdruck von 9 bar kompaktiert und durch ein Sieb mit einer Maschenweite von 0,8 mm gedrückt. Das resultierende Kompaktat wies eine Härte von 10 N und eine im wesentlichen einheitliche Körnung von ≥ 0,4 mm und ≤ 0,8 mm auf.

Das Kompaktat wurde anschließend in einem Rhönradmischer (Raddurchmesser: 650 mm, Trommelvolumen: 5 l) bei einer Drehzahl von ca. 30 U/min innerhalb von 30 Minuten mit, bezogen auf sein Gewicht, weiteren 2,5 Gew.-% des jeweils selben Graphits vermischt und anschließend in einem Kilian Rundläufer (9-fach-Tablettiermaschine) vom Typ S100 (Fa. Kilian, D-50735 Köln) unter Stickstoffatmosphäre zu ringförmigen Vollkatalysatorvorläuferformkörpern der Geometrie (5 x 3 x 2 mm, A (Außendurchmesser) x H (Höhe) x I (Innendurchmesser)) mit einer Seitendruckfestigkeit von 20 ± 1 N verdichtet (Füllhöhe: 7,5-9 mm, Presskraft: 3,0-3,5 kN).

Zur abschließenden thermischen Behandlung wurden jeweils 1000 g der jeweils hergestellten Vollkatalysatorvorläuferformkörper gleichmäßig aufgeteilt auf nebeneinander angeordnete 4 Gitternetze mit einer quadratischen Grundfläche von jeweils 150 mm x 150 mm (Schütthöhe: ca. 15 mm) in einem mit 1000 Nl/h Luft durchströmten Umluftofen (Firma Heraeus Instruments GmbH, D-63450 Hanau, Typ: K 750/2) zunächst mit einer Aufheizrate von 80°C/h von Raumtemperatur (25°C) auf 185°C aufgeheizt. Diese Temperatur wurde für 1 h aufrechterhalten und dann mit einer Aufheizrate von 48°C/h auf 225°C erhöht. Die 225°C wurden für 2 h gehalten, bevor sie mit einer Aufheizrate von 120°C/h, auf 270°C erhöht wurde. Diese Temperatur wurde ebenfalls 1 h aufrechterhalten, bevor sie, mit einer Aufheizrate von 60°C/h, auf 464°C erhöht wurde. Diese Endtemperatur wurde für 10 Stunden gehalten. Danach wurde auf Raumtemperatur abgekühlt.

4. Testung der mit den verschiedenen Graphiten jeweils erhaltenen Vollkatalysatorformkörper für die heterogen katalysierte Partialoxidation von Propen zu Acrolein

Ein Reaktionsrohr (V2A Stahl; 21 mm Außendurchmesser, 3 mm Wandstärke, 15 mm Innendurchmesser, Länge 120 cm) wurde von oben nach unten in Strömungsrichtung wie folgt beschickt:

Abschnitt 1:     ca. 30 cm Länge

40 g Steatitkugeln mit einem Durchmesser von 1,5 bis 2,0 mm als Vorschüttung.

Abschnitt 2: ca. 70 cm Länge
Katalysatorbeschickung mit 100 g des unter 3 hergestellten ringförmigen Vollkatalysator.

Die Temperierung des Reaktionsrohres erfolgte mittels eines mit Stickstoff geperlten Salzbades.

Der Reaktor wurde kontinuierlich mit einem Beschickungsgasgemisch (Gemisch aus Luft, polymer grade Propylen und Stickstoff) der Zusammensetzung:

5 Vol.-% Propen,
9,5 Vol.-% Sauerstoff und

als Restmenge bis 100 Vol.-% $N_2$

beschickt, wobei die Belastung des Reaktionsrohres mit Beschickungsgasgemisch 100 Nl/h (5 Nl/h Propen) betrug und die Thermostatisierung des Reaktionsrohres durch Variation der Salzbadtemperatur $T_s$ (°C) so erfolgte, dass der Propenumsatz U (mol-%) bei einmaligem Durchgang des Beschickungsgasgemisches durch das Reaktionsrohr kontinuierlich etwa 95 mol-% betrug.

Die verwendeten Graphite und ihre Partikeleigenschaften waren:

| | Graphit | $d_{10}$ ($\mu$m) | $d_{50}$ ($\mu$m) | $d_{90}$ ($\mu$m) | $O_G$ (m$^2$/g) |
|---|---|---|---|---|---|
| V1 | Timrex HSAG 100[a] | 3,3 | 13,1 | 78,4 | 121 |
| V2 | Asbury 230U[b] | 4,8 | 14,5 | 40,6 | 6,2 |
| V3 | Timrex T44[a] | 6,4 | 20,8 | 56,8 | 6,5 |
| B1 | Asbury 3160[b] | 37,0 | 123 | 259 | 1,6 |
| B2 | Asbury 4012[b] | 87,6 | 166 | 270 | 1,7 |
| a: Graphite der Firma Timcal Ltd., 6743 Bodio, Schweiz | | | | | |
| b: Graphite der Firma Asbury Graphite Mills, Inc. New Jersey 08802, USA | | | | | |

Die verschiedenen verwendeten Graphite wiesen weiterhin folgende Eigenschaften auf (ein Spiegelstrich bedeutet, dass keine Eigenschaftsbestimmung durchgeführt wurde):

| | Graphit | Aschegehalt (Gew.-%) | $T_i$(°C) | $T_m$°(C) |
|---|---|---|---|---|
| V1 | V1 | - | - | - |
| V2 | V2 | 0,3 | - | - |
| V3 | V3 | 0,1 | 505 | 670 |
| B1 | B1 | 0,7 | 505 | 700 |
| B2 | B2 | 0,2 | 550 | 745 |

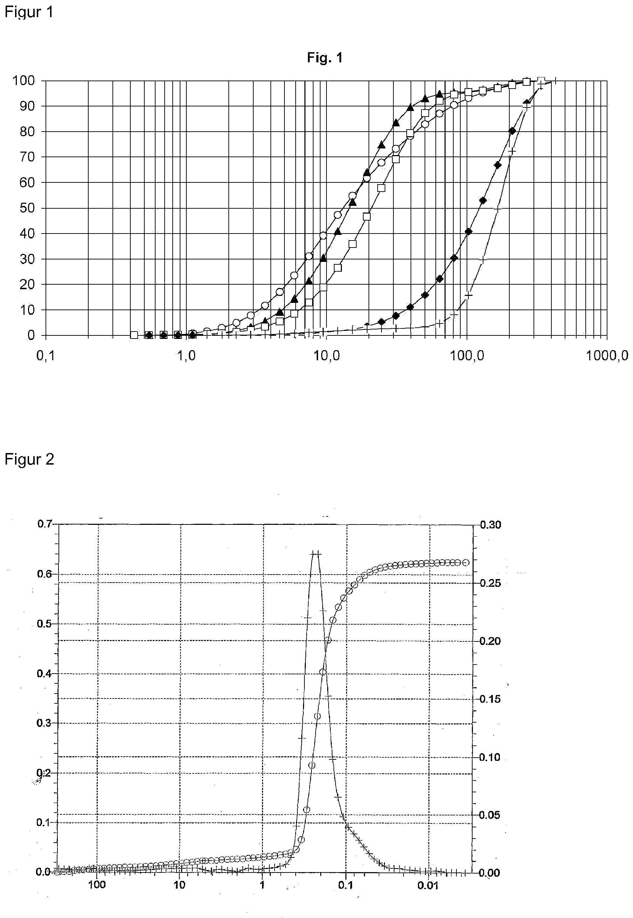

Figur 1 zeigt für die verschiedenen verwendeten Graphite die jeweilige Partikeldurchmesserverteilung (Laser, Malvern).

Dabei zeigt die Abszisse die Durchmesser [$\mu$m] im logarithmischen Maßstab. Die Ordniate zeigt den Volumenanteil das jeweilgen Graphits, der den jeweiligen Durchmesser oder einen kleineren Durchmesser aufweist [Vol.-%]. Dabei ist:

-□- V3
-▲- V2
-○- V1
-●- B1, und
-+- B2

Der Aschegehalt gibt den bei einer vollständigen Verbrennung des Graphits verbleibenden oxidischen Rückstand an (in Gew.-%, bezogen auf das Gewicht der verbrannten Graphitmenge).

Die nachfolgende Tabelle zeigt für die bei Verwendung der verschiedenen Graphite erhaltenen Katalysatorformkörper deren Porengesamtvolumen V ($cm^3/g$), deren spezifische Oberfläche O ($m^2/g$), den Porendurchmesser $d^{max}$ ($\mu m$) derjenigen Poren, die in ihrer Gesamtzahl den größten prozentualen Beitrag zum Porengesamtvolumen V leisten, die Graphitmenge (gerechnet als reiner Kohlenstoff) $\Delta m$ (Gew.-%, bezogen auf die in 100 Katalysatorvorläuferformkörpern enthaltene Graphitmenge), die während der thermischen Behandlung dieser Katalysatorvorläuferformkörper aus selbigen entschwindet, die (jeweils nach einer Betriebszeit von 60 h bestimmte) für den Propylenumsatz von 95 mol-% bei ihrer Verwendung als Katalysator für die beschriebene Partialoxidation von Propen zu Acrolein jeweils erforderliche Salzbadtemperatur $T_s$ (°C), die Selektivität $S^A$ der Acroleinbildung (mol-%) sowie die für den Fall einer nachfolgenden partiellen Oxidation des gebildeten Acroleins zu Acrylsäure wichtige Summe der Selektivität $S^A$ der Acroleinbildung und der Selektivität $S^{AS}$ der Acrylsäurenebenproduktbildung ($S^A+S^{AS}$, mol-%):

| verwendeter Graphit | $\Delta m$ | V | O | $d^{max}$ | $T_S$ | $S^A$ | $S^A+S^{AS}$ |
|---|---|---|---|---|---|---|---|
| V1 | 57 | 0,268 | 6,67 | 0,224 | 332 | 87,3 | 94,5 |
| V2 | 20 | 0,267 | 6,11 | 0,250 | 327 | 87,8 | 94,5 |
| V3 | 23 | 0,275 | 6,64 | 0,259 | 332 | 88,6 | 95,0 |
| B1 | 11 | 0,261 | 6,28 | 0,264 | 336 | 88,9 | 95,4 |
| B2 | 11 | 0,249 | 5,86 | 0,273 | 331 | 89,2 | 95,4 |

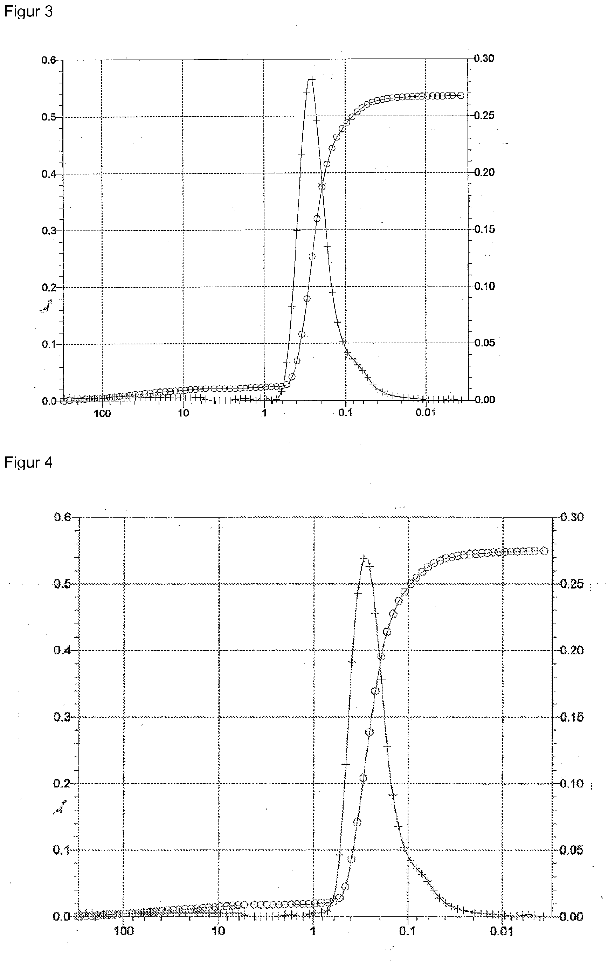

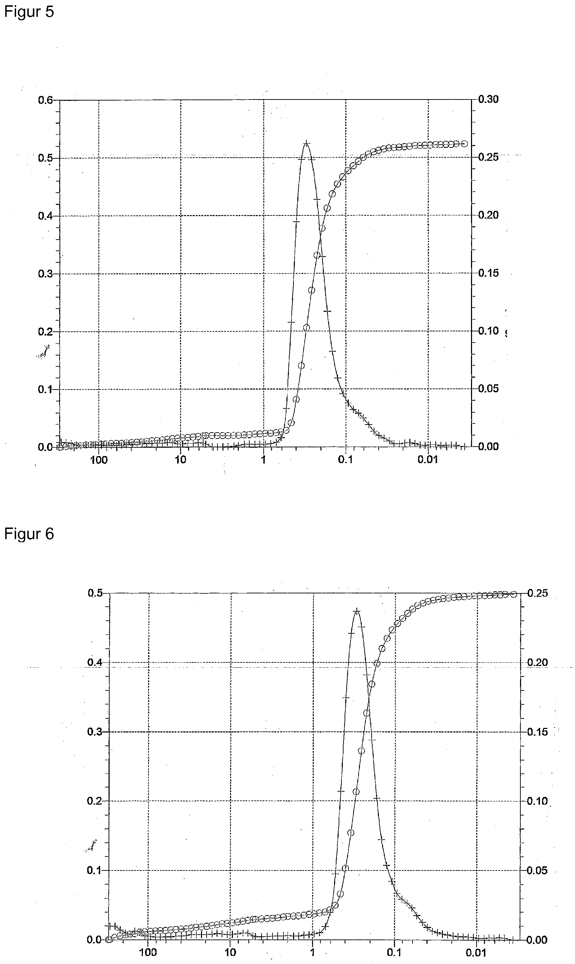

Die Figuren 2 bis 6 zeigen zusätzlich die Porenverteilung der mit den verschiedenen Graphiten erhaltenen Vollkatalysatorformkörper (V1 → Figur 2; V2 → Figur 3; V3 → Figur 4; B1 → Figur 5; B2→ Figur 6). Auf der Abszisse ist dabei jeweils der Porendurchmesser im logarithmischen Maßstab in $\mu m$ aufgetragen. Auf der linken Ordinate ist der Logarithmus des differentiellen Beitrags in ml/g des jeweiligen Porendurchmessers zum Porengesamtvolumen aufgetragen (Kurve +). Das Maximum weist den Porendurchmesser $d^{max}$ derjenigen Poren aus, die prozentual den größten Beitrag zum Porengesamtvolumen leisten. Auf der rechten Ordinate ist in ml/g das Integral über die individuellen Beiträge der den einzelnen Porendurchmessern zuzuordnenden Poren zum Porengesamtvolumen aufgetragen (Kurve O). Der Endpunkt ist jeweils das Porengesamtvolumen.

Bei einer Herstellung der wie vorstehend beschrieben herzustellenden Katalysatorformkörper im großtechnischen Maßstab wird man im Herstellungsabschnitt "3." im wesentlichen folgende Abänderungen vornehmen:

Mischen:

Eirich-Intensivmischer (Maschinenfabrik Gustav Eirich GmbH Co KG, D-74736 Hardheim) mit rotierenden Messerköpfen (Drehzahl: 3000 U/min, Mischdauer: 25 min).

Kompaktieren:

Kompaktor K200/100 mit konkaven, geriffelten Glattwalzen (Spaltweite: 2,8 mm, Siebweite: 1,0 mm, Siebweite Unterkorn: 400 $\mu m$, Presssollkraft: 75 kN, Schneckendrehzahl: < 70 U/min, Fa. Hosokawa Bepex GmbH, D-74211 Leingarten).

Tablettieren:

Kilian Rundläufer (Tablettiermaschine) vom Typ R x 73 (Presskraft: 3-15 kN, Fa. Kilian, D-50735 Köln).

Kalzinierung:

Anstatt die thermische Behandlung wie beschrieben durchzuführen, kann man sie auch wie in Beispiel 1 der DE-A 100 46 957 (die Schütthöhe in der Zersetzung (Kammern 1 bis 4) beläuft sich dabei jedoch vorteilhaft auf 44 mm bei einer Verweilzeit pro Kammer von 1,46 h und in der Kalzination (Kammern 5 bis 8) beläuft sie sich vorteilhaft auf 130 mm bei einer Verweilzeit von 4,67 h) beschrieben mittels einer Bandkalziniervorrichtung durchführen; die Kammern besitzen eine Grundfläche (bei einer einheitlichen Kammerlänge von 1,40 m) von 1,29 $m^2$ (Zersetzung) und 1,40 $m^2$ (Kalzination) und werden von unten durch das grobmaschige Band von 50-150 $Nm^3/h$ Zuluft durchströmt; zusätzlich wird die Luft durch rotierende Ventilatoren (900 bis 1500 U/min) umgewälzt. Innerhalb der Kammern ist die zeitliche und örtliche Abweichung der Temperatur vom Sollwert stets $\leq$ 2°C. Im übrigen wird wie in Beispiel 1 der DE-A 100 46 957 beschrieben verfahren.

Die resultierenden Katalysatorformkörper eignen sich in gleicher Weise als Katalysatoren für die beschriebene Partialoxidation von Propylen zu Acrolein.

B) Herstellung und Testung von ringförmigen Vollkatalysatorformkörpern mit der nachfolgenden Stöchiometrie der Aktivmasse und unter Verwendung verschiedener Graphite als Formungshilfsmittel (als Edukte dienten die bereits unter A) genannten Chemikalien):

$$Mo_{12}Co_7Fe_{2,94}Bi_{0,6}Si_{1,59}K_{0,08}O_x$$

Bei 60 °C wurden 213 kg Ammoniumheptamolybdattetrahydrat (81,5 Gew.-% $MoO_3$) in 600 l Wasser gelöst. In diese Lösung wurden unter Aufrechterhaltung der 60 °C 0,97 kg einer 46,8 gew.-%igen wässrigen Kaliumhydroxidlösung von 20 °C eingerührt (dabei wurde eine Lösung A erhalten).

Eine zweite Lösung B wurde hergestellt, indem man unter Rühren zu 331,0 kg einer wässrigen Kobalt-(II)-nitratlösung (12,5 Gew.-% Co) bei 60°C 119,6 kg einer 60°C aufweisenden wässrigen Eisen-(III)-nitrat-nonahydrat-schmelze (13,8 Gew.-% Fe) gab. Nach beendeter Zugabe wurde noch 30 min. bei 60°C gerührt. Danach wurden bei 60°C 112,3 kg einer 20°C aufweisenden wässrigen Wismutnitratlösung (11,2 Gew.-% Bi) unter Erhalt der Lösung B eingerührt. Innerhalb von 30 min. wurde bei 60°C die Lösung B in die Lösung A eingerührt. 15 min. nach beendetem Einrühren wurden bei 60°C 18,26 kg Kieselsol (49,1 Gew.-% $SiO_2$) in die erhaltene Maische gegeben. Unter Aufrechterhaltung der 60°C wurde noch 15 min. nachgerührt. Dann wurde die erhaltene Maische im Heißluftgegenstromverfahren sprühgetrocknet (Gaseingangstemperatur: 400 ± 10°C, Gasausgangstemperatur: 140 ± 5°C) wobei ein Sprühpulver erhalten wurde, dessen Glühverlust (3 h bei 600°C unter Luft) 30 % seines Gewichtes betrug. Das resultierende Sprühpulver wies einen Partikeldurchmesser $d_{50}$ von 20,3 μm sowie einen $d_{10}$ von 3,24 μm und einen $d_{90}$ von 53,6 μm auf.

In Teilmengen des erhaltenen Sprühpulvers wurden jeweils zusätzlich 1,5 Gew.-% (bezogen auf die Sprühpulvermenge) des jeweiligen feinteiligen Graphit eingemischt.

Das dabei jeweils resultierende Trockengemisch wurde mittels eines Kompaktors der Fa. Hosokawa Bepex GmbH (D-74211 Leingarten) vom Typ Kompaktor K 200/100 unter den Bedingungen von 2,8 mm Spaltbreite, 1,0 mm Siebweite, 400 μm Siebweite Unterkorn, 60 kN Presssollkraft und 65 bis 70 Upm Schneckendrehzahl durch Vorkompaktieren auf eine im wesentlichen einheitliche Korngröße von 400 μm bis 1 mm vergröbert. Das Kompaktat hatte eine Härte von 10 N.

Das Kompaktat wurde anschließend mit, bezogen auf sein Gewicht, weiteren 2 Gew.-% des jeweils selben Graphit vermischt und anschließend in einem Kilian Rundläufer (Tablettiermaschine) vom Typ Rx73, der Fa. Kilian, D-50735 Köln, unter Stickstoffatmosphäre zu ringförmigen Vollkatalysatorvorläuferformkörpern mit nicht gekrümmter Stirnfläche der Geometrie 5 mm x 3 mm x 2 mm (A x H x I) mit einer Seitendruckfestigkeit von 20 ± 1 N verdichtet.

Zur abschließenden thermischen Behandlung wurden jeweils 1900 g der Vollkatalysatorvorläuferformkörper in einer beheizbaren Umluftkammer (0,12 m³ Innenvolumen) aufgeschüttet (2 Nm³ Luft/min.). Anschließend wurde die Temperatur in der Schüttung wie folgt verändert:

- mit 1°C/min. von 25°C auf 160°C erhöht;
- dann 100 min. bei 160°C gehalten;
- danach mit 3°C/min. von 160°C auf 200°C erhöht;
- dann 100 min. bei 200°C gehalten;
- danach mit 2°C/min. von 200°C auf 230°C erhöht;
- dann 100 min. bei 230°C gehalten;
- danach mit 3°C/min. von 230°C auf 270°C erhöht;
- dann 100 min. bei 270°C gehalten;
- danach mit 1°C/min. auf 380°C erhöht;
- dann 4,5 h bei 380°C gehalten;
- danach mit 1°C/min. auf 430°C erhöht;
- dann 4,5 h bei 430°C gehalten;
- danach mit 1°C/min. auf 500°C erhöht;
- dann 9 h bei 500°C gehalten;
- danach innerhalb von 4 h auf 25°C abgekühlt.

Dabei wurden aus den ringförmigen Vollkatalysatorvorläuferformkörpern ringförmige Vollkatalysatorformkörper erhalten.

(Anstatt die thermische Behandlung wie beschrieben durchzuführen, kann man sie auch wie in Beispiel 3 der DE-A 100 46 957 beschrieben mittels einer Bandkalziniervorrichtung durchführen; die Kammern besitzen eine Grundfläche (bei einer einheitlichen Kammerlänge von 1,40 m) von 1,29 m$^2$ (Zersetzung, Kammern 1-4) und 1,40 m$^2$ (Kalzination, Kammern 5-8) und werden von unten durch das grobmaschige Band von 70-120 Nm$^3$/h Zuluft, bevorzugt von 75 Nm$^3$/h Zuluft, durchströmt; zusätzlich wird die Luft durch rotierende Ventilatoren (900 bis 1500 U/min) umgewälzt; innerhalb der Kammern war die zeitliche und örtliche Abweichung der Temperatur vom Sollwert stets $\leq$ 2°C; durch die Kammern werden die ringförmigen Vollkatalysatorvorläuferformkörper in einer Schichthöhe von 50 mm bis 110 mm, bevorzugt von 50 mm bis 70 mm, geführt; im übrigen wird wie in Beispiel 3 der DE-A 100 46 957 beschrieben verfahren; die resultierenden ringförmigen Vollkatalysatoren können wie die in A) erhaltenen Vollkatalysatorformkörper für die in A) 4. beschriebene gasphasenkatalytische Partialoxidation von Propen zu Acrolein eingesetzt werden).

Die erhaltenen ringförmigen Vollkatalysatoren wurden wie in A) 4. beschrieben als Katalysatoren für eine heterogen katalysierte partielle Gasphasenoxidation von Propen zu Acrolein eingesetzt (jedoch mit dem Unterschied, dass die Vorschüttung im Reaktionsrohr auf einer Länge von 30 cm aus Steatitringen der Geometrie 5 mm x 3 mm x 2 mm (A x H x I) und auf einer Schüttlänge von 70 cm aus den Vollkatalysatorformkörpern bestand).

Die dabei erhaltenen Selektivitäten S$^A$ der Acroleinbildung (ebenfalls nach 120 h Betriebszeit) waren:

| Verwendeter Graphit | S$^A$ (mol-%) |
|---|---|
| V1 | 89,8 |
| V2 | 89,9 |
| V3 | 90,5 |
| B1 | 90,8 |
| B2 | 91,0 |

C) Zur Herstellung und Testung von ringförmigen Vollkatalysatorformkörpern mit der nachfolgenden Stöchiometrie der Aktivmasse und unter Verwendung verschiedener Graphite als Formungshilfsmittel:

$$Mo_{12}P_{1,5}V_{0,6}Cs_{1,0}Cu_{0,5}Sb_1S_{0,04}O_x$$

In 619 l auf 45°C temperiertes Wasser in einem wassertemperierten Doppelmantelbehälter wurden unter Rühren (70 Umdrehungen pro Minute (UPM)) 537,5 kg Ammoniumheptamolybdattetrahydrat ((NH$_4$)$_6$Mo$_7$O$_{24}$·4 H$_2$O (81 Gew.-% MoO$_3$, 8 Gew.-% NH$_3$, $\leq$ 50 Gew.-ppm Na und $\leq$ 100 Gew.-ppm K) eindosiert. Die Temperatur der Lösung sank hierbei auf 37°C ab. Um ein sicheres Auflösen des Ammoniumheptamolybdats zu gewährleisten, wurde nach Ende der Zudosierung noch 15 Minuten nachgerührt, wobei die Temperatur von 37°C beibehalten wurde. Unter weiterem Rühren wurden bei derselben Temperatur innerhalb von 3 Minuten 17,82 kg Ammoniummetavanadat (NH$_4$VO$_3$, 77 Gew.-% V$_2$O$_5$, 14,5 Gew.-% NH$_3$, $\leq$ 150 Gew.-ppm Na und $\leq$ 500 Gew.-ppm K) zudosiert. Es wurde 2 Minuten nachgerührt. Dann wurde innerhalb einer Minute eine in einem separaten Lösebehälter hergestellte, farblose, klare, 60°C warme Lösung von 49,6 kg Cäsiumnitrat (CsNO$_3$ mit 72 Gew.-% Cs$_2$O und $\leq$ 50 Gew.-ppm Na, $\leq$ 100 Gew.-ppm K, $\leq$ 10 Gew.-ppm Al sowie $\leq$ 20 Gew.-ppm Fe) in 106 l Wasser eingerührt. Hierbei stieg die Temperatur der resultierenden Suspension auf 39°C. Nach einminütigem Nachrühren wurden innerhalb einer weiteren Minute unter fortgesetztem Rühren 31,66 l 75 gew.-%ige Phosphorsäure (Dichte bei 25°C und 1 atm: 1,57 g/ml, Viskosität bei 25°C und 1 atm: 0,147 cm$^2$/S) zudosiert. Aufgrund der exothermen Reaktion stieg die Temperatur hierbei auf 42°C. Erneut wurde 1 Minute nachgerührt. Dann wurden innerhalb einer Minute 1,34 kg Ammoniumsulfat ((NH$_4$)$_2$SO$_4$ (> 99 Gew.-%)) eingerührt und 1 weitere Minute nachgerührt. Unter fortgesetztem Rühren bei identischer Temperatur wurden innerhalb von 3 Minuten 37,04 kg Antimontrioxid (Sb$_2$O$_3$, Partikeldurchmesser d$_{50}$= ca. 2 $\mu$m, Kristallstruktur laut XRD: > 75 % Senarmontit, < 25 % Valentinit, Reinheit: > 99,3 Gew.-%, $\leq$ 0,3 Gew.-% As$_2$O$_3$, $\leq$ 0,3 Gew.-% PbO und $\leq$ 300 Gew.-ppm FeO) zugegeben (käuflich erhältlich als Triox White, Code No. 639000 der Fa. Antraco, D-10407 Berlin). Nun wurde die Rührerdrehzahl von 70 auf 50 UPM zurückgenommen. Anschließend wurde die gerührte Suspension mittels Dampf im Doppelmantel innerhalb von 30 Minuten linear auf 95°C aufgeheizt. Bei dieser Temperatur und 50 UPM wurden innerhalb von 4 Minuten 51,64 kg Kupfernitratlösung (wässrige Cu(NO$_3$)$_2$-Lösung mit 15,6 Gew.-% Cu) zugegeben. Nach vierminütigem Nachrühren bei 95°C wurde die Rührgeschwindigkeit weiter von 50 auf 35 UPM zurückgenommen. Anschließend wurde die gesamte Suspension innerhalb von 4 Minuten in einen mit Stickstoff überlagerten, auf 85°C temperierten und mit 35 UPM gerührten Sprühturmvorlagenbehälter abgelassen und es wurde mit 20 l Wasser (25°C) nachgespült. Aus diesem heraus wurde die Suspension in einem Drehscheibensprühturm im Gegenstrom mit einer Eintrittstemperatur von 285°C und einer Austrittstemperatur von 110°C innerhalb von 3,5 h sprühgetrocknet, wobei das resultierende Sprühpulver einen Glühverlust (1 h bei 500°C in Luft) von ca. 16 Gew.-% aufwies.

Das Sprühpulver wurde mit 1,5 Gew.-% des jeweiligen Graphit homogen vermischt und kompaktiert (Kompaktor der Fa. Hosokawa Bepex GmbH, D-74211 Leingarten, Typ K200/100 mit konkaven, geriffelten Glattwalzen, Spaltweite:

2,8 mm, Siebweite: 1,25 mm, Siebweite Unterkorn: 400 μm, Schneckendrehzahl: 65 bis 70 UPM). Für die Tablettierung wurden dem Kompaktat weitere 1 Gew.-% des jeweils selben Graphits zugemischt. Anschließend wurde das Kompaktat in einem Kilian Rundläufer (Tablettiermaschine vom Typ R x 73 der Fa. Kilian, D-50735 Köln) unter Stickstoffatmosphäre zu ringförmigen Vollringtabletten der Geometrie 7 mm x 7 mm x 3 mm (Außendurchmesser x Höhe x Innendurchmesser) mit einer Seitendruckfestigkeit von 35 ± 2 N tablettiert.

8 kg der Rohtabletten wurden in einem Drahtbehälter der Grundfläche 33,0 cm x 49,5 cm gleichmäßig verteilt, wobei sich eine Schütthöhe von 4 cm ergab. Der Drahtbehälter wurde in einem Kammerofen (Fa. Elino Industrie-Ofenbau, Carl Hanf GmbH & Co, D-52355 Düren, Typ KA-040/006-08 EW.OH, Abmessungen: Länge = 57 cm, Breite = 57 cm, Höhe = 80 cm) so angeordnet, dass die Schüttung der Tabletten gleichmäßig durchströmbar war. Es wurden 2 Nm$^3$/h Frischluft zugeführt und die Luftumwälzung im Ofen so eingestellt, dass die Schüttung mit einer Geschwindigkeit von 0,9 m/s (bestimmt mittels Aerometer, Fa. Testo, Typ 445) durchströmt wurde. Der Ofen wurde nun mit der folgenden Temperaturrampe auf 380 °C aufgeheizt: innerhalb von 40 min auf 180 °C aufheizen, 30 min halten, innerhalb von 10 min auf 220°C aufheizen, 30 min halten, innerhalb von 13 min auf 270°C aufheizen, 30 min halten, innerhalb von 25 min auf 340°C und dann innerhalb von 40 min auf 380°C aufheizen. Diese Temperatur wurde dann 390 min gehalten. Währenddessen wurde der NH$_3$-Gehalt in der abgesaugten Atmosphäre der thermischen Behandlung kontinuierlich durch FTIR-Spektroskopie überwacht (Spektrometer der Fa. Nicolet, Typ "Impact", IR-Edelstahlzelle mit CaF$_2$-Fenster, 10 cm Schichtdicke, Temperierung auf 120°C, Bestimmung der Konzentration anhand der Intensität der Bande bei 3.333 cm$^{-1}$). Der NH$_3$-Gehalt blieb während der gesamten thermischen Behandlung ≤ 2,4 Vol.-%. Dieser Maximalwert wurde bei 220°C erreicht. Die so erhaltenen ringförmigen Katalysatorformkörper wiesen alle eine Seitendruckfestigkeit von 15 ± 2 N, einen Ammonium-Gehalt (bestimmt durch Titration nach Kjeldahl) von 0,6 Gew.-% NH$_4^+$ und einen MoOs-Gehalt von 1 XRD-Intensitäts-% auf. Letzterer berechnet sich als Verhältnis der Intensität des (021)-MoO$_3$-Reflexes bei 2Θ = 27,3° zur Intensität des (222)-Reflexes der Heteropolyverbindung bei 2Θ = 26,5° im Röntgenpulverdiffraktogramm (mit Cu-Kα-Strahlung).

(Alternativ zur beschriebenen Kalzinierung im Kammerofen kann die Kalzinierung auch hier, wie in A) beschrieben, im Bandkalzinierer erfolgen.)

Zur Testung der jeweils erhaltenen ringförmigen Vollkatalysatoren für eine heterogen katalysierte Partialoxidation von Methacrolein zu Methacrylsäure wurden jeweils 2 kg der so hergestellten ringförmigen Katalysatorformkörper mit einer Vor- und einer Nachschüttung von jeweils 50 g Steatitringen (Steatit C220 der Fa. CeramTec) der Geometrie 7 mm x 7 mm x 4 mm Außendurchmesser x Höhe x Innendurchmesser in ein Modellrohr aus Edelstahl (Außendurchmesser = 30 mm, Innendurchmesser = 26 mm, Länge = 4,15 m) gefüllt (Füllhöhe: 397 cm). Dieses befand sich in einem mit Stickstoff geperlten, auf 287 °C temperierten Salzbad. Die katalytische Testung erfolgte unter Kreisgasfahrweise: Das Reaktoraustrittsgas wurde hierbei auf eine Venturidüse gefahren, dort mit 75 °C warmem Wasser abgeschreckt und dann in den Sumpf der auf 75°C temperierten Destillationskolonne A geleitet. Ca. 55 kg/Tag einer Mischung aus Reaktionsprodukt und Wasser (typischerweise ca. 9,5 Gew.-% Methacrylsäure, ca. 0,8 Gew.-% Essigsäure und ca. 0,1 Gew.-% Acrylsäure in Wasser) wurden hier entnommen. Der abgereicherte Gasstrom gelangte in die Kolonne A. In deren Mitte wurde ein Teilstrom entnommen und unten in eine auf 7 °C temperierte Kolonne B geleitet. Mit einer am Kopf der Kolonne B eingespeisten Lösung von 6 Gew.-% Hydrochinon in Wasser (2 kg/h) wurde das Abgas in dieser Kolonne von den verbliebenen organischen Komponenten befreit und entwich am Kollonnenkopf. Der Inhalt des Sumpfs der Kolonne B (im wesentlichen ca. 1,4 Gew.-% Methacrolein in Wasser) wurde an den Kopf der Kolonne A gepumpt; dort wurden zusätzlich 220 g/h Methacrolein eingespeist. Am Kopf der Kolonne A wurden bei einer Kopftemperatur von 66°C 1700 Nl/h Kreisgas entnommen, mit 450 Nl/h Frischluft gemischt und als Eduktgas das ca. 5 Vol.-% Methacrolein, ca. 12 Vol.-% O$_2$, ca. 21 Vol.-% Wasserdampf, ca. 2,5 Vol.-% CO, ca. 3 Vol.-% CO$_2$ und weiteres Inertgas (im wesentlichen Stickstoff) enthielt, in den Reaktor geleitet. Damit ergab sich eine massenbezogene Raumgeschwindigkeit (WHSV) von 0,17 h$^{-1}$.

Während der 5-tägigen Testung wurde der Methacrolein-Umsatz im einfachen Durchgang bei 65 mol-% gehalten; hierzu wurde die Salzbadtemperatur schrittweise erhöht.

Am jeweils 5. Tag ergaben sich in Abhängigkeit vom verwendeten Graphit die nachfolgenden Selektivitäten S$^{MA}$ der Methacrylsäurebildung:

| Graphit | S$^{MA}$ (mol-%) |
|---------|-------------------|
| V1 | 85,4 |
| V2 | 85,2 |
| V3 | 85,6 |
| B1 | 86,0 |
| B2 | 86,2 |

D) Herstellung und Testung von ringförmigen Vollkatalysatorformkörpern mit einer Vanadium, Phosphor, Eisen und Sauerstoff umfassenden Multimetalloxidaktivmasse und unter Verwendung verschiedener Graphite als Formungshilfsmittel

In einem mit Stickstoff inertisierten, über Druckwasser außenbeheizbaren 8 m³-Stahl/Email-Rührkessel mit Strombrechern wurden 4602 kg iso-Butanol vorgelegt. Nach Inbetriebnahme des dreistufigen Impellerrührers wurde das iso-Butanol unter Rückfluss auf 90°C aufgeheizt. Bei dieser Temperatur wurde nun über eine Förderschnecke mit der Zugabe von 690 kg Vanadiumpentoxid begonnen. Nachdem nach ca. 20 Minuten etwa 2/3 der gewünschten Menge an Vanadiumpentoxid zugegeben waren, wurde bei weiterer Zugabe an Vanadiumpentoxid mit der Einpumpung von 805 kg einer eine Temperatur von 50 °C aufweisenden Polyphosphorsäure mit einem $P_2O_5$-Gehalt von 76 Gew.-% (entspricht 105 Gew.-% $H_3PO_4$) begonnen. Nach beendeter Zugabe der Phosphorsäure wurde das Reaktionsgemisch unter Rückfluss auf etwa 100 bis 108°C erhitzt und unter diesen Bedingungen 14 Stunden belassen. Im Anschluss daran wurde die heiße Suspension innerhalb von 70-80 Minuten auf 60°C abgekühlt und 22,7 kg Fe-(III)Phosphat (29,9 Gew-% Fe) zugegeben. Nach erneutem Hochheizen innerhalb von 70 Minuten auf Rückfluss siedete die Suspension unter Rückfluss für eine weiter Stunde. Anschließend wurde die Suspension in eine zuvor mit Stickstoff inertisierte und beheizte Druckfilternutsche abgelassen und bei einer Temperatur von etwa 100 °C bei einem Druck oberhalb der Filternutsche von bis zu 0,35 MPa abs abfiltriert. Der Nutschkuchen wurde durch stetiges Einleiten von Stickstoff bei 100 °C und unter Rühren mit einem mittig angeordneten, in der Höhe verstellbaren Rührer innerhalb von etwa einer Stunde trockengeblasen. Nach dem Trockenblasen wurde auf ca. 155 °C aufgeheizt und auf einen Druck von 15 kPa abs (150 mbar abs) evakuiert. Die Trocknung wurde bis zu einem Rest-iso-butanolgehalt von < 2 Gew.-% in der getrockneten Katalysator-Vorläufermasse durchgeführt.

Das Fe/V-Verhältnis betrug 0,016.

Anschließend wurde das getrocknete Pulver 2 Stunden unter Luft in einem Drehrohr mit einer Länge von 6,5 m, einem Innendurchmesser von 0,9 m und innenliegenden spiralförmigen Wendeln (zu Durchmischungszwecken) behandelt. Die Drehzahl des Drehrohres betrug 0,4 U/min. Das Pulver wurde in einer Menge von 60 kg/h in das Drehrohr gefördert. Die Luftzufuhr betrug 100 m³/h. Die direkt an der Außenseite des von außen beheizten Drehrohrs gemessenen Temperaturen der fünf gleichlangen Heizzonen betrugen in Richtung "vom Ausgang des Pulvers" zum "Eingang des Pulvers" ins Drehrohr 250°C, 300°C, 345°C, 345°C und 345°C.

Die dem Drehrohr entnommene Vorläufermasse wurde mit 1 Gew.-% des jeweiligen Graphits in einem Rhönradmischer innig und homogen vermischt. Das resultierende Gemisch wurde dann in einem Laborkalander mit 2 gegenläufigen Stahlwalzen bei einem Pressdruck von 9 bar kompaktiert und durch ein Sieb mit einer Maschenweite von 0,8 mm gedrückt. Das resultierende Kompaktat wies eine im wesentlichen einheitliche Körnung von ≥ 0,4 mm und ≤ 0,8 mm auf. Es wurde mit weiteren 2 Gew.-% des jeweiligen Graphit in einem Rhönradmischer vermischt, und in einer Tablettiermaschine (Tablettiermaschine vom Typ Kilian LX 18 (Fa. Kilian, D-50735 Köln) Presskraft 5,3 N) zu 5 x 3,2 x 2,5 mm Hohlzylindern (äußerer Durchmesser x Höhe x Durchmesser des inneren Lochs) tablettiert. Diese wiesen eine Seitendruckfestigkeit von 11 N auf.

Die erhaltenen 5 x 3,2 x 2,5 mm (A x H x I) Hohlzylinder wurden gemäß der WO 03/78059, Seite 39 unter Beispiel 9 zu den jeweiligen Katalysatorformkörpern kalziniert.

Zum Zweck der Testung der jeweils erhaltenen Katalysatorformkörper als Katalysatoren in einem Verfahren der heterogen katalysierten partiellen Gasphasenoxidation von n-Butan zur Herstellung von Maleinsäureanhydrid wurde eine Versuchsanlage verwendet, die mit einer Zufuhr-Einheit und einer Rohrbündelreaktor-Einheit ausgestattet war. Die Anlage wurde im "geraden Durchgang" betrieben, wie in der EP-B 1 261 424 beschrieben.

Der Kohlenwasserstoff wurde mengengeregelt in flüssiger Form über eine Pumpe zugegeben. Als Sauerstoff-haltiges Gas wurde Luft mengengeregelt zugegeben. Triethylphosphat (TEP) wurde ebenfalls mengengeregelt, gelöst in Wasser, in flüssiger Form zugegeben.

Die Rohrbündelreaktor-Einheit bestand aus einem Rohrbündelreaktor mit einem Reaktorrohr. Die Länge des Reaktorrohrs aus Edelstahl betrug 6,5 m, der Innendurchmesser 22,3 mm, die Wanddicke 2,3 mm. Innerhalb des Reaktorrohres befand sich in einem axial zentriertenSchutzrohr mit 6 mm Außendurchmesser ein Multi-Thermoelement mit 20 Temperaturmessstellen. Die Temperierung des Reaktorrohres erfolgte durch einen Wärmeträger-Kreislauf mit einer Länge von 6,5 m. Als Wärmeträgermedium wurde eine Salzschmelze eingesetzt.

Das Reaktorrohr wurde von dem Reaktionsgasgemisch von oben nach unten durchströmt. Die oberen 0,2 m des 6,5 m langen Reaktorrohres blieben ungefüllt. Als Nächstes folgte eine 0,3 m lange Vorheizzone, welche mit Steatitformkörpern aus Steatit C220 der Geometrie 5 x 3,2 x 2 mm (A x H x I) als Inertmaterial gefüllt war. Im Anschluss an die Vorheizzone folgte die Katalysatorschüttung, welche insgesamt jeweils 2180 ml Katalysator enthielt.

Direkt nach der Rohrbündelreaktor-Einheit wurde gasförmiges Produkt entnommen und der gaschromatographischen online-Analytik zugeführt. Der Hauptstrom des gasförmigen Reaktoraustrags wurde aus der Anlage ausgeschleust.

Die Zusammensetzung des Beschickungsgasgemischs war

| 2 Vol.-% | n-Butan, |
| 3 Vol.-% | $H_2O$, |
| 2,25 Vol.-ppm | TEP und |

als Restmenge Luft.

Der Eingangsdruck betrug 3,3 bar. Die Gesamtbelastung des Katalysatorfestbetts (reine Inertmaterialabschnitte werden nicht einbezogen) betrug 2000 Nl/(l•h), die Salzbadtemperatur (ca. 415°C) wurde jeweils so eingestellt, dass der Butanumsatz, bezogen auf einmaligen Durchgang, 85 mol-% betrug.

Nach einer Betriebszeit der Versuchsanlage von jeweils 150 h wurde die Selektivität der Maleinsäureanhydridbildung $S^{MAH}$ bestimmt.

Die Werte waren in Abhängigkeit vom verwendeten Graphit wie folgt:

| Graphit | $S^{MAH}$ (mol-%) |
|---------|-------------------|
| V1 | 56,9 |
| V2 | 56,8 |
| V3 | 57,1 |
| B1 | 57,5 |
| B2 | 57,4 |

Man kann davon ausgehen, dass die Erfindung, im Rahmen der beigefügten Ansprüche, anders als hierin spezifisch beschrieben, ausgeführt werden kann.

**Patentansprüche**

1. Verfahren zur Herstellung von Katalysatorformkörpern, deren Aktivmasse ein Multielementoxid ist, bei dem man ein feinteiliges Vorläufergemisch, das als ein feinteiliges Formungshilfsmittel Graphit zugesetzt enthält, zur gewünschten Geometrie formt und die dabei resultierenden Katalysatorvorläuferformkörper bei erhöhter Temperatur unter Erhalt der Katalysatorformkörper, deren Aktivmasse ein Multielementoxid ist, thermisch behandelt, **dadurch gekennzeichnet, dass**

    a) für die spezifische Oberflächen $O_G$ des feinteiligen Graphits gilt:

$$0,5 \text{ m}^2/\text{g} \leq O_G \leq 5 \text{ m}^2/\text{g},$$

    und

    b) für den Partikeldurchmesser des feinteiligen Graphits gilt:

$$90 \text{ µm} \leq d_{50} \leq 200 \text{ µm},$$

$$20 \text{ µm} \leq d_{10} \leq 90 \text{ µm},$$

$$150 \text{ µm} \leq d_{90} \leq 300 \text{ µm}.$$

2. Verfahren nach Anspruch 1, **dadurch gekennzeichnet, dass** das feinteilige Vorläufergemisch, bezogen auf sein Gesamtgewicht, 0,1 bis 35 Gew.-% des feinteiligen Graphit zugesetzt enthält.

3. Verfahren nach einem der Ansprüche 1 bis 2, **dadurch gekennzeichnet, dass** bei der thermischen Behandlung der Katalysatorvorläuferformkörper bis zum Erhalt der Katalysatorformkörper 1 bis 70 Gew.-% des in den Katalysatorvorläuferformkörpern enthaltenen Graphit in gasförmig entweichende Verbindungen gewandelt wird.

4. Verfahren nach einem der Ansprüche 1 bis 3, **dadurch gekennzeichnet, dass** die thermische Behandlung der Katalysatorvorläuferformkörper in oxidierender Atmosphäre erfolgt.

5. Verfahren nach einem der Ansprüche 1 bis 4, **dadurch gekennzeichnet, dass** die thermische Behandlung der Katalysatorvorläuferformkörper bei Temperaturen von 150 bis 650°C erfolgt.

6. Verfahren nach einem der Ansprüche 1 bis 5, **dadurch gekennzeichnet, dass** die thermische Behandlung der Katalysatorvorläuferformkörper im Luftstrom erfolgt.

7. Verfahren nach einem der Ansprüche 1 bis 6, **dadurch gekennzeichnet, dass** der feinteilige Graphit natürlicher Graphit ist.

8. Verfahren nach einem der Ansprüche 1 bis 7, **dadurch gekennzeichnet, dass** der feinteilige Graphit eine Temperatur $T_i$ des Verbrennungsanfangs aufweist, die wenigstens 50°C größer ist, als die bei der thermischen Behandlung der Katalysatorvorläuferformkörper auf diese für einen Zeitraum von wenigstens 30 Minuten einwirkende höchste Temperatur.

9. Verfahren nach einem der Ansprüche 1 bis 8, **dadurch gekennzeichnet, dass** $T_i$ des feinteiligen Graphit $\geq$ 500°C beträgt.

10. Verfahren nach einem der Ansprüche 1 bis 9, **dadurch gekennzeichnet, dass** die maximale Verbrennungstemperatur $T_m$ des feinteiligen Graphit $\geq$ 680°C. beträgt.

11. Verfahren nach einem der Ansprüche 1 bis 10, **dadurch gekennzeichnet, dass** die Partikeldurchmesser des feinteiligen Vorläufergemischs, das zugesetzte Formungshilfsmittel ausgenommen, im Bereich 10 bis 2000 $\mu$m liegen.

12. Verfahren nach einem der Ansprüche 1 bis 11, **dadurch gekennzeichnet, dass** die Formung zur gewünschter Geometrie durch Tablettieren erfolgt.

13. Verfahren nach einem der Ansprüche 1 bis 12, **dadurch gekennzeichnet, dass** die Formung unter Anwendung eines Formgebungsdrucks von 50 kg/cm$^2$ bis 5000 kg/cm$^2$ erfolgt.

14. Verfahren nach einem der Ansprüche 1 bis 13, **dadurch gekennzeichnet, dass** der Katalysatorformkörper eine Kugel mit einem Durchmesser von 2 bis 10 mm ist.

15. Verfahren nach einem der Ansprüche 1 bis 13, **dadurch gekennzeichnet, dass** der Katalysatorformkörper ein Vollzylinder, ist, dessen Außendurchmesser und Länge 2 bis 10 mm betragen.

16. Verfahren nach einem der Ansprüche 1 bis 13, **dadurch gekennzeichnet, dass** der Katalysatorformkörper ein Ring ist, dessen Außendurchmesser und Länge 2 bis 10 mm betragen und dessen Wandstärke 1 bis 3 mm beträgt.

17. Verfahren nach einem der Ansprüche 1 bis 16, **dadurch gekennzeichnet, dass** die Aktivmasse ein Mulitmetalloxid ist.

18. Verfahren nach einem der Ansprüche 1 bis 16, **dadurch gekennzeichnet, dass** die Aktivmasse ein Multielementoxid ist, das

    a) die Elemente Mo, Fe und Bi, oder
    b) die Elemente Mo und V, oder
    c) die Elemente Mo, V und P, oder
    d) die Elemente V und P

umfasst.

19. Verfahren einer heterogen katalysierten Gasphasenreaktion, **dadurch gekennzeichnet, dass**

    i) ein Katalysatorformkörper nach einem Verfahren gemäß einem der Ansprüche 1 bis 18 hergestellt wird, und

ii) der Katalysatorformkörper in einer heterogen katalysierten Gasphasenreaktion verwendet wird, wobei die heterogen katalysierte Gasphasenreaktion eine heterogen katalysierte Partialoxidation einer organischen Verbindung ist.

20. Verfahren nach Anspruch 19, **dadurch gekennzeichnet, dass** die heterogen katalysierte Partialoxidation die partielle Oxidation von Propen zu Acrolein und/oder Acrylsäure ist.

21. Verfahren nach Anspruch 19, **dadurch gekennzeichnet, dass** die heterogen katalysierte Partialoxidation die partielle Oxidation von Propan zu Acrolein und/oder Acrylsäure ist.

22. Verfahren nach Anspruch 19, **dadurch gekennzeichnet, dass** die heterogen katalysierte Partialoxidation die partielle Oxidation von iso-Buten oder tert.-Butanol zu Methacrolein ist.

23. Verfahren nach Anspruch 19, **dadurch gekennzeichnet, dass** die heterogen katalysierte Partialoxidation die partielle Oxidation von Methacrolein zu Methacrylsäure ist.

24. Verfahren nach Anspruch 19, **dadurch gekennzeichnet, dass** die heterogen katalysierte Partialoxidation die partielle Oxidation von Acrolein zu Acrylsäure ist.

25. Verfahren nach Anspruch 19, **dadurch gekennzeichnet, dass** die heterogen katalysierte Partialoxidation die partielle Oxidation eines Kohlenwasserstoffs mit mindesten 4 Kohlenstoffatomen zu Maleinsäureanhydrid ist.

**Claims**

1. A process for preparing shaped catalyst bodies whose active composition is a multielement oxide, in which a finely divided precursor mixture which comprises added graphite as a finely divided shaping assistant is shaped to the desired geometry and the resulting shaped catalyst precursor bodies are treated thermally at elevated temperature to obtain the shaped catalyst bodies whose active composition is a multielement oxide, wherein

a) for the specific suface area $O_G$ of the finely divided graphite:

$$0.5 \ \mathrm{m^2/g} \leq O_G \leq 5 \ \mathrm{m^2/g}$$

and
b) for the particle diameter of the finely divided graphite:

$$90 \ \mu\mathrm{m} \leq d_{50} \leq 200 \ \mu\mathrm{m},$$

$$20 \ \mu\mathrm{m} \leq d_{10} \leq 90 \ \mu\mathrm{m},$$

$$150 \ \mu\mathrm{m} \leq d_{90} \leq 300 \ \mu\mathrm{m}.$$

2. The process according to claim 1, wherein the finely divided precursor mixture, based on its total weight, comprises from 0.1 to 35% by weight of the added finely divided graphite.

3. The process according to either of claims 1 and 2, wherein the thermal treatment of the shaped catalyst precursor bodies until the shaped catalyst bodies are obtained converts from 1 to 70% by weight of the graphite present in the shaped catalyst precursor bodies to compounds which escape in gaseous form.

4. The process according to any of claims 1 to 3, wherein the thermal treatment of the shaped catalyst precursor bodies is effected in oxidizing atmosphere.

5. The process according to any of claims 1 to 4, wherein the thermal treatment of the shaped catalyst precursor bodies is effected at temperatures of from 150 to 650°C.

6. The process according to any of claims 1 to 5, wherein the thermal treatment of the shaped catalyst precursor bodies is effected in an air stream.

7. The process according to any of claims 1 to 6, wherein the finely divided graphite is natural graphite.

8. The process according to any of claims 1 to 7, wherein the finely divided graphite has a temperature $T_i$ at the start of combustion which is at least 50°C greater than the highest temperature which is effective for a period of at least 30 minutes on the shaped catalyst bodies in the course of the thermal treatment.

9. The process according to any of claims 1 to 8, wherein $T_i$ of the finely divided graphite is $\geq$ 500°C.

10. The process according to any of claims 1 to 9, wherein the maximum combustion temperature $T_m$ of the finely divided graphite is $\geq$ 680°C.

11. The process according to any of claims 1 to 10, wherein the particle diameters of the finely divided precursor mixture, excluding the added shaping assistant, are in the range from 10 to 2000 $\mu$m.

12. The process according to any of claims 1 to 11, wherein the shaping to the desired geometry is effected by tableting.

13. The process according to any of claims 1 to 12, wherein the shaping is effected using a shaping pressure of from 50 kg/cm$^2$ to 5000 kg/cm$^2$.

14. The process according to any of claims 1 to 13, wherein the shaped catalyst body is a sphere having a diameter of from 2 to 10 mm.

15. The process according to any of claims 1 to 13, wherein the shaped catalyst body is a solid cylinder whose external diameter and length are from 2 to 10 mm.

16. The process according to any of claims 1 to 13, wherein the shaped catalyst body is a ring whose external diameter and length are from 2 to 10 mm and whose wall thickness is from 1 to 3 mm.

17. The process according to any of claims 1 to 16, wherein the active composition is a multimetal oxide.

18. The process according to any of claims 1 to 16, wherein the active composition is a multielement oxide which comprises

> a) the elements Mo, Fe and Bi, or
> b)the elements Mo and V, or
> c)the elements Mo, V and P, or
> d)the elements V and P.

19. A process for heterogeneously catalyzed gas phase reaction, wherein

> i) a shaped catalyst body is prepared by a process according to any of claims 1 to 18, and
> ii) the shaped catalyst body is used in a heterogeneously catalyzed gas phase reaction, the heterogeneously catalyzed gas phase reaction being a heterogeneously catalyzed partial oxidation of an organic compound.

20. The process according to claim 19, wherein the heterogeneously catalyzed partial oxidation is the partial oxidation of propene to acrolein and/or acrylic acid.

21. The process according to claim 19, wherein the heterogeneously catalyzed partial oxidation is the partial oxidation of propane to acrolein and/or acrylic acid.

22. The process according to claim 19, wherein the heterogeneously catalyzed partial oxidation is the partial oxidation of isobutene or tert-butanol to methacrolein.

**23.** The process according to claim 19, wherein the heterogeneously catalyzed partial oxidation is the partial oxidation of methacrolein to methacrylic acid.

**24.** The process according to claim 19, wherein the heterogeneously catalyzed partial oxidation is the partial oxidation of acrolein to acrylic acid.

**25.** The process according to claim 19, wherein the heterogeneously catalyzed partial oxidation is the partial oxidation of a hydrocarbon having at least 4 carbon atoms to maleic anhydride.

**Revendications**

**1.** Procédé pour la production de corps façonnés de catalyseur, dont la masse active est un oxyde de plusieurs éléments, dans lequel on façonne un mélange de précurseurs finement divisé, auquel on a ajouté du graphite comme un adjuvant de façonnage finement divisé, en une géométrie souhaitée et on traite thermiquement les corps façonnés de précurseur de catalyseur obtenu à température élevée avec obtention des corps façonnés de catalyseur, dont la masse active est un oxyde de plusieurs éléments, **caractérisé en ce que**

a) pour la surface spécifique $O_G$ du graphite finement divisé, l'équation suivante est d'application :

$$0,5 \ m^2/g \ \leq \ O_G \ \leq \ 5 \ m^2/g$$

et

b) pour le diamètre des particules du graphite finement divisé, les équations suivantes sont d'application :

$$90 \ \mu m \ \leq \ d_{50} \ \leq \ 200 \ \mu m,$$

$$20 \ \mu m \ \leq \ d_{10} \ \leq \ 90 \ \mu m,$$

$$150 \ \mu m \ \leq \ d_{90} \ \leq \ 300 \ \mu m.$$

**2.** Procédé selon la revendication 1, **caractérisé en ce que** le mélange de précurseurs finement divisé, par rapport à son poids total, contient 0,1 à 35% en poids du graphite finement divisé ajouté.

**3.** Procédé selon l'une quelconque des revendications 1 à 2, **caractérisé en ce que** lors du traitement thermique des corps façonnés de précurseur de catalyseur, jusqu'à l'obtention des corps façonnés de catalyseur, 1 à 70% en poids du graphite contenu dans les corps façonnés de précurseur de catalyseur sont modifiés en composés s'échappant sous forme gazeuse.

**4.** Procédé selon l'une quelconque des revendications 1 à 3, **caractérisé en ce que** le traitement thermique des corps façonnés de précurseur de catalyseur est réalisé dans une atmosphère oxydante.

**5.** Procédé selon l'une quelconque des revendications 1 à 4, **caractérisé en ce que** le traitement thermique des corps façonnés de précurseur de catalyseur est réalisé à des températures de 150°C à 650°C.

**6.** Procédé selon l'une quelconque des revendications 1 à 5, **caractérisé en ce que** le traitement thermique des corps façonnés de précurseur de catalyseur est réalisé dans un flux d'air.

**7.** Procédé selon l'une quelconque des revendications 1 à 6, **caractérisé en ce que** le graphite finement divisé est du graphite naturel.

**8.** Procédé selon l'une quelconque des revendications 1 à 7, **caractérisé en ce que** le graphite finement divisé présente une température $T_i$ de début de combustion qui est supérieure d'au moins 50°C à la température la plus élevée qui agit, lors du traitement thermique des corps façonnés de précurseur de catalyseur, sur ceux-ci pendant un laps de temps d'au moins 30 minutes.

9. Procédé selon l'une quelconque des revendications 1 à 8, **caractérisé en ce que** $T_i$ du graphite finement divisé est ≥ 500°C.

10. Procédé selon l'une quelconque des revendications 1 à 9, **caractérisé en ce que** la température de combustion maximale $T_m$ du graphite finement divisé est ≥ 680°C.

11. Procédé selon l'une quelconque des revendications 1 à 10, **caractérisé en ce que** les diamètres des particules du mélange de précurseurs finement divisé, à l'exception de l'adjuvant de façonnage, se situent dans la plage de 10 à 2000 μm.

12. Procédé selon l'une quelconque des revendications 1 à 11, **caractérisé en ce que** le façonnage en géométrie souhaitée est réalisé par compression en comprimés.

13. Procédé selon l'une quelconque des revendications 1 à 12, **caractérisé en ce que** le façonnage est réalisé avec utilisation d'une pression de façonnage de 50 kg/cm$^2$ à 5000 kg/cm$^2$.

14. Procédé selon l'une quelconque des revendications 1 à 13, **caractérisé en ce que** le corps façonné de catalyseur est une bille présentent un diamètre de 2 à 10 mm.

15. Procédé selon l'une quelconque des revendications 1 à 13, **caractérisé en ce que** le corps façonné de catalyseur est un cylindre plein dont le diamètre extérieur et la longueur sont de 2 à 10 mm.

16. Procédé selon l'une quelconque des revendications 1 à 13, **caractérisé en ce que** le corps façonné de catalyseur est un anneau dont le diamètre extérieur et la longueur sont de 2 à 10 mm et dont l'épaisseur de paroi est de 1 à 3 mm.

17. Procédé selon l'une quelconque des revendications 1 à 16, **caractérisé en ce que** la masse active est un oxyde de plusieurs métaux.

18. Procédé selon l'une quelconque des revendications 1 à 16, **caractérisé en ce que** la masse active est un oxyde de plusieurs métaux qui comprend

　　　a) les éléments Mo, Fe et Bi, ou
　　　b) les éléments Mo et V, ou
　　　c) les éléments Mo, V et P, ou
　　　d) les éléments V et P.

19. Procédé pour réaliser une réaction en phase gazeuse catalysée par voie hétérogène, **caractérisé en ce que**

　　　i) un corps façonné de catalyseur est préparé selon un procédé selon l'une quelconque des revendications 1 à 18 et
　　　ii) le corps façonné de catalyseur est utilisé dans une réaction en phase gazeuse catalysée par voie hétérogène, la réaction en phase gazeuse catalysée par voie hétérogène étant une oxydation partielle catalysée par voie hétérogène d'un composé organique.

20. Procédé selon la revendication 19, **caractérisé en ce que** l'oxydation partielle catalysée par voie hétérogène est l'oxydation partielle de propène en acroléine et/ou en acide acrylique.

21. Procédé selon la revendication 19, **caractérisé en ce que** l'oxydation partielle catalysée par voie hétérogène est l'oxydation partielle de propane acroléine et/ou en acide acrylique.

22. Procédé selon la revendication 19, **caractérisé en ce que** l'oxydation partielle catalysée par voie hétérogène est l'oxydation partielle d'iso-butène ou tert-butanol en méthacroléine.

23. Procédé selon la revendication 19, **caractérisé en ce que** l'oxydation partielle catalysée par voie hétérogène est l'oxydation partielle de méthacroléine en acide méthacrylique.

**24.** Procédé selon l'une quelconque des revendications 19, **caractérisé en ce que** l'oxydation partielle catalysée par voie hétérogène est l'oxydation partielle d'acroléine en acide acrylique.

**25.** Procédé selon la revendication 19, **caractérisé en ce que** l'oxydation partielle catalysée par voie hétérogène est l'oxydation partielle d'un hydrocarbure présentant au moins 4 atomes de carbone en anhydride de l'acide maléique.

Figur 1

**Fig. 1**

Figur 2

Figur 3

Figur 4

Figur 5

Figur 6

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2005030393 A
- EP 467144 A
- WO 0168245 A
- DE 10353014 A
- DE 10360396 A
- DE 102005035978 A
- EP 1060792 A
- WO 03078310 A
- WO 0378059 A
- DE 102005037678 A
- US 20050131253 A
- WO 02062737 A
- WO 0378310 A
- DE 19855913 A
- WO 0224620 A
- DE 19922113 A
- EP 467114 A
- WO 05030393 A
- EP 184790 A
- DE 10046957 A
- DE 4407020 A
- EP 575897 A
- DE 3338380 C
- DE 10325487 A
- WO 0053557 A
- WO 0053558 A
- DE 19910506 A
- EP 1106598 A
- WO 0136364 A
- DE 19927624 A
- DE 19948248 A
- DE 19948523 A
- DE 19948241 A
- EP 700714 A
- DE 10313213 A
- DE 10313209 A
- DE 10232748 A
- DE 10313208 A
- WO 03039744 A
- EP 279374 A
- DE 3338380 A
- DE 3300044 A
- DE 102004003212 A
- DE 102005013039 A
- DE 102005009891 A
- DE 102005010111 A
- DE 102005009885 A
- EP 990636 A

- DE 10246119 A
- DE 10245585 A
- DE 4431957 A
- EP 700893 A
- EP 468290 B
- DE 19746210 A
- EP 015565 A
- DE 10063162 A
- DE 10337788 A
- EP 962253 A
- DE 10122027 A
- EP 608838 A
- DE 19835247 A
- EP 895809 A
- EP 1254709 A
- EP 1192987 A
- EP 1262235 A
- EP 1193240 A
- JP 11343261 A
- JP 11343262 A
- EP 1090684 A
- EP 1301457 A
- EP 1254707 A
- EP 1335793 A
- DE 10046672 A
- DE 10034825 A
- EP 1556337 A
- DE 10033121 A
- WO 0198246 A
- EP 1558569 A
- DE 102004025445 A
- DE 2351151 A
- DE 2526238 A
- EP 092097 A
- EP 58927 A
- DE 4132263 A
- DE 4132684 A
- DE 4022212 A
- EP 522871 A
- DE 2106796 A
- DE 1624921 A
- GB 1464198 A
- GB 1291354 A
- DE 2025430 A
- DE 1254137 B
- DE 2159346 A
- EP 372972 A
- WO 8907101 A
- DE 4311608 A

- DE 10131297 A
- EP 529853 A
- WO 0196270 A

- DE 10028582 A
- EP 1261424 B

**In der Beschreibung aufgeführte Nicht-Patentliteratur**

- Quantitative Organische Elementaranalyse. VCN Verlagsgesellschaft mbH. 1991, 225 ff

- **BEYER.** Lehrbuch der organischen Chemie. Hirzel Verlag, 1973, 261